# FASCICULE DE BREVET EUROPEEN

(11) **EP 2 448 940 B1**
(45) Date de publication et mention de la délivrance du brevet: **21.05.2014**
(21) Numéro de dépôt: 10742013.5
(22) Date de dépôt: 01.07.2010
(51) Int. Cl.: C07D 487/04, A61K 31/519, A61P 35/00, C07D 239/06, C07D 239/42

(54) **Dérivés de 6,7,8,9-tétrahydro-pyrimido{1,2-a} pyrimidin-4-one, leur préparation et leur utilisation pharmaceutique**
6,7,8,9-Tetrahydro-pyrimido{1,2-a} pyrimidin-4-onderivate, Verfahren zu ihrer Herstellung und ihre pharmazeutische Anwendung
6,7,8,9-tetrahydro-pyrimido{1,2-a} pyrimidin-4-one derivatives, their preparation and pharmaceutical use thereof

(30) Priorité: 02.07.2009 FR 0903237; 10.09.2009 US 241098 P; 09.10.2009 FR 0957069
(43) Date de publication de la demande: 09.05.2012
(73) Titulaire: SANOFI, 75008 Paris (FR)
(72) Inventeur: BACQUE, Eric, F-75013 Paris (FR); BROLLO, Maurice, F-75013 Paris (FR); CLAUSS, Annie, F-75013 Paris (FR); EL AHMAD, Youssef, F-75013 Paris (FR); FILOCHE-ROMMÉ, Bruno, F-75013 Paris (FR); HALLEY, Frank, F-75013 Paris (FR); KARLSSON, Karl Andreas, F-75013 Paris (FR); MARCINIAK, Gilbert, F-75013 Paris (FR); RONAN, Baptiste, F-75013 Paris (FR); SCHIO, Laurent, F-75013 Paris (FR); VIVET, Bertrand, F-75013 Paris (FR); VIVIANI, Fabrice, F-75013 Paris (FR); ZIMMERMANN, André, F-75013 Paris (FR)
(74) Mandataire: Le Coupanec, Pascale A.M.P.
(86) Numéro de dépôt international: PCT/FR2010/051374
(87) Numéro de publication internationale: WO 2011/001113

(56) Documents cités:
- WO-A1-02/18386
- WO-A1-03/072579
- WO-A1-2006/109081
- WO-A2-03/027116
- WO-A2-2008/148074
- FR-A1- 2 378 792

## Description

La présente invention concerne de nouveaux composés chimiques (6,7,8,9-tétrahydro-pyrimido{1,2-a}pyrimidin-4-one), dérivés de pyrimidinones, leur procédé de préparation, les nouveaux intermédiaires obtenus, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme. Plus particulièrement, l'invention concerne, de nouveaux dérivés de pyrimidinones et leur utilisation pharmaceutique pour la prévention et le traitement d'affections capables d'être modulées par l'inhibition de la voie PI3K/AKT/mTOR. AKT est un acteur clé dans cette voie de signalisation. Un niveau élevé de phosphorylation d'AKT est le marqueur de l'activation de la voie qui est retrouvée dans de nombreux cancers humains.

Les produits de la présente invention peuvent ainsi notamment être utilisés pour la prévention ou le traitement d'affections capables d'être modulées par l'inhibition de la phosphorylation d'AKT (P-AKT). L'inhibition de P-AKT peut être notamment obtenue par l'inhibition de la voie PI3K/AKT/mTOR, et en particulier par l'inhibition de kinases appartenant à cette voie comme les récepteurs à activité tyrosine kinase tels EGFR, IGFR, ErbB2, la 3'-phosphoinositide-dependent protein kinase-1 (PDK1), la phosphoinositide kinase PI3K, la serine-threonine kinase AKT, la kinase mTOR.

L'inhibition et la régulation de la voie PI3K/AKT/mTOR constitue notamment un nouveau et puissant mécanisme d'action pour le traitement d'un grand nombre de maladies cancéreuses incluant des tumeurs solides et liquides. De telles affections que peuvent traiter les produits de la présente demande sont les tumeurs humaines solides ou liquides.
Cette invention concerne également, de nouveaux dérivés de pyrimidinones et leur utilisation pharmaceutique pour la prévention et le traitement d'affections impactées par la modulation de l'autophagie. L'inhibition et la régulation de l'autophagie constitue un nouveau mécanisme d'action pour le traitement d'un grand nombre de maladies cancéreuses incluant des tumeurs solides et liquides.
Cette invention concerne egalement, de nouveaux dérivés de pyrimidinones et leur utilisation pharmaceutique pour le traitement de maladies parasitaires telles que la malaria, la maladie du sommeil, la maladie de Chagas, les leishmanioses.

### Rôle de la voie PI3K/AKT/mTOR

La voie de signalisation PI3K/AKT/mTOR est un réseau complexe qui régule de multiples fonctions cellulaires, comme la croissance, la survie, la prolifération et la motilité cellulaire, qui sont des processus clés de la tumorigénèse.

Cette voie de signalisation est une cible importante dans le traitement du cancer car la plupart de ses effecteurs sont altérés dans les tumeurs humaines. Les principaux effecteurs contribuant à l'activation de la voie sont i) les oncogènes tels que ErbB1 (EGFR), ErbB2 (HER2), PIK3CA et AKT activés par mutation, amplification ou surexpression ; ii) la déficience des gènes suppresseurs de tumeurs tels que PTEN, TSC1/2, LKB et PML qui sont inactivés suite à des mutations ou à des délétions (Jiang L-Z & Liu L-Z, Biochim Biophys Acta, 2008, 1784 :150 ; Vivanco I & Sawyers CL, 2002, Nat Rev Cancer, 2 :489 ; Cully M et al., Nature Rev. Cancer, 2006, 6 :184).

L'activation des oncogènes de cette voie de signalisation est retrouvée dans de nombreuses maladies cancéreuses humaines.
- les mutations activatrices de PIK3CA sont présentes dans 15-30% des cancers du colon, du sein, de l'endomètre, du foie, de l'ovaire et de la prostate (TL Yuan and LC Cantley, Oncogene, 2008, 27:5497; Y. Samuels et al. Science, 2004, 304:554; KE. Bachman et al. Cancer Biol Ther, 2004, 3:772; DA Levine et al. Clin Canc Res. 2005, 11:2875; C. Hartmann et al. Acta Neuropathol. 2005, 109:639).
- les amplifications, mutations activatrices et surexpressions des RTKs tels qu'EGFR et HER2 dans les cancers du cerveau, du sein et du poumon (NSCLC)
- l'amplification et la surexpression activatrice d'AKT dans les cancers du cerveau, du poumon (NSCLC), du sein, du rein, de l'ovaire et du pancréas (Testa JR. and Bellacosa A., Proct. Natl. Acad. Sci. USA 2001, 98:10983 ; Cheng et al., Proct. Natl. Acad. Sci. USA 1992, 89: 9267 ; Bellacosa et al., Int. J. Cancer, 1995, 64:280 ; Cheng et al., Proct. Natl. Acad. Sci. USA 1996, 93 :3636 ; Yuan et al., Oncogene, 2000, 19 :2324).

La déficience des gènes suppresseurs de tumeurs de cette voie de signalisation est également retouvée dans de nombreuses maladies cancéreuses humaines:
o la délétion de *PTEN* dans 50% des cancers du poumon (NSCLC), du foie, du rein, de la prostate, du sein, du cerveau, du pancréas, de l'endomètre et du colon (Maxwell GL et al. Canc. Res. 1998, 58 :2500 ; Zhou X-P et al. Amer. J. Pathol., 2002, 161 :439 ; Endersby R & Baker SJ, Oncogene, 2008, 27 :5416 ; Li et al. Science, 1997, 275:1943; Steack PA et al., Nat. Genet., 1997, 15 :356)
o les mutations de *TSC1*/*2* dans plus de 50% des scléroses tubéreuses
o les mutations ou délétions de *LKB1* (or *STK11*) qui prédisposent aux cancers du tractus gastro-intestinal et au cancer du pancreas et qui sont retouvées en particulier dans 10-38% des adenocarcinomes du poumon (Shah U. et al. Cancer Res. 2008, 68 :3562)
o les modification de *PML* notament par translocation dans les tumeurs humaines (Gurrieri C et al, J. NAtI Cancer Inst. 2004, 96 :269).

De plus cette voie de signalisation est un facteur majeur de résistance à la chimiothérapie, la radiothérapie et à des thérapies ciblées tels que les inhibiteurs d'EGFR et HER2 par exemple (C. Sawyers et al. Nat Rev 2002).

### Role d'AKT

AKT (protéine kinase B ; PKB) est une sérine-thréonine kinase qui occupe une place centrale dans une des voies majeures de signalisation cellulaire, la voie PI3K/AKT. AKT est notamment impliquée dans la croissance, la prolifération et la survie des cellules tumorales. L'activation d'AKT se fait en deux étapes (i) par phosphorylation de la thréonine 308 (P-T308) par PDK1 et (2) par phosphorylation de la sérine 473 (P-S473) par mTORC2 (ou complexe mTOR-Rictor), résultant en une activation totale. AKT à son tour régule un grand nombre de protéines dont mTOR (mammalian target of Rapamycin), BAD, GSK3, p21, p27, FOXO ou FKHRL1 (Manning BD & Cantley LC, Cell, 2007 129 :1261). L'activation d'AKT promeut l'internalisation des nutriments, ce qui déclenche un processus de métabolisation anabolisante soutenant la croissance et la prolifération cellulaire. En particulier, AKT contrôle l'initiation de la synthèse protéique à travers une cascade d'interactions qui procède par l'intermédiaire de TSC1/2 (complexe de sclérose tubéreuse), Rheb, et TOR pour aboutir à deux cibles critiques de la voie de signalisation, p70S6K et 4EBP. AKT induit également une phosphorylation inhibitrice du facteur de transcription Forkhead et l'inactivation de GSK3β qui conduisent à l'inhibition de l'apoptose et à la progression du cycle cellulaire (Franke TF, Oncogene, 2008, 27 :6473). AKT est donc une cible pour la thérapie anti-cancéreuse et l'inhibition de l'activation d'AKT par l'inhibition de sa phosphorylation peut induire l'apoptose des cellules malignes et par la même, présenter un traitement pour le cancer.

### Les récepteurs à activité tyrosyne kinase comme IGF1R

Des niveaux anormalement élevés d'activité protéine kinase ont été impliqués dans de nombreuses maladies résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc): Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, prostate, myelome multiple) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo [Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie, radiation, et l'apoptose induite par des cytokines. De plus, l'inhibition d'IGF-I-R endogène par un dominant négatif, la formation de triple hélice ou l'expression d'un antisens provoque une suppression de l'activité transformante in vitro et la diminution de la croissance de tumeurs dans les modèles animaux.

### PDK1

La 3'-phosphoinositide-dependent protein kinase-1 (PDK1) est une des composantes essentielles de la voie de signalisation PI3K-AKT. C'est une sérine-thréonine (Ser/Thr) kinase dont le rôle est de phosphoryler et d'activer d'autres Ser/Thr kinases de la famille des AGC impliquées dans le contrôle de la croissance, la prolifération, la survie cellulaire et dans la régulation du métabolisme. Ces kinases incluent la protéine kinase B (PKB ou AKT), SGK (ou serum and glucocorticoïd regulated kinase), RSK (ou p90 ribosomal S6 kinase), p70S6K (ou p70 ribosomal S6 kinase) ainsi que diverses isoformes de la protéine kinase C (PKC) (Vanhaesebroeck B. & Alessi DR., Biochem J, 2000, 346:561). Un des rôles clés de PDK1 est donc l'activation d'AKT : en présence de PIP3, le second messager généré par PI3K, PDK-1 est recruté à la membrane plasmique via son domaine PH (plekstrin homology) et phosphoryle AKT sur la thréonine 308 situé dans la boucle d'activation, une modification essentielle de l'activation d'AKT. PDK1 est exprimée de façon ubiquitaire et est une kinase constitutivement active. PDK1 est un élément clé dans la voie de signalisation PI3K/AKT pour la régulation de processus clés dans la tumorigénèse comme la prolifération et la survie cellulaire. Cette voie étant activée dans plus de 50% des cancers humains, PDK1 représente une cible pour la thérapie anticancéreuse. L'inhibition de PDK1 devrait résulter en une inhibition effective de la proliferation et de la survie des cellules cancéreuses et donc apporter un bénéfice thérapeutique pour les cancers humains (Bayascas JR, Cell cycle, 2008, 7 :2978 ; Peifer C. & Alessi DR, ChemMedChem, 2008, 3 :1810).

### Les phosphoinositides-3 kinases (PI3Ks)

La lipide kinase PI3K est une cible importante dans cette voie de signalisation pour l'oncologie. Les PI3Ks de la classe I sont réparties en classe la (PI3Kα,β,δ) activée par les récepteurs à activité tyrosine kinase (RTKs), les récepteurs couplés aux protéines G (GPCRs), les GTPases de la famille Rho, p21-Ras et en classe Ib (PI3Kγ) activé par les GPCRs et par p21-Ras. Les PI3Ks de la classe Ia sont des hétérodimères qui consistent en une sous unité catalytique p110α, β ou δ et une sous unité régulatrice p85 ou p55. La classe Ib (p110γ) est monomérique. Les PI3Ks de la classe I sont des lipides/protéines kinases qui sont activées par les RTKs, les GPCRs ou Ras après recrutement à la membrane. Ces PI3Ks de la classe I phosphorylent le phosphatidylinositol 4,5 diphosphate (PIP2) sur la position 3 de l'inositol pour donner le phosphatidylinositol 3,4,5 triphosphate (PIP3), messager secondaire clé de cette voie de signalisation. A son tour, PIP3 recrute AKT et PDK1 à la membrane où ils se fixent par leur domaine homologue à la pleckstrine (domaine PH), conduisant à l'activation d'AKT par phosphorylation de PDK1 sur la thréonine 308. AKT phosphoryle de nombreux substrats, jouant ainsi un rôle clé dans de nombreux processus aboutissant à la transformation cellulaire comme la prolifération, la croissance et la survie cellulaire ainsi que l'angiogénèse.

Les PI3Ks de classe I sont impliquées dans les cancers humains : des mutations somatiques du gène PIK3CA qui code pour PI3K se retrouvent dans 15-35% des tumeurs humaines avec notamment deux mutations oncogéniques principales H1047R (dans le domaine kinase) et E545K/E542K (dans le domaine hélical) (Y. Samuels et al. Science, 2004, 304:554; TL Yuan and LC Cantley, Oncogene, 2008, 27:5497). Des inhibiteurs de PI3K sont attendus efficaces pour le traitement de nombreux cancers humains présentant des altérations génétiques aboutissant à l'activation de la voie PI3K/AKT/mTOR (Vogt P. et al., Virology, 2006, 344 :131 ; Zhao L & Vogt PK, Oncogene, 2008, 27 :5486).

### mTOR

mTOR (mammalian target of rapamycin) est une serine-threonine kinase apparentée aux lipide kinases de la famille PI3K. mTOR a été impliquée dans divers processus biologiques incluant la croissance, la prolifération, la motilité et la survie cellulaires. mTOR est une kinase multifonctionnelle intégrant à la fois les signaux venant des facteurs de croissance et ceux venant des nutriments pour réguler la traduction des protéines, la capture des nutriments, l'autophagie et la fonction mitochondriale. mTOR existe sous la forme de deux complexes différent appelés mTORC1 et mTORC2. mTORC1 contient la sous-unité raptor et mTORC2, la sous-unité rictor. Ces deux complexes sont régulés de façon différente : mTORC1 phosphoryle la kinase S6 (S6K) et 4EBP1, stimulant ainsi la traduction et la biogenèse des ribosomes pour faciliter la croissance des cellules et la progression dans le cycle cellulaire. S6K agit aussi dans une voie de rétro-contrôle pour atténuer l'activation d'AKT. mTORC1 est sensible à la rapamycine alors que mTORC2 est généralement insensible à la rapamycine. mTORC2 modulerait la signalisation des facteurs de croissance en phosphorylant AKT sur le résidu serine 473. mTOR a été impliquée dans diverses pathologies incluant notamment le cancer, le diabète, l'obésité, les maladies cardio-vasculaires et les désordres neurologiques. mTOR module de nombreux processus biologiques incluant la traduction, l'autophagie, et la biogenèse des ribosomes en intégrant des signaux intra et extra-cellulaires comme les signaux transportés par les facteurs de croissance, les nutriments, les niveau d'énergie et le stress cellulaire (Guertin D.A. and Sabatini D., Cancer Cell, 2007, 12 :9 ; Menon S. and Manning B.D., Oncogene, 2009, 27 :S43).

### Rôle de l'autophagie

L'autophagie est un mécanisme de dégradation intracellulaire (organelles, protéines de longues vies...) dépendant des lysosomes. Le processus autophagie implique la formation de vésicules particulières appelées autophagosomes. La lipide kinase PI3K de classe III (Vps34) est impliquée dans la formation des autophagosomes. Cette PI3K de la classe III phosphoryle le phosphatidylinositol (PI) sur la position 3 de l'inositol pour donner le phosphatidylinositol 3 triphosphate (PI3P). Le PI3P est un messager secondaire clé dans la formation des autophagosomes via le recrutement de protéines telles que WIPI, DFCP1 et Alfy. L'autophagie est un mécanisme de survie cellulaire permettant à la cellule de survivre en situation de stress, comme par exemple face à un stress métabolique. Dans le cas du cancer, l'autophagie est impliquée dans la résistance des cellules tumorales face aux stress envirronnementaux tels que : l'hypoxie, les stress oxydatifs, la carence en nutriments, mais aussi face aux stress thérapeutiques : traitements par agents anticancéreux, radiations ionisantes.

### Application en chimiothérapie anti-paludique

Le paludisme est l'une des premières causes infectieuses de mortalité au monde et touche chaque année 100 à 200 millions de personnes. La forte recrudescence de la maladie observée depuis quelques années est due à plusieurs facteurs, dont:
- les vecteurs, à savoir les anophèles, qui deviennent résistants aux insecticides classiques et bon marché,
- l'augmentation de la population dans les zones à risque et, principalement,
- la résistance de nombreuses souches de Plasmodium falciparum, parasite responsable des formes mortelles de la maladie, aux médicaments classiquement utilisés, tels que la chloroquine et la méfloquine.

La propagation de la résistance parmi les souches de Plasmodium, en particulier P. falciparum, contre la plupart des médicaments anti-paludéens démontre le besoin urgent de développer de nouveaux composés possédant un nouveau mode d'action et permettant ainsi une diminution du risque de résistance croisée. Les kinases humaines sont des cibles validées dans le traitement de nombreuses pathologies et le kinome de P. falciparum a été proposé comme un réservoir de nouvelles cibles pour le développement de nouveaux médicaments, non encore explorées dans le traitement du paludisme.

Le kinome de Plasmodium falciparum est composé de 64 kinases, dont certaines sont orthologues de kinases humaines. Des inhibiteurs des voies de signalisation kinases ont été testés pour leur capacité à inhiber in vitro et in vivo la croissance de P. falciparum et d'autres espècess pathogènes à l'origine du paludisme.

Les molécules de l'invention inhibent la croissance de P. falciparum (hautement résistant à la chloroquine souche Fcm29-Cameroun) à 1 uM et 0,1 uM dans un test in vitro utilisant des érythrocytes humains infectés, comme indiqué dans le tableau 2.

Des kinomes similaires sont présents dans toutes les espèces de Plasmodium, tels que P. falciparum, P. vivax, P. malariae, P. ovale et P. knowlesi. Les composés de l'invention peuvent donc être utiles dans le traitement du paludisme induit par tous les parasites mentionnés ci-dessus. En outre, les kinases se trouvent dans d'autres parasites, tels que Trypanosoma (par exemple T. brucei, T. cruzei) et Leishmania (par exemple L. major, L. donovani). Les composés de l'invention peuvent donc être utilisés dans le traitement de la maladie du sommeil, la maladie de Chagas, les différentes formes de leishmaniose et d'autres infections parasitaires.

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de PI3Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions.

On connaît également, des applications WO 03/027116, WO02/18386 et WO03/072579, des dérivés pyrimidone régulant l'activité de GSK3β ou GSK3β et cdk5/p25, dans le cas de maladies neurodégénératives comme la maladie d'Alzheimer. Ces produits sont des pyrimidin-4-ones et des pyrimidin-5(1H)ones qui diffèrent des produits de la présente invention en raison de la nature de leur chaîne carbonée et de leurs substitutions.

La présente invention a pour objet les produits de formule (1): 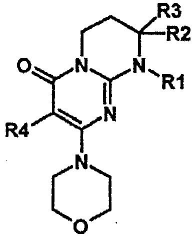 dans laquelle :
R1 représente un radical -L-aryle ou -L-hétéroaryle, tel que L représente :
   soit une simple liaison,
   soit un radical alkyle linéaire ou ramifié renfermant de 1 à 6 atomes de carbone et éventuellement substitué par un radical hydroxyle,
   soit un groupe CO ou -CO-Alk- ;
   soit un groupe L'-X où L' représente un radical alkyle linéaire ou ramifié renfermant de 1 à 6 atomes de carbone et X un atome d'oxygène ou de soufre ;
   les radicaux aryle et hétéroaryle étant éventuellement substitués par un ou plusieurs radicaux identiques ou différents choisis parmi les atomes d'halogène et les radicaux hydroxyle, CN, nitro, -COOH, -COOalk, -NRxRy,-CONRxRy, -NRxCORy, -NRxCO2Rz, -CORy, alcoxy, phénoxy, alkylthio, alkyle, cycloalkyle et hétérocycloalkyle ;
   ces derniers radicaux alcoxy, phénoxy, alkylthio, alkyle et hétérocycloalkyle, étant eux-mêmes éventuellement substitués par un ou plusieurs radicaux identiques ou différents choisis parmi les atomes d'halogène et NRvRw ;
   les radicaux hétérocycloalkyle et hétéroaryle pouvant de plus renfermer un radical oxo ;
R2 représente un atome d'hydrogène ou un radical alkyle ;
R3 représente un radical alkyle éventuellement substitué par un ou plusieurs atomes d'halogène ;
R4 représente un atome d'hydrogène ou un atome d'halogène ;
   NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
   Rz représente les valeurs de Ry à l'exception de hydrogène ;
   Rx, Ry et Rz dans les radicaux -NRxCORy, -CORy et NRxCO₂Rz étant choisis parmi les significations indiquées ci-dessus pour Rx, Ry, et Rz ;
   lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a ainsi pour objet les produits de formule (I) tels que définis ci-dessus dans laquelle :
R1 représente un radical -L-aryle ou -L-hétéroaryle, tel que L représente :
   soit une simple liaison,
   soit un radical alkyle linéaire ou ramifié renfermant de 1 à 6 atomes de carbone et éventuellement substitué par un radical hydroxyle,
   soit un groupe CO
   soit un groupe L'-X où L' représente un radical alkyle linéaire ou ramifié renfermant de 1 à 6 atomes de carbone et X un atome d'oxygène ou de soufre ;
   les radicaux aryle et hétéroaryle étant éventuellement substitués par un ou plusieurs radicaux identiques ou différents choisis parmi les atomes d'halogène et les radicaux hydroxyle, CN, nitro, -COOH, -COOalk, -NRxRy,-CONRxRy, -NRxCORy, -NRxCO2Rz, -CORy, alcoxy, phénoxy, alkylthio, alkyle, cycloalkyle et hétérocycloalkyle ;
   ces derniers radicaux alcoxy, phénoxy, alkylthio, alkyle et hétérocycloalkyle, étant eux-mêmes éventuellement substitués par un ou plusieurs radicaux identiques ou différents choisis parmi les atomes d'halogène et NRvRw ;
   les radicaux hétérocycloalkyle et hétéroaryle pouvant de plus renfermer un radical oxo ;
R2 représente un atome d'hydrogène ou un radical alkyle ;
R3 représente un radical alkyle éventuellement substitué par un ou plusieurs atomes d'halogène ;
R4 représente un atome d'hydrogène ou un atome d'halogène ;
   NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
   Rz représente les valeurs de Ry à l'exception de hydrogène ;
   Rx, Ry et Rz dans les radicaux -NRxCORy, -CORy et NRxCO₂Rz étant choisis parmi les significations indiquées ci-dessus pour Rx, Ry, et Rz ;
   lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) :
- le terme radical alkyle (ou alk) désigne les radicaux, linéaires et le cas échéant ramifiés, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et le cas échéant ramifiés, méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;
- le terme radical alkylthio désigne les radicaux linéaires et le cas échéant ramifiés, méthylthio, éthylthio, propylthio , isopropylthio, butylthio linéaire, secondaire ou tertiaire, pentylthio ou hexylthio ainsi que leurs isomères de position linéaires ou ramifiés : on préfère les radicaux alkylthio renfermant de 1 à 4 atomes de carbone de la liste ci-dessus ;

- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropylee, cyclobutyle, cyclopentyle et cyclohexyle et tout particulièrement les radicaux cyclopropyle, cyclopentyle et cyclohexyle ;
- dans le radical -O-cycloalkyle, cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, azirldyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
- le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
- le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;
   Comme exemples de radicaux hétéroaryles ou bicycliques, on peut citer plus particulièrement les radicaux pyrimidinyle, pyridyle, pyrrolyle, azaindolyle, indazolyle ou pyrazolyle, benzothiazolyle ou benzimidazolyle éventuellement substitués par un ou plusieurs substituants identiques ou différents comme indiqué ci-dessus.
   Le ou les radicaux carboxy des produits de formule (I) peuvent être salifiés ou estérifiés par les groupements divers connus de l'homme du métier parmi lesquels on peut citer, par exemple :
   - parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
   - parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a pour objet les produits de formule (I) tels que définis ci-dessus dans laquelle :
R1 représente un radical -L-phényle ou -L-hétéroaryle, tel que L représente :
   soit une simple liaison,
   soit un radical alkyle linéaire ou ramifié renfermant de 1 à 6 atomes de carbone et éventuellement substitué par un radical hydroxyle,
   soit un groupe CO ou -CO-Alk- ;
   soit un groupe L'-X où L' représente un radical alkyle linéaire ou ramifié renfermant de 1 à 6 atomes de carbone et X un atome d'oxygène ou de soufre ;
   les radicaux phényle et hétéroaryle étant éventuellement substitués par un ou plusieurs radicaux identiques ou différents choisis parmi les atomes d'halogène et les radicaux -NRxRy, alcoxy et alkyle ;
   ces derniers radicaux alcoxy et alkyle étant eux-mêmes éventuellement substitués par un ou plusieurs radicaux choisis parmi les atomes d'halogène ;
R2 représente un radical alkyle ;
R3 représente un radical alkyle éventuellement substitué par un ou plusieurs atomes d'halogène ;
R4 représente un atome d'hydrogène ou un atome de fluor ;
   NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical morpholino ;
   tous les radicaux alkyle (alk) ou alcoxy ci-dessus étant linéaires ou ramifiés et renfermant de 1 à 6 atomes de carbone,
   lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Notamment, lorsque NRxRy ou NRvRw forme un cycle comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.

Le cycle NRxRy ou NRvRw peut plus particulièrement être choisi parmi les radicaux pyrrolidinyle, morpholinyle éventuellement substitué par un ou deux radicaux alkyle ou pipérazinyle éventuellement substitué sur le second atome d'azote par un radical alkyle, phényle, ou et CH2-phényle, eux-mêmes éventuellement substitués par un ou plusieurs radicaux identiques ou différents choisis parmi les atomes d'halogène et les radicaux alkyle, hydroxyle et alcoxy.

La présente invention a tout particulièrement pour objet les produits de formule (I) tels que définis ci-dessus répondant aux formules suivantes :
- (8S)-9-[2-(4-méthoxyphényl)éthyl]-2-(morpholin-4-yl)-8-(trifluorométhyl)-6,7,8,9-tétrahydro-4H-pyrimido[1,2-a]pyrimidin-4-one
- 9-[2-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(2-phényléthyl)-8-(trifluorométhyl)-6,7,8,9-tétrahydro-4H-pyrimido[1,2-a]pyrimidin-4-one
- (8S)-9-benzyl-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2R)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-[(1R)-1-phényléthyl]-8-(trifluorométhyl)-6,7,8,9-tétrahydro-4H-pyrimido[1,2-a]pyrimidin-4-one
- (8S)-9-[1-(4-méthoxyphényl)éthyl]-2-(morpholin-4-yl)-8-(trifluorométhyl)-6,7,8,9-tétrahydro-4H-pyrimido[1,2-a]pyrimidin-4-one
- (85)-9-[(1S)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-phényl-8-(trifluorométhyl)-6,7,8,9-tétrahydro-4H-pyrimido[1,2-a]pyrimidin-4-one
- (8S)-9-(4-fluorophényl)-2-(morpholin-4-yl)-8-(trifluorométhyl)-6,7,8,9-tétrahydro-4H-pyrimido[1,2-a]pyrimidin-4-one
- (8S)-9-(3-fluorophényl)-2-(morpholin-4-yl)-8-(trifluorométhyl)-6,7,8,9-tétrahydro-4H-pyrimido[1,2-a]pyrimidin-4-one
- (8S)-9-(2-fluorophényl)-2-(morpholin-4-yl)-8-(trifluorométhyl)-6,7,8,9-tétrahydro-4H-pyrimido[1,2-a]pyrimidin-4-one
- (8S)-9-[(1R)-1-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(4-fluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(phénylcarbonyl)-8-(trifluorométhyl)-6,7,8,9-tétrahydro-4H-pyrimido[1,2-a]pyrimidin-4-one
- (8S)-2-(morpholin-4-yl)-9-(pyridin-3-yl)-8-(trifluorométhyl)-6,7,8,9-tétrahydro-4H-pyrimido[1,2-a]pyrimidin-4-one
- (8S)-2-(morpholin-4-yl)-9-(pyridin-4-yl)-8-(trifluorométhyl)-6,7,8,9-tétrahydro-4H-pyrimido[1,2-a]pyrimidin-4-one
- (8S)-9-(4-méthylphényl)-2-(morpholin-4-yl)-8-(trifluorométhyl)-6,7,8,9-tétrahydro-4H-pyrimido[1,2-a]pyrimidin-4-one
- (8S)-9-(2-chlorobenzyl)-2-(morpholin-4-yl)-8-(trifluorométhyl)-6,7,8,9-tétrahydro-4H-pyrimido[1,2-a]pyrimidin-4-one
- (8S)-9-(3-fluorobenzyl)-2-(morpholin-4-yl)-8-(trifluorométhyl)-6,7,8,9-tétrahydro-4H-pyrimido[1,2-a]pyrimidin-4-one
- (8S)-9-[2-(2-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthoxybenzyl)-2-(morpholin-4-yl)-8-(trifluorométhyl)-6,7,8,9-tétrahydro-4H-pyrimido[1,2-a]pyrimidin-4-one
- (8S)-9-(4-méthoxyphényl)-2-(morpholin-4-yl)-8-(trifluorométhyl)-6,7,8,9-tétrahydro-4H-pyrimido[1,2-a]pyrimidin-4-one
- (8S)-9-[(2-fluorophényl)carbonyl]-2-(morpholin-4-yl)-8-(trifluorométhyl)-6,7,8,9-tétrahydro-4H-pyrimido[1,2-a]pyrimidin-4-one
- (8S)-9-(3,5-difluorobenzyl)-2-(morpholin-4-yl)-8-(trifluorométhyl)-6,7,8,9-tétrahydro-4H-pyrimido[1,2-a]pyrimidin-4-one
- (8S)-9-(2,4-difluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(2,3,4-trifluorobenzyl)-8-(trifluorométhyl)-6,7,8,9-tétrahydro-4H-pyrimido[1,2-a]pyrimidin-4-one
- (8S)-9-[(5-chloro-1-benzothiophén-3-yl)méthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthylphényl)-2-(morpholin-4-yl)-8-(trifluorométhyl)-6,7,8,9-tétrahydro-4H-pyrimido[1,2-a]pyrimidin-4-one
- (8S)-9-(4-chlorophényl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-8-(trifluorométhyl)-9-[4-(trifluorométhyl)phényl]-6,7,8,9-tétrahydro-4H-pyrimido[1,2-a]pyrimidin-4-one
- (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-benzyl-3-fluoro-2-(morpholin-4-yl)-8-(trifluorométhyl)-6,7,8,9-tétrahydro-4H-pyrimido[1,2-a]pyrimidin-4-one
- (8S)-9-(3,5-difluorophényl)-2-(morpholin-4-yl)-8-(trifluorométhyl)-6,7,8,9-tétrahydro-4H-pyrimido[1,2-a]pyrimidin-4-one
- (8S)-9-[(2,6-difluorophényl)carbonyl]-2-(morpholin-4-yl)-8-(trifluorométhyl)-6,7,8,9-tétrahydro-4H-pyrimido[1,2-a]pyrimidin-4-one
- (8S)-9-[(2,4-difluorophényl)carbonyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(phénylacétyl)-8-(trifluorométhyl)-6,7,8,9-tétrahydro-4H-pyrimido[1,2-a]pyrimidin-4-one
- (8S)-9-[2-(3-chlorophényl)éthyl]-2-(morpholin-4-yl)-8-(trifluorométhyl)-6,7,8,9-tétrahydro-4H-pyrimido[1,2-a]pyrimidin-4-one
- 9-((R)-2-Benzo[b]thiophen-2-yl-2-hydroxy-ethyl)-2-morpholin-4-yl-8-(S)-trifluoromethyl-6,7,8,9-tetrahydro-pyrimido[1,2-a]pyrimidin-4-one
- 9-[(S)-2-Hydroxy-2-(3-hydroxy-phenyl)-ethyl]-2-morpholin-4-yl-8-(S)-trifluoromethyl-6,7,8,9-tetrahydro-pyrimido[1,2-a]pyrimidin-4-one
- 2-Dimethylamino-N-{3-[(S)-1-hydroxy-2-((S)-8-morpholin-4-yl-6-oxo-2-trifluoromethyl-3,4-dihydro-2H,6H-pyrimido[1,2-a]pyrimidin-1-yl)-ethyl]-phenyl}-acetamide
- 9-[(S)-2-Hydroxy-2-(2-methoxy-phenyl)-ethyl]-2-morpholin-4-yl-8-(S)-trifluoromethyl-6,7,8,9-tetrahydro-pyrimido[1,2-a]pyrimidin-4-one
- 9-[(S)-2-(4-Fluoro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(4-Chloro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(2-Chloro-4-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-(2-Hydroxy-3-phenyl-propyl)-2-morpholin-4-yl-8-(S)-trifluoromethyl-6,7,8,9-tetrahydro-pyrimido[1,2-a]pyrimidin-4-on
   9-[2-(4-Hydroxy-phenyl)-ethyl]-2-morpholin-4-yl-8-(S)-trifluoromethyl-6,7,8,9-tetrahydro-pyrimido[1,2-a]pyrimidin-4-one
   ainsi que les sels d'addition avec les acides minéraux et organiques ou avec les bases minérales et organiques desdits produits de formule (I).

La présente invention a encore pour objet tout procédé de préparation des produits de formule (I) tels que définis ci-dessus.

Les produits selon l'invention peuvent être préparés à partir de méthodes conventionnelles de chimie organique.

### Préparation de composés de formule (I)

Les schémas généraux 1 et 2 ci-dessous sont illustratifs des méthodes utilisées pour la préparation des produits de formule (I). A ce titre, elles ne sauraient constituer une limitation de la portée de l'invention, en ce qui concerne les méthodes de préparation des composés revendiqués.

Les produits de formule (I) tels que définis ci-dessus selon la présente invention peuvent ainsi notamment être préparés selon les procédés décrits dans les schémas 1 et 2.

La présente invention a ainsi également pour objet le procédé de préparation de produits de formule (I) selon le schéma 1 tel que défini ci-après.

La présente invention a ainsi également pour objet le procédé de préparation de produits de formule (I) selon le schéma général 2 tel que défini ci-après.

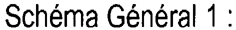

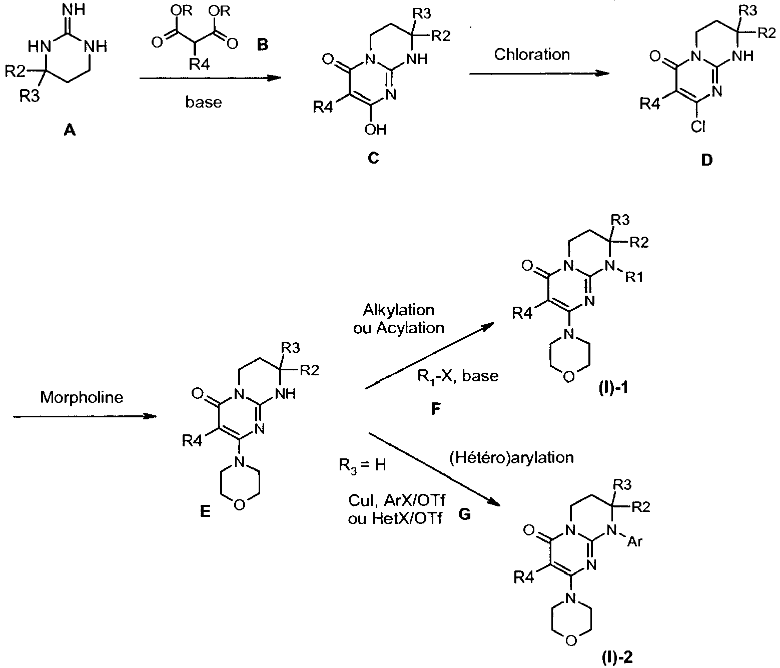

Dans le schéma général 1 :
Les guanidines A sont soit commerciales soit préparées selon les procédés décrits dans Lochead, A.W. et coll. (EP1460076 2002), Lochead, A.W. et coll. (EP1340761 2003), Lochead, A.W. et coll. (EP1454909 2004) et Lochead, A.W. et coll. (WO2005058908 2005).
Les composés C peuvent être obtenus par condensation d'une guanidine A avec un malonate de dialkyle (de préférence de diéthyl) B, en présence d'une base telle que le méthylate de sodium, à une température comprise entre 60°C et 100°C, selon les conditions décrites par exemple par Badawey E.-S.A.M. et coll. (Eur J Med Chem, 1998, 33(5), 349-361.
Les composés D peuvent être obtenus à partir d'un composé C par traitement avec un agent de chloration tel que l'oxychlorure de phosphore, en l'absence de solvant, à une température comprise entre 20°C et 120°C, ou en présence d'un solvant tel que le dichloroéthane, à une température comprise entre 20°C et la température d'ébullition du solvant, comme par exemple dans les conditions décrites par Yamashita, A. et coll.(Syn. Commun. (2004), 34(5), 795-803).
Les composés E peuvent être obtenus à partir d'un composé D par réaction avec la morpholine, en l'absence de solvant, à une température comprise entre 20°C et 120°C, ou en présence d'un solvant tel que l'acétonitrile, à une température comprise entre 20°C et la température de reflux du solvant, comme décrit par exemple par Aliabiev S.B. (Lett. Org. Chem. (2007), 4(4), 273-280.
Les composés (I)-1 peuvent être obtenus par une réaction d'alkylation ou d'acylation, par addition d'un composé F (R1-X avec R1 = L-Aryl ou Hétéroaryl tel que défini ci-dessus et X = Cl, Br, I ou OTf dans le cas d'une alkylation et X = Cl dans le cas d'une acylation) sur un mélange d'un composé E et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 80°C, tel que décrit par exemple par Ting P.C. et coll. (J. Med. Chem. (1990), 33(10), 2697-2706) dans le cas de la réaction d'alkylation.

En suivant la procédure décrite par E. P. Seest et al. dans Tet. Assymetry 17 (2006) 2154-2182, les composés F correspondants à des 1-aryl-2-chloroéthanols ou 1-hétéroaryl-2-chloroéthanols chiraux on été synthétisés à partir des dérivés chlorocétone correspondants qui sont eux-mêmes issues de la chloration dans des conditions standards des dérivés acétyles disponibles commercialement.

Les composés (I)-2 peuvent être obtenus par réaction d'un composé E avec un halogénure (X = Cl, Br ou I) ou triflate d'aryle ou d'hétéroaryle G, en présence d'un agent de couplage tel que l'iodure de cuivre, en présence ou non d'un ligand du cuivre tel le (+/-)-trans-1,2-diaminocyclohexane ou la 4,7-diméthoxy-1,10-phénantroline, en présence d'une base telle que le phosphate de potassium, dans un solvant tel que la N-méthyl pyrrolidone ou la N,N-diméthylformamide, sous irradiation micro-ondes, à une température comprise entre 100°C et 200°C, tel que décrit par exemple par Lianbo Z. et coll. (J. Org. Chem. (2009), 74(5), 2200-2202).

Alternativement, les composés (I)-1 peuvent être obtenus selon le schéma général 2.

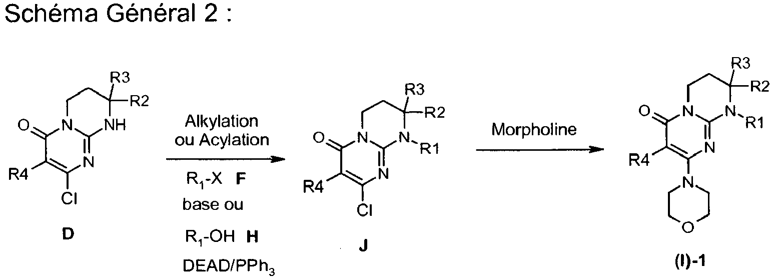

Les composés (I)-1 peuvent être obtenus à partir d'un composé J par réaction avec la morpholine, en l'absence de solvant à une température comprise entre 20°C et 120°C, ou en présence d'un solvant tel que l'acétonitrile, à une température comprise entre 20°C et la température de reflux du solvant, comme décrit par exemple par Aliabiev S.B. (Lett. Org. Chem. (2007), 4(4), 273-280.

Les composés J peuvent être obtenus par une réaction d'alkylation ou d'acylation, par addition d'un composé F (R1-X avec R1 = L-Aryl ou Hétéroaryl tel que défini ci-dessus et X = Cl, Br, I ou OTf dans le cas d'une alkylation et X = Cl dans le cas d'une acylation) sur un mélange d'un composé E et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 80°C, tel que décrit par exemple par Ting P.C. et coll. (J. Med. Chem. (1990), 33(10), 2697-2706) dans le cas de la réaction d'alkylation.

Alternativement, les composés J peuvent être obtenus par réaction de Mitsunobu entre un composé D et un alcool H, en présence d'azodicarboxylate de diéthyle et de triphénylphosphine (éventuellement supportée sur résine), dans un solvant tel que le tétrahydrofuranne, à une température comprise entre 0°C et 65°C, tel que décrit par exemple par Mitsunobu O. et coll. (Synthesis (1981), 1-28).

Dans les cas où R2 est différent de R3 et si la synthèse n'est pas stéréosélective, les énantiomères ou les éventuels diastéréoisomères des intermédiaires de synthèse ou des composés (H) peuvent être séparés par chromatographie sur support chiral.

Les exemples suivants de produits de formule (I) illustrent l'invention.

Parmi les produits de départs de formule A ou B certains sont connus et peuvent être obtenus soit commercialement, soit selon les méthodes usuelles connues de l'homme du métier, par exemple à partir de produits commerciaux.

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, trityle, benzyle, tert-butoxycarbonyle, BOC, benzyloxycarbonyle, phtallmido ou d'autres radicaux connus dans la chimie des peptides,

Les fonctions acide peuvent être, protégées par exemple sous forme d'esters formés avec les esters facilement clivables tels que les esters benzyliques ou ter butyliques ou des esters connus dans la chimie des peptides.

On trouvera une liste de différents groupements protecteurs utilisables dans les manuels connus de l'homme du métier et par exemple dans le brevet BF 2 499 995.

On peut noter que l'on peut soumettre, si désiré et si nécessaire, des produits intermédiaires ou des produits de formule (I) ainsi obtenus par les procédés indiqués ci-dessus, pour obtenir d'autres intermédiaires ou d'autres produits de formule (I), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.
Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxanne ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxanne ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol.
g) Les éventuelles formes optiquement actives des produits décrits ci-dessus peuvent être préparées par dédoublement des racémiques selon les méthodes usuelles connues de l'homme du métier.

Les produits de formule (I) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits répondant aux formules suivantes :
- (8S)-9-[2-(4-méthoxyphényl)éthyl]-2-(morpholin-4-yl)-8-(trifluorométhyl)-6,7,8,9-tétrahydro-4H-pyrimido[1,2-a]pyrimidin-4-one
- 9-[2-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(2-phényléthyl)-8-(trifluorométhyl)-6,7,8,9-tétrahydro-4H-pyrimido[1,2-a]pyrimidin-4-one
- (8S)-9-benzyl-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2R)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-[(1R)-1-phényléthyl]-8-(trifluorométhyl)-6,7,8,9-tétrahydro-4H-pyrimido[1,2-a]pyrimidin-4-one
- (8S)-9-[1-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-[(1S)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-phényl-8-(trifluorométhyl)-6,7,8,9-tétrahydro-4H-pyrimido[1,2-a]pyrimidin-4-one
- (8S)-9-(4-fluorophényl)-2-(morpholin-4-yl)-8-(trifluorométhyl)-6,7,8,9-tétrahydro-4H-pyrimido[1,2-a]pyrimidin-4-one
- (8S)-9-(3-fluorophényl)-2-(morpholin-4-yl)-8-(trifluorométhyl)-6,7,8,9-tétrahydro-4H-pyrimido[1,2-a]pyrimidin-4-one
- (8S)-9-(2-fluorophényl)-2-(morpholin-4-yl)-8-(trifluorométhyl)-6,7,8,9-tétrahydro-4H-pyrimido[1,2-a]pyrimidin-4-one
- (8S)-9-[(1R)-1-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(4-fluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(phénylcarbonyl)-8-(trifluorométhyl)-6,7,8,9-tétrahydro-4H-pyrimido[1,2-a]pyrimidin-4-one
- (8S)-2-(morpholin-4-yl)-9-(pyridin-3-yl)-8-(trifluorométhyl)-6,7,8,9-tétrahydro-4H-pyrimido[1,2-a]pyrimidin-4-one
- (8S)-2-(morpholin-4-yl)-9-(pyridin-4-yl)-8-(trifluorométhyl)-6,7,8,9-tétrahydro-4H-pyrimido[1,2-a]pyrimidin-4-one
- (8S)-9-(4-méthylphényl)-2-(morpholin-4-yl)-8-(trifluorométhyl)-6,7,8,9-tétrahydro-4H-pyrimido[1,2-a]pyrimidin-4-one
- (8S)-9-(2-chlorobenzyl)-2-(morpholin-4-yl)-8-(trifluorométhyl)-6,7,8,9-tétrahydro-4H-pyrimido[1,2-a]pyrimidin-4-one
- (8S)-9-(3-fluorobenzyl)-2-(morpholin-4-yl)-8-(trifluorométhyl)-6,7,8,9-tétrahydro-4H-pyrimido[1,2-a]pyrimidin-4-one
- (8S)-9-[2-(2-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthoxybenzyl)-2-(morpholin-4-yl)-8-(trifluorométhyl)-6,7,8,9-tétrahydro-4H-pyrimido[1,2-a]pyrimidin-4-one
- (8S)-9-(4-méthoxyphényl)-2-(morpholin-4-yl)-8-(trifluorométhyl)-6,7,8,9-tétrahydro-4H-pyrimido[1,2-a]pyrimidin-4-one
- (8S)-9-[(2-fluorophényl)carbonyl]-2-(morpholin-4-yl)-8-(trifluorométhyl)-6,7,8,9-tétrahydro-4H-pyrimido[1,2-a]pyrimidin-4-one
- (8S)-9-(3,5-difluorobenzyl)-2-(morpholin-4-yl)-8-(trifluorométhyl)-6,7,8,9-tétrahydro-4H-pyrimido[1,2-a]pyrimidin-4-one
- (8S)-9-(2,4-difluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(2,3,4-trifluorobenzyl)-8-(trifluorométhyl)-6,7,8,9-tétrahydro-4H-pyrimido[1,2-a]pyrimidin-4-one
- (8S)-9-[(5-chloro-1-benzothiophén-3-yl)méthyl]-2-(morpholin-4-yl)-8-(trifluorométhyl)-6,7,8,9-tétrahydro-4H-pyrimido[1,2-a]pyrimidin-4-one
- (8S)-9-[(1 R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthylphényl)-2-(morpholin-4-yl)-8-(trifluorométhyl)-6,7,8,9-tétrahydro-4H-pyrimido[1,2-a]pyrimidin-4-one
- (8S)-9-(4-chlorophényl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-8-(trifluorométhyl)-9-[4-(trifluorométhyl)phényl]-6,7,8,9-tétrahydro-4H-pyrimido[1,2-a]pyrimidin-4-one
- (8S)-9-[(1 R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-benzyl-3-fluoro-2-(morpholin-4-yl)-8-(trifluorométhyl)-6,7,8,9-tétrahydro-4H-pyrimido[1,2-a]pyrimidin-4-one
- (8S)-9-(3,5-difluorophényl)-2-(morpholin-4-yl)-8-(trifluorométhyl)-6,7,8,9-tétrahydro-4H-pyrimido[1,2-a]pyrimidin-4-one
- (8S)-9-[(2,6-difluorophényl)carbonyl]-2-(morpholin-4-yl)-8-(trifluorométhyl)-6,7,8,9-tétrahydro-4H-pyrimido[1,2-a]pyrimidin-4-one
- (8S)-9-[(2,4-difluorophényl)carbonyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(phénylacétyl)-8-(trifluorométhyl)-6,7,8,9-tétrahydro-4H-pyrimido[1,2-a]pyrimidin-4-one
- (8S)-9-[2-(3-chlorophényl)éthyl]-2-(morpholin-4-yl)-8-(trifluorométhyl)-6,7,8,9-tétrahydro-4H-pyrimido[1,2-a]pyrimidin-4-one
- 9-((R)-2-Benzo[b]thiophen-2-yl-2-hydroxy-ethyl)-2-morpholin-4-yl-8-(S)-trifluoromethyl-6,7,8,9-tetrahydro-pyrimido[1,2-a]pyrimidin-4-one
- 9-[(S)-2-Hydroxy-2-(3-hydroxy-phenyl)-ethyl]-2-morpholin-4-yl-8-(S)-trifluoromethyl-6,7,8,9-tetrahydro-pyrimido[1,2-a]pyrimidin-4-one
- 2-Dimethylamino-N-{3-[(S)-1-hydroxy-2-((S)-8-morpholin-4-yl-6-oxo-2-trifluoromethyl-3,4-dihydro-2H,6H-pyrimido[1,2-a]pyrimidin-1-yl)-ethyl]-phenyl}-acetamide
- 9-[(S)-2-Hydroxy-2-(2-methoxy-phenyl)-ethyl]-2-morpholin-4-yl-8-(S)-trifluoromethyl-6,7,8,9-tetrahydro-pyrimido[1,2-a]pyrimidin-4-one
- 9-[(S)-2-(4-Fluoro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(4-Chloro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(2-Chloro-4-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-(2-Hydroxy-3-phenyl-propyl)-2-morpholin-4-yl-8-(S)-trifluoromethyl-6,7,8,9-tetrahydro-pyrimido[1,2-a]pyrimidin-4-on
   9-[2-(4-Hydroxy-phenyl)-ethyl]-2-morpholin-4-yl-8-(S)-trifluoromethyl-6,7,8,9-tetrahydro-pyrimido[1,2-a]pyrimidin-4-one
   ainsi que les sels d'addition avec les acides minéraux et organiques ou avec les bases minérales et organiques pharmaceutiquement acceptables desdits produits de formule (I).

L'invention concerne aussi des compositions pharmaceutiques contenant à titre de principe actif l'un au moins des produits de formule (I) tels que définis ci-dessus ou un sel pharmaceutiquement acceptable de ce produit ou un prodrug de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

La présente invention a également pour objet l'utilisation de produits de formule (I) tels que définis ci-dessus pour la préparation d'un médicament destiné au traitement ou à la prévention d'une maladie caractérisée par le dérèglement de l'activité d'une protéine ou d'une lipide kinase.

Un tel médicament peut notamment être destiné au traitement ou à la prévention d'une maladie chez un mammifère.

La présente invention a notamment pour objet l'utilisation d'un produit de formule (I) tel que défini ci-dessus pour la préparation d'un médicament destiné à la prévention ou au traitement de différentes maladies comme les maladies cardiovasculaires incluant notamment la thrombose.

La présente invention a notamment pour objet l'utilisation d'un produit de formule (I) tel que défini ci-dessus pour la préparation d'un médicament destiné à la prévention ou au traitement de maladies liées à une prolifération non contrôlée.

La présente invention a ainsi tout particulièrement pour objet l'utilisation d'un produit de formule (I) tel que défini ci-dessus pour la préparation d'un médicament destiné au traitement ou à la prévention de maladies en oncologie et notamment destiné au traitement de cancers.

Parmi ces cancers, on s'intéresse au traitement de tumeurs solides ou liquides, au traitement de cancers résistant à des agents cytotoxiques

Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes. Sont concernées notamment les maladies présentant des anomalies génétiques aboutissant à l'activation de la voie PI3K/AKT/mTOR et/ou à l'activation de la voie MAP Kinase.

La présente invention a aussi pour objet l'utilisation des produits de formule (I) telle que définie ci-dessus pour la préparation de médicaments destinés à la chimiothérapie de cancers.

La présente invention a ainsi pour objet les produits de formule (I) tels que définis ci-dessus pour leur utilisation pour le traitement de cancers.

La présente invention a pour objet les produits de formule (I) tels que définis ci-dessus pour leur utilisation pour le traitement de tumeurs solides ou liquides.

La présente invention a donc pour objet les produits de formule (I) tels que définis ci-dessus pour leur utilisation pour le traitement de cancers résistant à des agents cytotoxiques.

La présente invention a donc pour objet les produits de formule (I) tels que définis ci-dessus pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a donc pour objet les produits de formule (I) tels que définis ci-dessus pour leur utilisation pour la chimiothérapie de cancers.

La présente invention a donc pour objet les produits de formule (I) tels que définis ci-dessus pour leur utilisation pour la chimiothérapie de cancers, seuls ou en en association.

De tels médicaments destinés à la chimiothérapie de cancers peuvent être utilisés seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

On peut notamment attendre un bénéfice thérapeutique en administrant les produits de la présente demande en combinaisons avec des thérapies ciblées variées. Ces thérapies ciblées sont notamment les suivantes : i) les thérapies inhibant la voie de signalisation MAP Kinase comme les thérapies inhibant RAS, RAF, MEK ou ERK ; ii) les thérapies ciblées inhibant les kinases ou pseudo-kinases de la voie PI3K/AKT/mTOR comme EGFR, HER2, HER3, ALK, MET, PI3K, PDK1, AKT, mTOR et S6K.

La présente invention a notamment pour objet l'utilisation d'un produit de formule (I) tel que défini ci-dessus pour la préparation d'un médicament destiné à la prévention ou au traitement de maladies lysosomales telles que la glycogénose de type II ou maladie de Pompe. De tels médicaments destinés au traitement des maladies lysosomales peuvent être utilisés seuls ou en en association par exemple avec d'autres agents thérapeutiques.

La présente invention a ainsi pour objet les produits de formule (I) tels que définis ci-dessus pour la prévention ou le traitement de maladies lysosomales telles que la glycogénose de type II ou maladie de Pompe.

La présente invention a ainsi pour objet l'utilisation des produits de formule (I) tel que défini ci-dessus pour la préparation d'un médicament destiné à la prévention ou au traitement de maladies lysosomales telles que la glycogénose de type II ou maladie de Pompe.

La présente invention a ainsi pour objet l'utilisation telle que définie ci-dessus dans laquelle lesdits produits de formule (I) sont seuls ou en association.

La présente invention a également pour objet l'utilisation d'un produit de formule (I) tel que défini ci-dessus pour la préparation d'un médicament destiné au traitement de maladies parasitaires telles que la malaria, la maladie du sommeil, la maladie de Chagas, les leishmanioses. De tels médicaments destinés au traitement des infections parasitaires peuvent être utilisés seuls ou en en association par exemple avec d'autres agents thérapeutiques.

La présente invention a ainsi pour objet les produits de formule (I) tels que définis ci-dessus pour le traitement de maladies parasitaires telles que la malaria, la maladie du sommeil, la maladie de Chagas, les leishmanioses.

La présente invention a ainsi pour objet l'utilisation des produits de formule (I) tels que définis ci-dessus pour la préparation d'un médicament pour le traitement de maladies parasitaires telles que la malaria, la maladie du sommeil, la maladie de Chagas, les leishmanioses.

La présente invention a encore pour objet à titre de produits industriels nouveaux, les intermédiaires de synthèse de formules C, D, E et J tels que définis ci-dessus et rappelés ci-après : 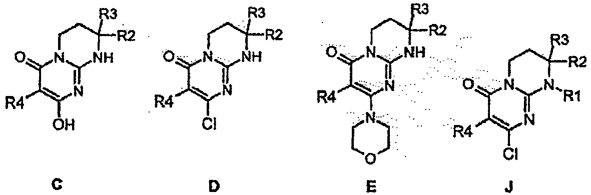 dans lesquels R1, R2, R3 et R4 ont les définitions indiquées à l'une quelconque des revendications 1 à 2.
Les exemples suivants qui sont des produits de formule (I) illustrent l'invention.

### Partie expérimentale

La nomenclature des composés de cette présente invention a été effectuée avec le logiciel ACDLABS version 10.0.

Le four à microondes utilisé est un appareil Biotage, Initiator™ 2.0, 400W max, 2450 MHz.

Les spectres de RMN ¹H à 400 MHz et ¹H à 500 MHz ont été effectués sur spectromètre BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K.

Les spectres de masse (SM) ont été obtenus soit par la méthode A, soit par la méthode B, soit par la méthode E :

### Méthode A :

Appareil WATERS UPLC-SQD ; Ionisation : électrospray en mode positif et/ou négatif (ES+/-) ; Conditions chromatographiques : Colonne : ACQUITY BEH C18 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).

### Méthode B :

Appareil WATERS ZQ ; Ionisation : électrospray en mode positif et/ou négatif (ES+/-) ; Conditions chromatographiques : Colonne : XBridge C₁₈ 2,5 µm - 3 x 50 mm ; Solvants : A : H₂O (0,1% acide formique) B : CH₃CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).

### Méthode E :

Appareil WATERS UPLC-SQD ; Ionisation : électrospray en mode positif et/ou négatif (ES+/-) ; Conditions chromatographiques : Colonne : Ascentis express C18 2,7 µm - 2,1 x 50 mm ; Solvants : A : H2O (0,02 % acide trifluorocétique) B : CH3CN (0,014 % acide trifluoroacétique) ; Température de colonne : 55 °C ; Débit : 1 ml/min ; Gradient: T0min 2%B, T1min 98%B, T1.3min 98%B, T1.33min 2%B, T1.5 min autre injection; Temps de rétention = Tr (min).

Les pouvoirs rotatoires (PR) ont été mesurés sur un polarimètre modèle 341 de chez Perkin Elmer. Longueur d'onde: raie α du sodium (589 nanomètres).

Purifications par HPLC / MS préparative :

### ■ Méthode C

Colonne de phase inverse C18 SunFire (Waters) 30 x 100, 5 µ. Gradient d'acétonitrile (+ 0.07 % TFA) dans l'eau (+ 0.07 % TFA)

| | |
|---|---|
| T0 : | 20 % acétonitrile (+ 0.07 % TFA) |
| T1 : | 20 % acétonitrile (+ 0.07 % TFA) |
| T11.5 : | 95 % acétonitrile (+ 0.07 % TFA) |
| T15 : | 95 % acétonitrile (+ 0.07 % TFA) |
| T15.5: | 20% acétonitrile (+ 0.07 % TFA) |

Débit : 30 ml/min
Masse : 130_800 UMA= ; ESP+, ESP

### ■ Méthode D

Colonne de phase inverse C18 SunFire (Waters) 30 x 100, 5 µ. Gradient d'acétonitrile (+ 0.07 % TFA) dans l'eau (+ 0.07 % TFA)

| | |
|---|---|
| T0 : | 40 % acétonitrile (+ 0.07 % TFA) |
| T1 : | 40 % acétonitrile (+ 0.07 % TFA) |
| T11 : | 95 % acétonitrile (+ 0.07 % TFA) |
| T14.5 : | 95 % acétonitrile (+ 0.07 % TFA) |
| T15 : | 10% acétonitrile (+ 0.07 % TFA) |

Débit : 30 ml/min
Masse : 130_800 UMA= ; ESP+, ESP

### Exemple 1 : (S)-9-[2-(4-méthoxy-phényl)-éthyl]-2-morpholin-4-yl-8-trifluorométhyl-6,7,8,9-tétrahydro-pyrimido[1,2-a]pyrimidin-4-one

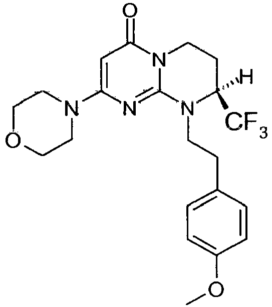

### Stade f : (S)-9-[2-(4-méthoxy-phényl)-éthyl]-2-morpholin-4-yl-8-trifluorométhyl-6,7,8,9-tétrahydro-pyrimido[1,2-a]pyrimidin-4-one

A une solution de 150 mg de la (S)-2-morpholin-4-yl-8-trifluorométhyl-6,7,8,9-tétrahydro-pyrimido[1,2-a]pyrimidin-4-one dans 5 mL de diméthylformamide anhydre sont ajoutés, à température ambiante et sous atmosphère d'argon, 0.5 g de carbonate de césium, 0.23 g du bromure de 4-méthoxyphénéthyle et 5 mg du chlorure de benzyl triéthylammonium (BTEAC). Le mélange réactionnel est chauffé à 80°C pendant 18 heures.

Après refroidissement, on ajoute au mélange obtenu 10 mL d'eau froide et 50 mL d'acétate d'éthyle. La phase organique est séparée puis séchée sur sulfate de magnésium, filtrée et concentrée sous pression réduite. Le résidu obtenu est purifié par chromatographie sur silice (gradient de 0% à 20% de l'éluant CH2Cl2/MeOH/NH4OH 28% 38/17/2 dans le dichlorométhane), pour donner 160 mg de la (S)-9-[2-(4-méthoxy-phényl)-éthyl]-2-morpholin-4-yl-8-trifluorométhyl-6,7,8,9-tétrahydro-pyrimido[1,2-a]pyrimidin-4-one dont les caractéristiques sont les suivantes :
Spectre RMN 1 H: 1,82 à 2,05 (m, 1 H) ; 2,25 à 2,39 (m, 1 H) ; 2,73 à 2,83 (m, 1 H); 2,88 à 2,99 (m, 1 H) ; 3,10 à 3,21 (m, 1 H) ; 3,34 à 3,41 (m, 1 H) ; 3,43 à 3,46 (m, 4 H) ; 3,65 (m, 4 H) ; 3,72 (s, 3 H) ; 4,03 à 4,23 (m, 2 H) ; 4,47 à 4,60 (m, 1 H) ; 4,99 (s, 1 H) ; 6,87 (d, J=8,6 Hz, 2 H) ; 7,12 (d, J=8,6 Hz, 2 H)
Spectrométrie de Masse : Méthode A
Temps de rétention Tr (min) = 0,93
[M+H]+ : m/z 439
Pouvoir rotatoire : PR= +91; C=2.426mg/0.5ML MeOH.

### Stade e : (S)-2-morpholin-4-yl-8-trifluorométhyl-6,7,8,9-tétrahydro-pyrimido[1,2-a]pyrimidin-4-one

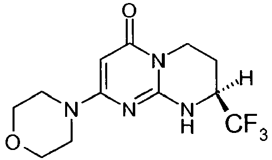

Un mélange de 1 g de la (S)-2-chloro-8-trifluorométhyl-6,7,8,9-tétrahydro-pyrimido[1,2-a]pyrimidin-4-one et de 15 mL de morpholine est chauffé à 80°C. Après une heure et demie de chauffage et après contrôle par LC/MS, la réaction est terminée. Après refroidissement, le mélange réactionnel est concentré sous pression réduite. Sur le résidu obtenu, on ajoute 10 mL d'eau froide et 100 mL d'acétate d'éthyle. La phase organique résultante est séparée puis séchée sur sulfate de magnésium, filtrée et concentrée sous pression réduite pour donner 1.2 g de la (S)-2-morpholin-4-yl-8-trifluorométhyl-6,7,8,9-tétrahydro-pyrimido[1,2-a]pyrimidin-4-one dont les caractéristiques sont les suivantes :
Spectrométrie de Masse : Méthode A
Temps de rétention Tr (min) = 0,49
[M+H]+ : m/z 305 ; [M-H]- : m/z 303
Pouvoir rotatoire : PR= +14.2+/-0.6; C=2.25mg/0.5ML MeOH.

### Stade d : (S)- 2-chloro-8-trifluorométhyl-6,7,8,9-tétrahydro-pyrimido[1,2-a]pyrimidin-4-one

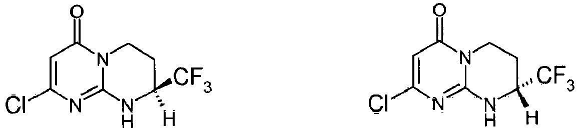

La séparation des deux énantiomères de la (R,S)-2-chloro-8-trifluorométhyl-6,7,8,9-tétrahydro-pyrimido[1,2-a]pyrimidin-4-one (17 g) est réalisée par chromatographie chirale : phase stationnaire : Chiralpak AD; phase mobile: EtOH (20%) / Heptane (80%).

L'énantiomère levogyre est concentré pour donner 8.52 g de la (R)-2-chloro-8-trifluorométhyl-6,7,8,9-tétrahydro-pyrimido[1,2-a]pyrimidin-4-one, sous forme d'une poudre blanche.

L'énantiomère dextrogyre est concentré pour obtenir 8.21 g de la (S)-2-chloro-8-trifluorométhyl-6,7,8,9-tétrahydro-pyrimido[1,2-a]pyrimidin-4-one, sous forme d'une poudre blanche, dont les caractéristiques sont les suivantes:
Spectrométrie de Masse : Méthode A
Temps de rétention Tr (min) = 0,51
[M+H]+ : m/z 254 ; [M-H]- : m/z 252
Pouvoir rotatoire : PR= +21.3+/-0.5. MeOH.

### Stade c : (R,S)-2-chloro-8-trifluorométhyl-6,7,8,9-tétrahydro-pyrimido[1,2-a]pyrimidin-4-one

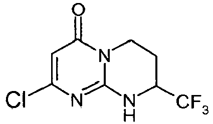

A une suspension de 34 g de la (R,S)-2-hydroxy-8-trifluorométhyl-6,7,8,9-tétrahydro-pyrimido[1,2-a]pyrimidin-4-one dans 500 mL de 1,2-dichloroéthane, sont ajoutés, à température ambiante et sous atmosphère d'argon, 60 mL d'oxychlorure de phosphore. Le mélange obtenu est alors chauffé à 65°C. Après trois heures d'agitation à 65°C, la réaction est terminée d'après le contrôle par LC/MS. Après refroidissement, le mélange réactionnel est évaporé à sec sous pression réduite. Le résidu obtenu est repris par 100 mL d'eau froide et 400 mL d'acétate d'éthyle. Sur le mélange obtenu, on additionne de la soude 32% jusqu'à pH = 6. La phase organique résultante est séparée puis séchée sur sulfate de magnésium, filtrée et concentrée sous pression réduite pour donner un résidu orange. Ce résidu est purifié par chromatographie sur silice (éluent : CH2Cl2/MeOH : 97/03) pour donner 20 g de la (R,S)-2-chloro-8-trifluorométhyl-6,7,8,9-tétrahydro-pyrimido[1,2-a]pyrimidin-4-one, sous forme d'un solide blanc, dont les caractéristiques sont les suivantes :
Spectrométrie de Masse : Méthode A
Temps de rétention Tr (min) = 0,51
[M+H]+ : m/z 254 ; [M-H]- : m/z 252

### Stade b : (R,S)-2-hydroxy-8-trifluorométhyl-6,7,8,9-tétrahydro-pyrimido[1,2-a]pyrimidin-4-one

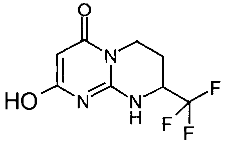

Sur un mélange de 50 mL de malonate de diéthyle sont ajoutés 10 g de chlorhydrate de 6-trifluorométhyl-1,4,5,6-tétrahydropyrimidin-2-ylamine et 10 g de méthylate de sodium. Le mélange obtenu est porté à 100°C pendant 75 minutes. Le mélange hétérogène s'épaissit et devient jaune avec un léger dégagement gazeux. Après refroidissement, le mélange réactionnel est évaporé à sec sous pression réduite. Le résidu obtenu est trituré avec de l'éther éthylique. Le solide formé est filtré sur fritté puis est repris par 20 mL d'eau froide. Sur la suspension épaisse obtenue, on additionne de l'acide chlorhydrique 12N jusqu'à pH=5-6. La suspension obtenue est filtrée sur verre fritté et l'insoluble est rincé avec de l'éther éthylique pour donner 11.5 g de la (R,S)-2-hydroxy-8-trifluoromethyl-6,7,8,9-tetrahydro-pyrimido[1,2-a]pyrimidin-4-one, sous forme d'un solide blanc, dont les caractéristiques sont les suivantes :
Spectrométrie de Masse : Méthode A
Temps de rétention Tr (min) = 0,26
[M+H]+ : m/z 236 ; [M-H]- : m/z 234

### Stade a : chlorhydrate de la (R,S)-6-trifluorométhyl-1,4,5,6-tétrahydropyrimidin-2-ylamine

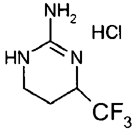

Dans un autoclave, on hydrogène, sous 3 bars, à 22 °C, pendant 24 heures, un mélange de 1.1 g de Pd/C à 10%, de 22 g de 2-amino-4-(trifluorométhyl)pyrimidine dissous dans 200 ml d'eau, 50 mL de méthanol et 50 ml d'HCl 12N.. Le mélange résultant est alors filtré et le filtrat est concentré sous pression réduite. Le résidu obtenu est séché en étuve, en présence de P2O5, pour donner 27 g du chlorhydrate de la (R,S)-6-trifluorométhyl-1,4,5,6-tétrahydropyrimidin-2-ylamine, sous forme d'un solide gris, dont les caractéristiques sont les suivantes :
Spectrométrie de Masse : Méthode A
Temps de rétention Tr (min) = 0,17
[M+H]+ : m/z 168

### Exemple 2 : (R,S)-9-[2-(4-méthoxy-phényl)-éthyl]-2-morpholin-4-yl-8-trifluorométhyl-6,7,8,9-tétrahydro-pyrimido[1,2-a]pyrimidin-4-one

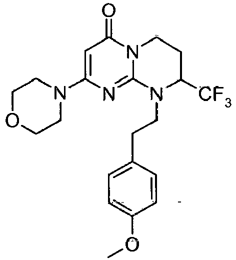

### Stade b : (R,S)-9-[2-(4-méthoxy-phényl)-éthyl]-2-morpholin-4-yl-8-trifluorométhyl-6,7,8,9-tétrahydro-pyrimido[1,2-a]pyrimidin-4-one

A une solution de 120 mg de la (R,S)-2-morpholin-4-yl-8-trifluorométhyl-6,7,8,9-tétrahydro-pyrimido[1,2-a]pyrimidin-4-one dans 5 mL de diméthylformamide anhydre sont ajoutés, à température ambiante et sous atmosphère d'argon, 0.5 g de carbonate de césium, 0.23 g du bromure de 4-methoxyphénéthyle et 10 mg du chlorure du benzyl triéthylammonium (BTEAC). Le mélange résultant est chauffé à 80°C pendant 18 heures.

Après refroidissement, le mélange réactionnel est concentré sous pression réduite. Le résidu obtenu est repris par 50 mL d'acétate d'éthyle et la solution obtenue est lavée par 3 mL d'eau. La phase organique est séparée puis séchée sur sulfate de magnésium, filtrée et évaporée sous pression réduite. Après purification par chromatographie sur silice (gradient de 0% à 20% de l'éluant CH2Cl2/MeOH/NH4OH 28% 38/17/2 dans le dichlorométhane), on obtient 80 mg de la (R,S)-(9-[2-(4-méthoxy-phényl)-éthyl]-2-morpholin-4-yl-8-trifluorométhyl-6,7,8,9-tétrahydro-pyrimido[1,2-a]pyrimidin-4-one dont les caractéristiques sont les suivantes :
Spectre RMN 1 H:
   1,85 à 2,02 (m, 1 H) ; 2,27 à 2,35 (m, 1 H) ; 2,72 à 2,84 (m, 1 H) ; 2,88 à 2,98 (m, 1 H) ; 3,09 à 3,20 (m, 1 H) ; 3,37 à 3,42 (m, 1 H) ; 3,45 (m, 4 H) ; 3,63 à 3,67 (m, 4 H) ; 3,72 (s, 3 H) ; 4,08 (m, 1 H) ; 4,14 à 4,21 (m, 1 H) ; 4,51 à 4,63 (m, 1 H) ; 5,00 (s, 1 H) ; 6,88 (d, J=8,6 Hz, 2 H) ; 7,13 (d, J=8,6 Hz, 2 H)
   Spectrométrie de Masse : Méthode A
   Temps de rétention Tr (min) = 0,93
   [M+H]+ : m/z 439

### Stade a : (R,S)-2-morpholin-4-yl-8-trifluorométhyl-6,7,8,9-tétrahydro-pyrimido[1,2-a]pyrimidin-4-one

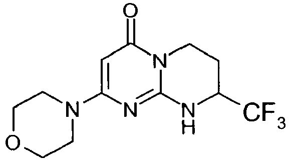

Un mélange de 220 mg de la (R,S)-2-chloro-4-yl-8-trifluorométhyl-6,7,8,9-tétrahydro-pyrimido[1,2-a]pyrimidin-4-one dans 5 mL de morpholine est chauffé à 80°C. Après une heure et demie de chauffage, la réaction est terminée d'après le contrôle par LC/MS. Après refroidissement, le mélange réactionnel est concentré sous pression réduite. Le résidu obtenu est purifié par chromatographie sur silice (gradient de 5% à 20% de l'éluant CH2Cl2/MeOH/NH4OH 28% 38/17/2 dans le dichlorométhane) pour donner 270 mg de la (R,S)-2-morpholin-4-yl-8-trifluorométhyl-6,7,8,9-tétrahydro-pyrimido[1,2-a]pyrimidin-4-one dont les caractéristiques sont les suivantes :
Spectrométrie de Masse : Méthode B
Temps de rétention Tr (min) = 2,53
[M+H]+ : m/z 305 ; [M-H]- : m/z 303

### Exemple 3 : (S)-2-morpholin-4-yl-9-phénéthyl-8-trifluorométhyl-6,7,8,9-tétrahydro-pyrimido[1,2-a]pyrimidin-4-one

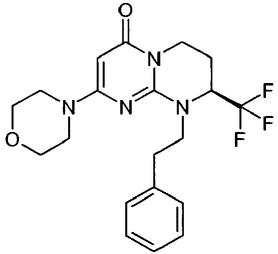

### Stade a :

La (R,S)-2-morpholin-4-yl-9-phénéthyl-8-trifluorométhyl-6,7,8,9-tétrahydro-pyrimido[1,2-a]pyrimidin-4-one est préparée en suivant le mode opératoire décrit à l'exemple 2 à partir de 80 mg de la (R,S)-2-morpholin-4-yl-8-trifluorométhyl-6,7,8,9-tétrahydro-pyrimido[1,2-a]pyrimidin-4-one et de 240 mg de (2-bromoéthyl)benzène. Après purification par chromatographie sur silice (éluant : un gradient de 5% à 20% de l'éluant CH2Cl2/MeOH/NH4OH 28% 38/17/2 dans le dichlorométhane), on obtient 75 mg de la (R,S)-2-morpholin-4-yl-9-phénéthyl-8-trifluorométhyl-6,7,8,9-tétrahydro-pyrimido[1,2-a]pyrimidin-4-one dont les caractéristiques sont les suivantes :
   Spectre RM N 1 H: 1,85 à 2,01 (m, 1 H) ; 2,27 à 2,35 (m, 1 H) ; 2,86 (m, 1 H) ; 2,95 à 3,05 (m, 1 H) ; 3,11 à 3,21 (m, 1 H) ; 3,39 à 3,49 (m, 5 H) ; 3,63 à 3,68 (m, 4 H) ; 4,08 à 4,23 (m, 2 H) ; 4,53 à 4,62 (m, 1 H) ; 5,00 (s, 1 H) ; 7,18 à 7,25 (m, 3 H) ; 7,29 à 7,34 (m, 2 H)
   Spectrométrie de Masse : Méthode A
   Temps de rétention Tr (min) = 0,95
   [M+H]+ : m/z 409

### Stade b :

La séparation des deux énantiomères de la (R,S)-2-morpholin-4-yl-9-phénéthyl-8-trifluorométhyl-6,7,8,9-tétrahydro-pyrimido[1,2-a]pyrimidin-4-one est réalisée par chromatographie chirale : colonne Chiralpak IC 20 µm; élution 75 % Heptane 20% EtOH 5 % MeOH.

L'énantiomère dextrogyre est concentré pour obtenir 27 mg de la (S)-2-morpholin-4-yl-9-phénéthyl-8-trifluorométhyl-6,7,8,9-tétrahydro-pyrimido[1,2-a]pyrimidin-4-one dont les caractéristiques sont les suivantes :
Spectre RM N 1 H: 1,94 (m, 1 H) ; 2,23 à 2,35 (m, 1 H) ; 2,83 à 2,91 (m, 1 H) ; 2,96 à 3,04 (m, 1 H) ; 3,16 (m, 1 H) ; 3,40 à 3,52 (m, 5 H) ; 3,65 (m, 4 H) ; 4,07 à 4,21 (m, 2 H) ; 4,50 à 4,62 (m, 1 H) ; 5,00 (s, 1 H) ; 7,18 à 7,25 (m, 3 H) ; 7,29 à 7,35 (m, 2 H)
Spectrométrie de Masse : Méthode A
Temps de rétention Tr (min) = 0,95
[M+H]+ : m/z 409
Pouvoir rotatoire : PR = signe positif (+40); C=1.093mg/0.5ML DMSO.

La purification chromatographique conduit aussi à 30 mg de l'énantiomère lévogyre, la (R)-2-morpholin-4-yl-9-phénéthyl-8-trifluorométhyl-6,7,8,9-tétrahydro-pyrimido[1,2-a]pyrimidin-4-one.

### Exemple 4 : (S)-9-benzyl-2-morpholin-4-yl-8-trifluorométhyl-6,7,8,9-tétrahydro-pyrimido[1,2-a]pyrimidin-4-one

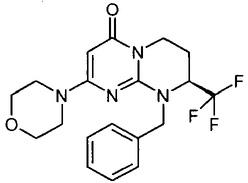

### Stade b :

La séparation des deux énantiomères du (R,S)-9-benzyl-2-morpholin-4-yl-8-trifluorométhyl-6,7,8,9-tétrahydro-pyrimido[1,2-a]pyrimidin-4-one est réalisée par chromatographie chirale : colonne 6x35cm; phase mobile : EtOH 60% Heptane 40%.

L'énantiomère dextrogyre est concentré pour donner 36 mg de la (S)-9-benzyl-2-morpholin-4-yl-8-trifluorométhyl-6,7,8,9-tétrahydro-pyrimido[1,2-a]pyrimidin-4-one dont les caractéristiques sont les suivantes :
Spectre RM N 1 H: 2,10 à 2,25 (m, 1 H) ; 2,35 à 2,43 (m, 1 H) ; 3,19 à 3,27 (m, 5 H) ; 3,39 à 3,53 (m, 4 H) ; 4,16 à 4,27 (m, 1 H) ; 4,51 (d, J=15,9 Hz, 1 H) ; 4,57 à 4,72 (m, 1 H) ; 4,96 (s, 1 H) ; 5,23 (d, J=15,9 Hz, 1 H) ; 7,20 à 7,27 (m, 3 H) ; 7,28 à 7,34 (m, 2 H)
Spectrométrie de Masse : Méthode A
Temps de rétention Tr (min) = 0,88
[M+H]+ : m/z 395
Pouvoir rotatoire : PR= +16.3+/-0.7. C=1.960mg/0.5ML DMSO.

La purification chromatographique ci-dessus conduit aussi à 38 mg de l'énantiomère lévogyre, la ((R)-9-benzyl-2-morpholin-4-yl-8-trifluorométhyl-6,7,8,9-tétrahydro-pyrimido[1,2-a]pyrimidin-4-one.

### Stade a : (R,S)-9-benzyl-2-morpholin-4-yl-8-trifluorométhyl-6,7,8,9-tétrahydro-pyrimido[1,2-a]pyrimidin-4-one

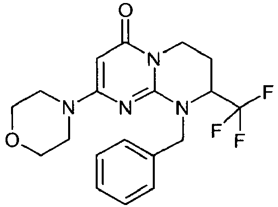

Le produit est préparé en suivant le mode opératoire décrit à l'exemple 2, à partir de 140 mg de la (R,S)-2-morpholin-4-yl-8-trifluorométhyl-6,7,8,9-tétrahydro-pyrimido[1,2-a]pyrimidin-4-one et de 0.270 mL de bromure de benzyle. Après purification par chromatographie sur silice (gradient de 5% à 20% de l'éluant CH2Cl2/MeOH/NH4OH 28% 38/17/2 dans le dichlorométhane), on obtient 32 mg du (R,S)-9-benzyl-2-morpholin-4-yl-8-trifluorométhyl-6,7,8,9-tétrahydro-pyrimido[1,2-a]pyrimidin-4-one dont les caractéristiques sont les suivantes :
Spectre RMN 1 H: 2,11 à 2,25 (m, 1 H) ; 2,35 à 2,44 (m, 1 H) ; 3,17 à 3,27 (m, 5 H) ; 3,40 à 3,53 (m, 4 H) ; 4,18 à 4,29 (m, 1 H) ; 4,51 (d, J=15,9 Hz, 1 H); 4,58 à 4,72 (m, 1 H) ; 4,96 (s, 1 H) ; 5,22 (d, J=15,9 Hz, 1 H) ; 7,20 à 7,27 (m, 3 H) ; 7,28 à 7,36 (m, 2 H)
Spectrométrie de Masse : Méthode A
Temps de rétention Tr (min) = 0,88
[M+H]+ : m/z 395

### Exemple 5: 9-((S)-2-hydroxy-2-phényl-éthyl)-2-morpholin-4-yl-8-(S)-trifluorométhyl-6,7,8,9-tétrahydro-pyrimido[1,2-a]pyrimidin-4-one

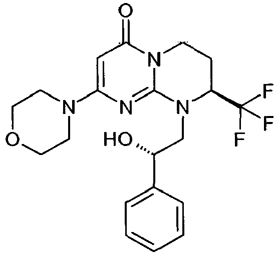

Le produit est préparé en suivant le mode opératoire décrit à l'exemple 2 à partir de 135 mg de la (R,S)-2-morpholin-4-yl-8-trifluorométhyl-6,7,8,9-tétrahydro-pyrimido[1,2-a]pyrimidin-4-one et de 342 mg de (S)-2-chloro-1-phényl-éthanol. Après purification par chromatographie sur silice (gradient de 5% à 20% de l'éluant CH2Cl2/MeOH/NH4OH 28% 38/17/2 dans le dichlorométhane), on obtient 26 mg de la 9-((S)-2-hydroxy-2-phényl-éthyl)-2-morpholin-4-yl-8-(R)-trifluorométhyl-6,7,8,9-tétrahydro-pyrimido[1,2-a]pyrimidin-4-one et 18 mg de (S)-9-((S)-2-hydroxy-2-phényl-éthyl)-2-morpholin-4-yl-8-trifluorométhyl-6,7,8,9-tétrahydro-pyrimido[1,2-a]pyrimidin-4-one dont les caractéristiques sont les suivantes :
Spectre RM N 1 H
2,24 (m, 1 H) ; 2,36 à 2,46 (m, 1 H) ; 3,08 (dd, J=10,0 et 14,2 Hz, 1 H) ; 3,17 à 3,27 (m, 1 H) ; 3,40 à 3,48 (m, 4 H) ; 3,63 à 3,69 (m, 4 H) ; 4,17 à 4,32 (m, 2 H) ; 4,74 à 4,85 (m, 1 H) ; 4,99 (m 1 H) ; 5,02 (s, 1 H) ; 5,67 (d, J=5,1 Hz, 1 H) ; 7,22 à 7,44 (m, 5 H)
Spectrométrie de Masse : Méthode A
Temps de rétention Tr (min) = 0,85
[M+H]+ : m/z 425; [M-H+HCO2H]- : m/z 469
Pouvoir rotatoire : PR= +7.4+/-0.6; C=1.959mg/0.5ML CH3OH.

### Exemple 6 : 9-((R)-2-hydroxy-2-phényl-éthyl)-2-morpholin-4-yl-8-(S)-trifluorométhyl-6,7,8,9-tétrahydro-pyrimido[1,2-a]pyrimidin-4-one

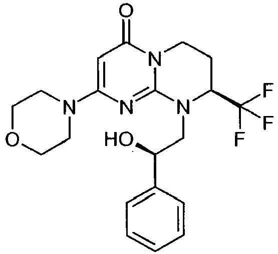

Le produit est préparé en suivant le mode opératoire décrit à l'exemple 2 à partir de 135 mg de la (R,S)-2-morpholin-4-yl-8-trifluorométhyl-6,7,8,9-tétrahydro-pyrimido[1,2-a]pyrimidin-4-one et de 342 mg (R)-2-chloro-1-phényl-éthanol. Après purification par chromatographie sur silice (gradient de 5% à 20% de l'éluant CH2Cl2/MeOH/NH4OH 28% 38/17/2 dans le dichlorométhane), on obtient 25mg du 9-((R)-2-hydroxy-2-phényl-éthyl)-2-morpholin-4-yl-8-(S)-trifluorométhyl-6,7,8,9-tétrahydro-pyrimido[1,2-a]pyrimidin-4-one dont les caractéristiques sont les suivantes :
Spectre RM N 1 H
1,40 à 1,56 (m, 1 H) ; 2,05 à 2,17 (m, 1 H) ; 3,05 à 3,20 (m, 2 H) ; 3,36 à 3,47 (m, 4 H) ; 3,62 à 3,67 (m, 4 H) ; 3,84 à 3,94 (m, 1 H) ; 3,96 à 4,03 (m, 1 H) ; 4,54 (dd, J=6,0 et 13,8 Hz, 1 H) ; 4,96 (s, 1 H) ; 4,99 à 5,04 (m, 1 H) ; 5,56 (s large, 1 H) ; 7,18 à 7,37 (m, 5 H)
Spectrométrie de Masse : Méthode A
Temps de rétention Tr (min) = 0,73
[M+H]+ : m/z 425 ; [M-H+HCO2H]- : m/z 469
Pouvoir rotatoire : PR=+63.3 +/- 1.4 dans le MeOH

Dans la purification ci-dessus, on obtient également 20 mg de 9-((R)-2-hydroxy-2-phényl-éthyl)-2-morpholin-4-yl-8-(R)-trifluorométhyl-6,7,8,9-tétrahydro-pyrimido[1,2-a]pyrimidin-4-one.

### Exemple 7 : (8S)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one

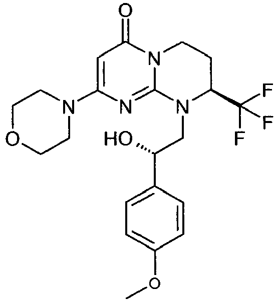

Le produit est préparé en suivant le mode opératoire décrit à l'exemple 2 à partir de 135 mg de la (R,S)-2-morpholin-4-yl-8-trifluorométhyl-6,7,8,9-tétrahydro-pyrimido[1,2-a]pyrimidin-4-one et de 760 mg de (S)-2-chloro-1-(4-méthoxy-phényl)-éthanol. Après purification par chromatographie sur silice (gradient de 5% à 20% de l'éluant CH2Cl2/MeOH/NH4OH 28% 38/17/2 dans le dichlorométhane), on obtient 66 mg de la (8S)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one dont les caractéristiques sont les suivantes:
Spectre RMN 1H :
2,14 à 2,29 (m, 1 H) ; 2,35 à 2,44 (m, 1 H) ; 3,06 (dd, J=9,8 et 13,9 Hz, 1 H) ; 3,14 à 3,27 (m, 1 H) ; 3,37 à 3,50 (m, 4 H) ; 3,62 à 3,69 (m, 4 H) ; 3,74 (s, 3 H) ; 4,14 à 4,28 (m, 2 H) ; 4,79 (m, 1 H); 4,90 à 4,98 (m, 1 H) ; 5,01 (s, 1 H) ; 5,57 (d, J=4,9 Hz, 1 H) ; 6,93 (d, J=8,6 Hz, 2 H) ; 7,25 (d, J=8,6 Hz, 2 H).
Spectrométrie de Masse : méthode A
Temps de rétention Tr (min) = 0,84
[M+H]+ : m/z 499
Pouvoir rotatoire : PR= +4; C=1.397mg/0.5ml dans CH3OH.

On obtient également 36 mg de la (8R)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one.

### Exemple 8: (8S)-2-(morpholin-4-yl)-9-[(1R ou 1S)-1-phényléthyl]-8-(trifluorométhyl)-6,7,8,9-tétrahydro-4H-pyrimido[1,2-a]pyrimidin-4-one

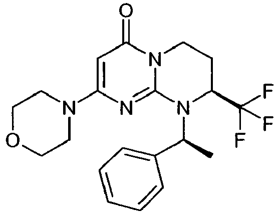

Le produit est préparé en suivant le mode opératoire décrit à l'exemple 1f, à partir de 135 mg de la (S)-2-morpholin-4-yl-8-trifluorométhyl-6,7,8,9-tétrahydro-pyrimido[1,2-a]pyrimidin-4-one (exemple 1d) dans 5 mL d'acétonitrile et de 520 mg (1-bromoéthyl)benzène. Après purification par chromatographie sur silice (gradient de 5% à 20% de l'éluant CH2Cl2/MeOH/NH4OH 28% 38/17/2 dans le dichlorométhane), on obtient 23 mg du (8S)-2-(morpholin-4-yl)-9-[1-phényléthyl]-8-(trifluorométhyl)-6,7,8,9-tétrahydro-4H-pyrimido[1,2-a]pyrimidin-4-one, sous forme d'un seul diastéréoisomère de configuration indéterminée sur la chaîne phénéthyl dont les caractéristiques sont les suivantes :
Spectre RMN 1 H : 1,65 (d, J=7,0 Hz, 3 H) ; 1,72 à 1,84 (m, 1 H) ; 2,29 à 2,38 (m, 1 H) ; 3,15 à 3,26 (m, 5 H) ; 3,43 à 3,55 (m, 4 H) ; 4,08 (m, 1 H) ; 4,31 à 4,44 (m, 1 H) ; 4,96 (s, 1 H) ; 5,67 (q, J=7,0 Hz, 1 H) ; 7,23 à 7,40 (m, 5 H).
Spectrométrie de Masse : méthode A
Temps de rétention Tr (min) = 4,07
[M+H]+ : m/z 409
Pouvoir rotatoire : PR= +54.5+/-0.6; C=1.594mg/0.5ML CH3OH.

### Exemple 9 : (8S)-9-[(1R et 1S)-1-(4-méthoxyphényl)éthyl]-2-(morpholin-4-yl)-8-(trifluorométhyl)-6,7,8,9-tétrahydro-4H-pyrimido[1,2-a]pyrimidin-4-one

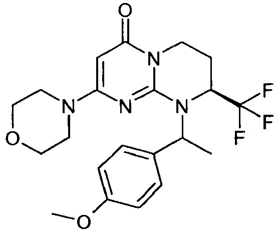

On introduit dans un ballon 130 mg de la (S)-2-chloro-8-trifluorométhyl-6,7,8,9-tétrahydro-pyrimido[1,2-a]pyrimidin-4-one (exemple 1d) dans 3 mL de tétrahydrofuranne, 250 mg de triphénylphosphine supportée sur résine (3 mmol/g) et 116 mg 1-(4-méthoxyphényl)éthanol. On ajoute alors, goutte à goutte, 0.12 mL d'azo-dicarboxylate de diéthyle. Après addition, le mélange réactionnel est agité pendant 18 heures à température ambiante. 250 mg de triphénylphosphine supportée sur résine (3 mmol/g) sont alors ajoutées au mélange réactionnel. Après 18 heures supplémentaires d'agitation à température ambiante, le mélange résultant est filtré sur Millex et le filtrat obtenu est ensuite concentré sous pression réduite.

Le résidu est dissout dans 5 mL de morpholine et le mélange résultant est chauffé à 80 °C pendant 2 heures. Le mélange réactionnel est concentré sous pression réduite. Après purification par chromatographie sur silice (gradient de 0% à 20% de l'éluant CH2Cl2/MeOH/NH4OH 28% 38/17/2 dans le dichlorométhane), on obtient 40 mg de la (8S)-9-[1-(4-méthoxyphényl)éthyl]-2-(morpholin-4-yl)-8-(trifluorométhyl)-6,7,8,9-tétrahydro-4H-pyrimido[1,2-a]pyrimidin-4-one, sous forme d'un mélange 60/40 des deux diastéréoisomères, dont les caractéristiques sont les suivantes:
Spectre RMN 1 H : 1,61 (d, J=7,1 Hz, 1,8 H) ; 1,68 (d, J=7,1 Hz, 1,2 H) ; 2,09 à 2,47 (m, 2 H) ; 3,10 à 3,65 (m partiellement masqué, 9 H) ; 3,72 (s, 1,2 H) ; 3,74 (s, 1,8 H) ; 3,85 à 4,34 (m, 1,6 H) ; 4,68 à 4,87 (m, 0,4 H) ; 4,93 (s, 0,4 H) ; 4,98 (s, 0,6 H) ; 5,47 (q, J=7,1 Hz, 0,4 H) ; 5,79 (q, J=7,1 Hz, 0,6 H) ; 6,86 (d, J=8,6 Hz, 0,8 H) ; 6,92 (d, J=8,6 Hz, 1.2 H) ; 7,26 (d, J=8,6 Hz, 2 H)
Spectrométrie de Masse : méthode A
Temps de rétention Tr (min) = 0,94 et 0,89 (mélange 60/40 des deux diastéréoisomères)
[M+H]+ : m/z 439

### Exemple 10: (8S)-9-[(1R ou 1S)-1-(4-bromophényl)éthyl]-2-(morpholin-4-yl)-8-(trifluorométhyl)-6,7,8,9-tétrahydro-4H-pyrimido[1,2-a]pyrimidin-4-one

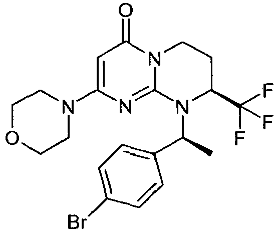

Le produit est préparé en suivant le mode opératoire décrit à l'exemple 9 à partir de 300 mg de la (S)-2-chloro-8-trifluorométhyl-6,7,8,9-tétrahydro-pyrimido[1,2-a]pyrimidin-4-one dans 6 mL de tétrahydrofuranne, de 600 mg de triphénylphosphine supportée sur résine (3 mmol/g) et de 354 mg 1-(4-bromophényl)éthanol. On ajoute alors, goutte à goutte, 0.28 mL d'azodicarboxylate de diéthyle. Après addition, le mélange réactionnel est agité pendant 18 heures à température ambiante. 600 mg de triphénylphosphine supportée sur résine (3 mmol/g) sont alors ajoutées au mélange réactionnel. Après 18 heures supplémentaires d'agitation à température ambiante, le mélange résultant est filtré sur Millex et le filtrat obtenu est ensuite concentré sous pression réduite.

Le résidu d'évaporation est dissout dans 5 mL de morpholine et le mélange obtenu est agité à température ambiante pendant 4 jours. Le mélange réactionnel est alors concentré sous pression réduite. Après purification par chromatographie sur silice (gradient de 0 à 20 % de l'éluant CH2Cl2/MeOH/NH4OH 28% 38/17/2 dans le dichlorométhane) on obtient 15 mg de l'un des diastéréoisomères de la (8S)-9-[1-(4-bromophényl)éthyl]-2-(morpholin-4-yl)-8-(trifluorométhyl)-6,7,8,9-tétrahydro-4H-pyrimido[1,2-a]pyrimidin-4-one (configuration indéterminée sur la chaîne phénéthyl) dont les caractéristiques sont les suivantes :
Spectre RMN 1H : 1,73 (d, J=7,1 Hz, 3 H) ; 2,16 à 2,31 (m, 1 H) ; 2,39 à 2,47 (m, 1 H) ; 3,12 à 3,25 (m, 5 H) ; 3,35 à 3,51 (m, 4 H) ; 4,15 (m, 1 H); 4,82 à 4,90 (m, 1 H) ; 4,92 (s, 1 H) ; 5,33 (q, J=7,1 Hz, 1 H) ; 7,27 (d, J=8,6 Hz, 2 H) ; 7,49 (d, J=8,6 Hz, 2 H).
Spectrométrie de Masse : méthode B
Temps de rétention Tr (min) = 4,26
[M+H]+ : m/z 487

### Exemple 11 : (8S)-9-[(1S ou 1R)-1-(4-bromophényl)éthyl]-2-(morpholin-4-yl)-8-(trifluorométhyl)-6,7,8,9-tétrahydro-4H-pyrimido[1,2-a]pyrimidin-4-one

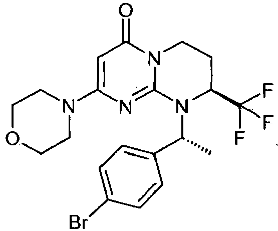

On obtient également à la suite de la purification précédente 60 mg du second diastéréoisomère de la (8S)-9-[1-(4-bromophényl)éthyl]-2-(morpholin-4-yl)-8-(trifluorométhyl)-6,7,8,9-tétrahydro-4H-pyrimido[1,2-a]pyrimidin-4-one (configuration indéterminée sur la chaîne phénéthyl) dont les caractéristiques sont les suivantes :
Spectre RMN 1 H : 1,64 (d, J=7,1 Hz, 3 H) ; 1,78 à 1,94 (m, 1 H) ; 2,30 à 2,40 (m, 1 H) ; 3,08 à 3,26 (m, 5 H) ; 3,39 à 3,52 (m, 4 H) ; 4,11 (m, 1 H) ; 4,52 (m, 1 H) ; 4,95 (s, 1 H) ; 5,50 (q, =7,1 Hz, 1 H); 7,25 (d, J=8,6 Hz, 2 H) ; 7,53 (d, J=8,6 Hz, 2 H)
Spectrométrie de Masse : méthode B
Temps de rétention Tr (min) = 4,37
[M+H]+ : m/z 487

### Exemple 12 : (8S)-2-(morpholin-4-yl)-9-phényl-8-(trifluorométhyl)-6,7,8,9-tétrahydro-4H-pyrimido[1,2-a]pyrimidin-4-one

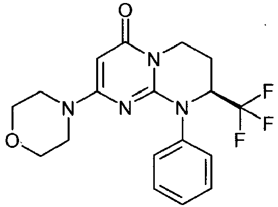

Dans un tube micro-ondes, on introduit 425 mg de la (S)-2-morpholin-4-yl-8-trifluorométhyl-6,7,8,9-tétrahydro-pyrimido[1,2-a]pyrimidin-4-one (exemple 1e) dans 1 mL de diméthylformamide, 422 mg de phosphate de tripotassium, 380 mg d'iodure de cuivre et 2 mL d'iodo benzène. Le mélange obtenu est chauffé dans un four micro-ondes, pendant 30 minutes à 150°C. On centrifuge ensuite le mélange réactionnel. Le surnageant séparé est ensuite rincé à l'acétate d'éthyle puis évaporé à sec. Le résidu est repris par de l'acétate d'éthyle et la solution obtenue est lavée avec de l'eau. La phase organique est séparée puis séchée sur sulfate de magnésium, filtrée et concentrée sous vide. Après purification par chromatographie sur silice (gradient de 5 à 15 % de l'éluant CH2Cl2/MeOH/NH4OH 28% 38/17/2 dans le dichlorométhane), on obtient 150 mg de la (8S)-2-(morpholin-4-yl)-9-phényl-8-(trifluorométhyl)-6,7,8,9-tétrahydro-4H-pyrimido[1,2-a]pyrimidin-4-one dont les caractéristiques sont les suivantes :
Spectre RMN 1 H : 2,40 à 2,48 (m, 2 H) ; 3,00 à 3,13 (m, 4 H) ; 3,31 à 3,37 (m, 1 H) ; 3,38 à 3,48 (m, 4 H) ; 4,33 à 4,40 (m, 1 H) ; 4,93 (m, 1 H) ; 4,99 (s, 1 H) ; 7,28 à 7,37 (m, 3 H) ; 7,43 (t, J=7,7 Hz, 2 H).
Spectrométrie de Masse : méthode A
Temps de rétention Tr (min) = 0,83
[M+H]+ : m/z 381

### Exemple 13 : (8S)-9-(4-fluorophényl)-2-(morpholin-4-yl)-8-(trifluorométhyl)-6,7,8,9-tétrahydro-4H-pyrimido[1,2-a]pyrimidin-4-one

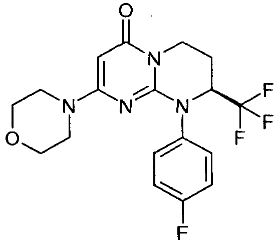

Le produit est préparé en suivant le mode opératoire décrit à l'exemple 12, à partir de 140 mg de la (S)-2-morpholin-4-yl-8-trifluorométhyl-6,7,8,9-tétrahydro-pyrimido[1,2-a]pyrimidin-4-one (exemple 1e) et de 0.66 mL de 1-fluoro-4-iodobenzène. Après purification par chromatographie sur silice (gradient de 5% à 15% de l'éluant CH2Cl2/MeOH/NH4OH 28% 38/17/2 dans le dichlorométhane), on obtient 125 mg de la (8S)-9-(4-fluorophényl)-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre RMN 1 H : 2,41 à 2,48 (m, 2 H) ; 3,01 à 3,15 (m, 4 H) ; 3,33 à 3,37 (m, 1 H) ; 3,40 à 3,50 (m, 4 H) ; 4,35 (m, 1 H) ; 4,82 à 4,94 (m, 1 H) ; 5,00 (s, 1 H) ; 7,25 (t, J=8,8 Hz, 2 H) ; 7,40 (dd, J=5,6 et 8,8 Hz, 2 H).
Spectrométrie de Masse : méthode A
Temps de rétention Tr (min) = 0,86
[M+H]+ : m/z 399

### Exemple 14 : (8S)-9-(3-fluorophényl)-2-(morpholin-4-yl)-8-(trifluorométhyl)-6,7,8,9-tétrahydro-4H-pyrimido[1,2-a]pyrimidin-4-one

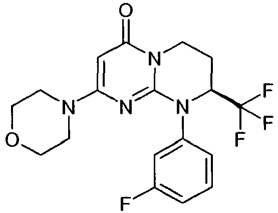

Le produit est préparé en suivant le mode opératoire décrit à l'exemple 12 à partir de 140 mg de la (S)-2-morpholin-4-yl-8-trifluorométhyl-6,7,8,9-tétrahydro-pyrimido[1,2-a]pyrimidin-4-one (exemple 1e) et de 0.66 mL de 1-fluoro-3-iodobenzène. Après purification par chromatographie sur silice (gradient de 5% à 15%de l'éluant CH2Cl2/MeOH/NH4OH 28% 38/17/2 dans le dichlorométhane), on obtient 60 mg de la (8S)-9-(3-fluorophényl)-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre RMN 1H : 2,41 à 2,48 (m, 2 H) ; 3,03 à 3,15 (m, 4 H) ; 3,25 à 3,27 (m, 1 H) ; 3,42 à 3,49 (m, 4 H) ; 4,32 à 4,41 (m, 1 H) ; 4,93 à 5,00 (m, 1 H) ; 5,02 (s, 1 H) ; 7,14 à 7,24 (m, 2 H) ; 7,30 (td, J=2,2 et 10,5 Hz, 1 H) ; 7,46 (dt, J=6,7 et 8,1 Hz, 1H).
Spectrométrie de Masse : méthode A
Temps de rétention Tr (min) = 0,86
[M+H]+ : m/z 399

### Exemple 15 : (8S)-9-(2-fluorophényl)-2-(morpholin-4-yl)-8-(trifluorométhyl)-6,7,8,9-tétrahydro-4H-pyrimido[1,2-a]pyrimidin-4-one

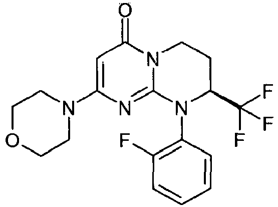

Le produit est préparé en suivant le mode opératoire décrit à l'exemple 12, à partir de 140 mg de la (S)-2-morpholin-4-yl-8-trifluorométhyl-6,7,8,9-tétrahydro-pyrimido[1,2-a]pyrimidin-4-one (exemple 1e) et de 0.66 mL de 1-fluoro-2-iodobenzène. Après purification par chromatographie sur silice (gradient de 5% à 15% de l'éluant CH2Cl2/MeOH/NH4OH 28% 38/17/2 dans le dichlorométhane), on obtient 12 mg de la (8S)-9-(2-fluorophényl)-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre RMN 1H : 2,37 à 2,47 (m, 2 H) ; 3,05 à 3,09 (m, 5 H) ; 3,41 à 3,47 (m, 4 H) ; 4,35 à 4,44 (m, 1 H) ; 4,89 (m, 1 H) ; 5,02 (s, 1 H) ; 7,24 à 7,33 (m, 2 H) ; 7,36 à 7,45 (m, 1 H) ; 7,51 (m, 1 H).
Spectrométrie de Masse : méthode A
Temps de rétention Tr (min) = 0,83
[M+H]+ : m/z 399

### Exemple 16 : (8S)-9-[(1R ou 1S)-1-(3-fluorophényl)éthyl]-2-(morpholin-4-yl)-8-(trifluorométhyl)-6,7,8,9-tétrahydro-4H-pyrimido[1,2-a]pyrimidin-4-one

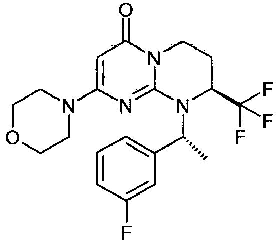

### Stade b :

Dans un ballon, on chauffe à 80°C pendant 30 minutes un mélange de 300 mg de la (S)-2-chloro-1-[1-(3-fluoro-phényl)-éthyl]-8-trifluorométhyl-1,6,7,8-tétrahydro-pyrimido[1,2-a]pyrimidin-4-one et de 3 mL de morpholine. Après refroidissement, le mélange réactionnel est concentré sous pression réduite. Le résidu est repris par de l'acétate d'éthyle et de l'eau. La phase organique est séparée puis séchée sur sulfate de magnésium, filtrée et concentrée sous pression réduite. Après purification par chromatographie sur silice (éluant : CH2Cl2/MeOH 97.5/2.5), on obtient 152 mg de la (8S)-9-[(1-(3-fluorophényl)éthyl]-2-(morpholin-4-yl)-8-(trifluorométhyl)-6,7,8,9-tétrahydro-4H-pyrimido[1,2-a]pyrimidin-4-one, sous forme d'un seul diastéréoisomère (configuration indéterminée au niveau de la chaîne phénéthyl) dont les caractéristiques sont les suivantes :
Spectre RMN 1 H :
   1,66 (d, J=7,0 Hz, 3 H) ; 1,80 à 1,95 (m, 1 H) ; 2,33 à 2,41 (m, 1 H) ; 3,09 à 3,28 (m, 5 H) ; 3,40 à 3,52 (m, 4 H) ; 4,06 à 4,16 (m, 1 H) ; 4,56 (m, 1 H) ; 4,95 (s, 1 H) ; 5,51 (q, J=7,0 Hz, 1 H) ; 7,00 à 7,17 (m, 3 H) ; 7,33 à 7,42 (m, 1 H).
   Spectrométrie de Masse : méthode A
   Temps de rétention Tr (min) = 0,94
   [M+H]+ : m/z 427

### Stade a :

Dans un ballon, on introduit 400 mg de la (S)-2-chloro-8-trifluorométhyl-6,7,8,9-tétrahydro-pyrimido[1,2-a]pyrimidin-4-one (exemple 1d) dans 20 mL de tétrahydrofuranne, 1.6 g de triphénylphosphine supportée sur résine (3 mmol/g) et 663 mg 1-(3-fluorophényl)éthanol. Le mélange réactionnel est alors agité à température ambiante pendant 5 minutes avant ajout de 0.790 mL de l'azodicarboxylate de diéthyle. Après 1 heure d'agitation à température ambiante, le mélange réactionnel est filtré et le filtrat concentré sous pression réduite.

Le résidu est repris par de l'acétate d'éthyle et de l'eau. La phase organique est séparée puis séchée sur sulfate de magnésium, filtrée et concentrée sous pression réduite. Après purification par chromatographie sur silice (éluant CH2Cl2/AcOEt 96/04), on obtient 150 mg de la (S)-2-chloro-1-[1-(3-fluoro-phényl)-éthyl]-8-trifluorométhyl-1,6,7,8-tétrahydro-pyrimido[1,2-a]pyrimidin-4-one, sous forme d'un seul diastéréoisomère (configuration indéterminée au niveau de la chaîne phénéthyl) dont les caractéristiques sont les suivantes:
Spectrométrie de Masse : méthode A
Temps de rétention Tr (min) = 1,11
[M+H]+ : m/z 376 ; [M-H]- : m/z 253 (pic de base)

### Exemple 17: (8S)-9-(4-fluorobenzyle)-2-(morpholin-4-yl)-8-(trifluorométhyl)-6,7,8,9-tétrahydro-4H-pyrimido[1,2-a]pyrimidin-4-one

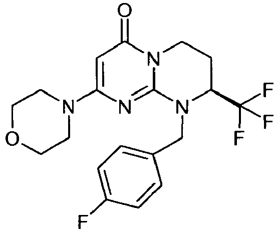

A une solution de 300 mg de la 2(S)-2-morpholin-4-yl-8-trifluorométhyl-6,7,8,9-tétrahydro-pyrimido[1,2-a]pyrimidin-4-one (exemple 1e) dans 3 mL d'acétonitrile, sont ajoutés, sous atmosphère d'argon, 0.536 g de carbonate de césium et 0.44 mL de 1-(bromométhyl)-4-fluorobenzène. Le mélange résultant est alors chauffé à 80°C durant deux heures. Le mélange réactionnel est alors évaporé sous pression réduite et le résidu obtenu est ensuite purifié par chromatographie sur silice (éluant : CH2Cl2/MeOH 98/02) pour donner 61 mg de la (8S)-9-(4-fluorobenzyl)-2-(morpholin-4-yl)-8-(trifluorométhyl)-6,7,8,9-tétrahydro-4H-pyrimido[1,2-a]pyrimidin-4-one, sous forme d'un solide blanc, dont les caractéristiques sont les suivantes :
Spectre RM N 1 H : 2,09 à 2,25 (m, 1 H) ; 2,34 à 2,44 (m, 1 H) ; 3,18 à 3,32 (m, 5 H) ; 3,41 à 3,53 (m, 4 H) ; 4,21 (m, 1 H) ; 4,51 (d, J=15,2 Hz, 1 H) ; 4,60 à 4,72 (m, 1 H) ; 4,96 (s, 1 H) ; 5,17 (d, J=15,2 Hz, 1 H) ; 7,13 (t, J=8,7 Hz, 2 H) ; 7,30 (dd, J=5,4 et 8,7 Hz, 2 H)
Spectrométrie de Masse : Methode A
Temps de rétention Tr (min) = 0,89
[M+H]+ : m/z 413

### Exemple 18: (S)-9-Benzoyl-2-morpholin-4-yl-8-trifluorométhyl-6,7,8,9-tétrahydro-pyrimido[1,2-a]pyrimidin-4-one

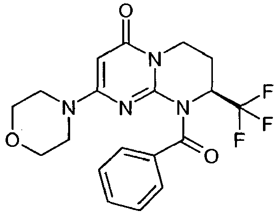

A une solution de 300 mg de la 2(S)-2-morpholin-4-yl-8-trifluorométhyl-6,7,8,9-tétrahydro-pyrimido[1,2-a]pyrimidin-4-one (exemple 1e) dans 3 mL de tétrahydrofuranne, sont ajoutés, sous atmosphère d'argon, 35.7 mg d'hydrure de sodium puis, après 10 minutes d'agitation, 0.135 mL de chlorure de benzoyle. Après six heures d'agitation à température ambiante, on ajoute au mélange réactionnel une solution saturée en bicarbonate de sodium et de l'acétate d'éthyle. La phase organique est séparée puis successivement lavée avec une solution saturée en chlorure de sodium, séchée sur sulfate de magnésium, filtrée et concentrée sous pression réduite. Le résidu obtenu est purifié par chromatographie sur silice (éluant : CH2Cl2) pour donner 74 mg de la (S)-9-benzoyl-2-morpholin-4-yl-8-trifluorométhyl-6,7,8,9-tétrahydro-pyrimido[1,2-a]pyrimidin-4-one, sous forme de solide blanc, dont les caractéristiques sont les suivantes :
Spectre RMN 1 H : 2,20 à 2,31 (m, 1 H) ; 2,68 à 2,82 (m, 3 H) ; 2,86 à 2,96 (m, 2 H) ; 3,15 à 3,44 (m partiellement masqué, 4 H) ; 3,75 à 3,87 (m, 1 H) ; 4,17 à 4,30 (m, 1 H) ; 5,15 (s, 1 H) ; 5,38 à 5,53 (m, 1 H) ; 7,37 à 7,43 (m, 2 H) ; 7,45 à 7,53 (m, 3 H).
Spectrométrie de Masse : méthode B
Temps de rétention Tr (min) = 3,59
[M+H]+ : m/z 409
Pouvoir rotatoire : PR= -15.8+/-0.8; C= 1.650mg/0.5ML DMSO.

### Exemple 19 : (S)-2-morpholin-4-yl-9-pyridin-3-yl-8-trifluorométhyl-6,7,8,9-tétrahydro-pyrimido[1,2-a]pyrimidin-4-one

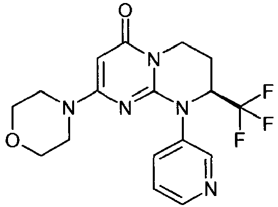

Le produit est préparé en suivant le mode opératoire décrit à l'exemple 12, à partir de 200 mg de la (S)-2-morpholin-4-yl-8-trifluorométhyl-6,7,8,9-tétrahydro-pyrimido[1,2-a]pyrimidin-4-one (exemple 1e) et de 380 mg de 3-iodopyridine. Après purification par chromatographie sur silice (gradient d'élution de CH2Cl2 à CH2Cl2/MeOH 96/04), on obtient 48 mg de la (S)-2-morpholin-4-yl-9-pyridin-3-yl-8-trifluorométhyl-6,7,8,9-tétrahydro-pyrimido[1,2-a]pyrimidin-4-one, sous forme de solide jaune, dont les caractéristiques sont les suivantes :
Spectre RMN 1H : 2,44 à 2,48 (m, 2 H) ; 3,00 à 3,13 (m, 4 H) ; 3,31 à 3,37 (m, 1 H) ; 3,41 à 3,47 (m, 4 H) ; 4,37 (d, J=16,1 Hz, 1 H) ; 4,97 à 5,09 (m, 2 H) ; 7,48 (dd, J=4,9 et 8,3 Hz, 1 H) ; 7,80 à 7,85 (m, 1 H) ; 8,50 (dd, J=1,4 et 4,9 Hz, 1 H) ; 8,57 (d, J=2,2 Hz, 1 H).
Spectrométrie de Masse : méthode A
Temps de rétention Tr (min) = 0,55
[M+H]+ : m/z 382
Pouvoir rotatoire : PR=-40 +/-1.6, C=0.2% dans le DMSO.

### Exemple 20 : (S)-2-morpholin-4-yl-9-pyridin-4-yl-8-trifluorométhyl-6,7,8,9-tétrahydro-pyrimido[1,2-a]pyrimidin-4-one SAR236152

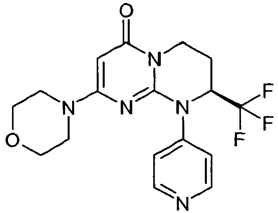

Le produit est préparé en suivant le mode opératoire décrit à l'exemple 12, à partir de 200 mg de la (S)-2-morpholin-4-yl-8-trifluorométhyl-6,7,8,9-tétrahydro-pyrimido[1,2-a]pyrimidin-4-one (exemple 1e) et de 380 mg de 4-iodopyridine. Après purification par chromatographie sur silice (gradient d'élution de CH2Cl2 à CH2Cl2/MeOH 96/04), on obtient 26 mg de la (S)-2-morphol in-4-yl-9-pyridin-4-yl-8-trifluorométhyl-6,7,8,9-tétrahydro-pyrimido[1,2-a]pyrimidin-4-one, sous forme de solide jaune, dont les caractéristiques sont les suivantes :
Spectre RMN 1H : 2,43 à 2,48 (m, 2 H) ; 3,07 à 3,19 (m, 4 H) ; 3,33 à 3,39 (m, 1 H) ; 3,45 à 3,50 (m, 4 H) ; 4,30 à 4,38 (m, 1 H) ; 5,06 (s, 1 H) ; 5,09 à 5,17 (m, 1 H) ; 7,45 (d, J=6,1 Hz, 2 H) ; 8,62 (d, J=6,1 Hz, 2 H).
Spectrométrie de Masse : méthode A
[M+H]+ : m/z 382 ; [M-H]- : m/z 380
Temps de rétention Tr (min) = 0,42
Pouvoir rotatoire : PR=-31 +/- 1.3, C=0.2% dans le DMSO.

### Exemple 21: (8S)-9-(4-méthylphényl)-2-(morpholin-4-yl)-8-(trifluorométhyl)-6,7,8,9-tétrahydro-4H-pyrimido[1,2-a]pyrimidin-4-one

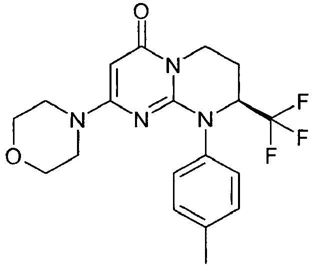

Le produit est préparé en suivant le mode opératoire décrit à l'exemple 12, à partir de 100 mg de la (8S)-2-morpholin-4-yl-8-trifluorométhyl-6,7,8,9-tétrahydro-pyrimido[1,2-a]pyrimidin-4-one (exemple 1e) et de 100 mg de 1-iodo-4-méthylbenzène. Après purification par chromatographie sur silice (éluant: CH2Cl2/MeOH 98/02), on obtient 23 mg de la (8S)-9-(4-méthylphényl)-2-(morpholin-4-yl)-8-(trifluorométhyl)-6,7,8,9-tétrahydro-4H-pyrimido[1,2-a]pyrimidin-4-one, sous forme de solide crème, dont les caractéristiques sont les suivantes :
Spectre RMN 1 H : 2,32 (s, 3 H) ; 2,44 (m, 2 H) ; 2,97 à 3,15 (m, 4 H) ; 3,33 à 3,50 (m partiellement masqué, 5 H) ; 4,34 (m, 1 H) ; 4,86 (m, 1 H) ; 4,98 (s, 1 H) ; 7,22 (s, 4 H).
Spectrométrie de Masse : méthode A
Temps de rétention Tr (min) = 0,90
[M+H]+ : m/z 395

### Exemple 22 : (8S)-9-(2-chlorobenzyl)-2-(morpholin-4-yl)-8-(trifluorométhyl)-6,7,8,9-tétrahydro-4H-pyrimido[1,2-a]pyrimidin-4-one

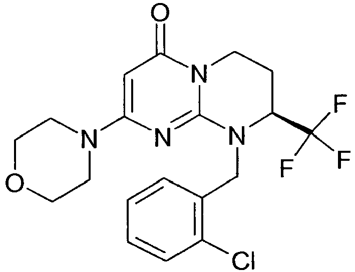

A une solution de 100 mg de la (8S)-2-(morpholin-4-yl)-8-(trifluorométhyl)-6,7,8,9-tétrahydro-4H-pyrimido[1,2-a]pyrimidin-4-one (exemple 1e) dans 2 mL de diméthylformamide sont ajoutés 214 mg de carbonate de césium et 74 mg de 1-(bromométhyl)-2-chlorobenzène. Après 16 heures à une température voisine de 20°C, le milieu réactionnel est versé dans de l'eau. La phase organique est séparée et la phase aqueuse est extraite par de l'acétate d'éthyle. Les phases organiques rassemblées sont concentrées à sec sous pression réduite. Le résidu est purifié par HPLC/MS préparative (Méthode C). Après évaporation de l'acétonitrile et lyophilisation, on obtient 94 mg de (8S)-9-(2-chlorobenzyl)-2-(morpholin-4-yl)-8-(trifluorométhyl)-6,7,8,9-tétrahydro-4H-pyrimido[1,2-a]pyrimidin-4-one, sous forme d'une huile dont les caractéristiques, sont les suivantes :
Spectre RM N 1 H : 2,29 à 2,66 (m, 2 H) ; 3,09 à 3,18 (m, 4 H) ; 3,23 à3,50 (m partiellement masqué, 5 H) ; 4,27 (m, 1 H) ; 4,68 (d, J=16,6 Hz, 1 H) ; 4,74 (m, 1 H) ; 4,96 (s, 1 H); 5,12 (d, J=16,6 Hz, 1 H) ; 7,16 à 7,22 (m, 1 H) ; 7,24 à 7,32 (m, 2 H) ; 7,41 à 7,48 (m, 1 H).
Spectrométrie de Masse : méthode A
Temps de rétention Tr (min) = 0,96
[M+H]+ : m/z 429

### Exemple 23 : (8S)-9-(3-fluorobenzyl)-2-(morpholin-4-yl)-8-(trifluorométhyl)-6,7,8,9-tétrahydro-4H-pyrimido[1,2-a]pyrimidin-4-one

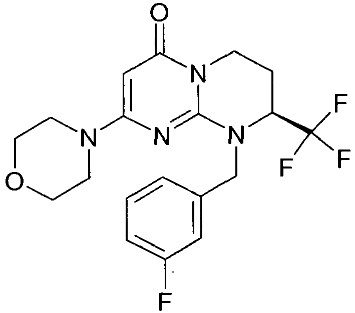

Le produit est préparé en suivant le mode opératoire décrit à l'exemple 22, à partir de 100 mg de la (8S)-2-(morpholin-4-yl)-8-(trifluorométhyl)-6,7,8,9-tétrahydro-4H-pyrimido[1,2-a]pyrimidin-4-one, de 214 mg de carbonate de césium et de 68 mg de 1-(bromométhyl)-3-fluorobenzène. Après purification par HPLC/MS préparative (Méthode C), on obtient 102 mg de la (8S)-9-(3-fluorobenzyl)-2-(morpholin-4-yl)-8-(trifluorométhyl)-6,7,8,9-tétrahydro-4H-pyrimido[1,2-a]pyrimidin-4-one, sous la forme d'une huile, dont les caractéristiques sont les suivantes :
Spectre RMN 1 H : 2,17 à 2,45 (m, 2 H) ; 3,15 à 3,31 (m, 5 H) ; 3,39 à 3,49 (m, 4 H) ; 4,23 (m, 1 H) ; 4,58 (d, J=16,1 Hz, 1 H) ; 4,66 à 4,78 (m, 1 H) ; 4,97 (s, 1 H) ; 5,13 (d, J=16,1 Hz, 1 H) ; 6,98 à 7,15 (m, 3 H); 7,35 (dt, J=6,0 et 8,1 Hz, 1 H)
Spectrométrie de Masse : méthode A
Temps de rétention Tr (min) = 0,89
[M+H]+ : m/z 413

### Exemple 24 : (8S)-9-[2-(2-méthoxyphényl)éthyl]-2-(morpholin-4-yl)-8-(trifluorométhyl)-6,7,8,9-tétrahydro-4H-pyrimido[1,2-a]pyrimidin-4-one

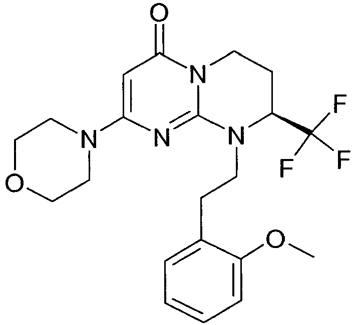

A une solution de 100 mg de la (8S)-2-(morpholin-4-yl)-8-(trifluorométhyl)-6,7,8,9-tétrahydro-4H-pyrimido[1,2-a]pyrimidin-4-one (exemple 1 e) dans 2 mL de diméthylformamide sont ajoutés 214 mg de carbonate de césium et 78 mg de 1-(2-bromoéthyl)-3-méthoxybenzène. Après 18 heures à température de 60°C, on rajoute 78 mg de 1-(2-bromoéthyl)-3-fluorobenzène. Après 2 jours à une température de 60°C, le mélange réactionnel obtenu est versé dans de l'eau. La phase organique est séparée et la phase aqueuse est extraite par de l'acétate d'éthyle. Les phases organiques rassemblées sont concentrées à sec sous pression réduite. Le résidu est purifié par HPLC/MS préparative (Méthode C). Après évaporation de l'acétonitrile et lyophilisation, on obtient 25 mg de la (8S)-9-[2-(2-méthoxyphényl)éthyl]-2-(morpholin-4-yl)-8-(trifluorométhyl)-6,7,8,9-tétrahydro-4H-pyrimido[1,2-a]pyrimidin-4-one, sous forme d'une huile, dont les caractéristiques sont les suivantes :
Spectre RMN 1 H : 1,84 à 2,00 (m, 1 H) ; 2,25 à 2,35 (m, 1 H) ; 2,83 à 3,03 (m, 2 H) ; 3,06 à 3,30 (m, 2 H) ; 3,44 (m, 4 H) ; 3,61 à 3,67 (m, 4 H) ; 3,76 (s, 3 H) ; 4,08 à 4,24 (m, 2H) ; 4,37 à 4,52 (m, 1 H) ; 4,99 (s, 1 H) ; 6,88 (dt, J=0,9 et 7,6 Hz, 1 H) ; 6,96 (d large, J=7,9 Hz, 1 H) ; 7,14 (dd, J=1,5 et 7,6 Hz, 1 H) ; 7,18 à 7,26 (m, 1 H)
Spectrométrie de Masse : méthode A
Temps de rétention Tr (min) = 0,98
[M+H]+ : m/z 439

### Exemple 25 :(8S)-9-[2-(3-méthoxyphényl)éthyl]-2-(morpholin-4-yl)-8-(trifluorométhyl)-6,7,8,9-tétrahydro-4H-pyrimido[1,2-a]pyrimidin-4-one

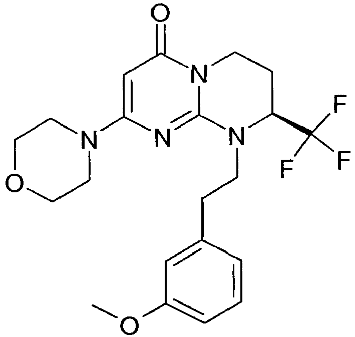

Le produit est préparé en suivant le mode opératoire décrit à l'exemple 24, mais à partir de 100 mg de la (8S)-2-(morpholin-4-yl)-8-(trifluorométhyl)-6,7,8,9-tétrahydro-4H-pyrimido[1,2-a]pyrimidin-4-one, de 78 mg de 1-(2-bromoéthyl)-3-méthoxybenzène et de 214 mg de carbonate de césium dans 2 mL de diméthylformamide. Après 3 jours de réaction à une température de 60°C, traitement comme décrit à l'exemple 24 et purification par HPLC/MS préparative (Méthode C), on obtient 20 mg de la (8S)-9-[2-(3-méthoxyphényl)éthyl]-2-(morpholin-4-yl)-8-(trifluorométhyl)-6,7,8,9-tétrahydro-4H-pyrimido[1,2-a]pyrimidin-4-one, sous la forme d'une huile, dont les caractéristiques sont les suivantes :
Spectre RMN 1 H : 1,88 à 2,01 (m, 1 H) ; 2,32 (m, 1 H) ; 2,74 à 2,90 (m, 1 H) ; 2,96 (m, 1 H) ; 3,10 à 3,22 (m, 1 H) ; 3,26 à 3,39 (m partiellement masqué, 1 H) ; 3,43 à 3,48 (m, 4 H) ; 3,62 à 3,68 (m, 4 H) ; 3,73 (s, 3 H) ; 4,02 à 4,23 (m, 2 H) ; 4,49 à 4,65 (m, 1 H) ; 5,00 (s, 1 H) ; 6,70 à 6,88 (m, 3 H) ; 7,14 à 7,26 (m, 1 H)
Spectrométrie de Masse : méthode A
Temps de rétention Tr (min) = 0,94
[M+H]+ : m/z 439

### Exemple 26 : (8S)-9-(3-méthoxybenzyl)-2-(morpholin-4-yl)-8-(trifluorométhyl)-6,7,8,9-tétrahydro-4H-pyrimido[1,2-a]pyrimidin-4-one

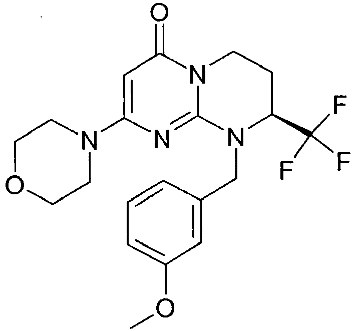

Le produit est préparé en suivant le mode opératoire décrit à l'exemple 22, mais à partir de 100 mg de la (8S)-2-(morpholin-4-yl)-8-(trifluorométhyl)-6,7,8,9-tétrahydro-4H-pyrimido[1,2-a]pyrimidin-4-one, de 57 mg de 1-(chlorométhyl)-3-méthoxybenzène et de 214 mg de carbonate de césium dans 2 mL de diméthylformamide. Après traitement, le résidu est agité dans de l'acétonitrile. Le solide est essoré, rincé par de l'oxyde de diéthyle puis séché sous cloche à vide. On obtient ainsi 111 mg de (8S)-9-(3-méthoxybenzyl)-2-(morpholin-4-yl)-8-(trifluorométhyl)-6,7,8,9-tétrahydro-4H-pyrimido[1,2-a]pyrimidin-4-one, sous forme d'un solide blanc, dont les caractéristiques sont les suivantes :
Spectre RMN 1 H : 2,07 à 2,23 (m, 1 H) ; 2,35 à 2,44 (m, 1 H) ; 3,19 à 3,28 (m, 5 H) ; 3,40 à 3,55 (m, 4 H) ; 3,72 (s, 3 H) ; 4,22 (m, 1 H) ; 4,45 (d, J=15,9 Hz, 1 H) ; 4,56 à 4,70 (m, 1 H) ; 4,96 (s, 1 H) ; 5,21 (d, J=15,9 Hz, 1 H) ; 6,75 à 6,88 (m, 3 H) ; 7,19 à 7,27 (m, 1 H)
Spectrométrie de Masse : méthode A
Temps de rétention Tr (min) = 0,88
[M+H]+ : m/z 425

### Exemple 27 : (8S)-9-(4-méthoxyphényl)-2-(morpholin-4-yl)-8-(trifluorométhyl)-6,7,8,9-tétrahydro-4H-pyrimido[1,2-a]pyrimidin-4-one

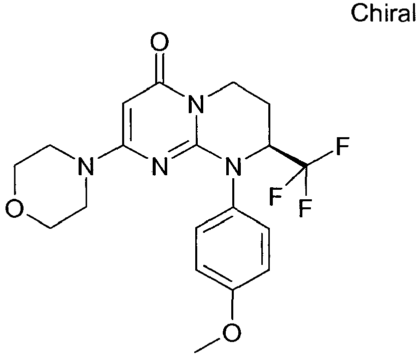

Le produit est préparé en suivant le mode opératoire décrit à l'exemple 12, à partir de 100 mg de la (8S)-2-morpholin-4-yl-8-trifluorométhyl-6,7,8,9-tétrahydro-pyrimido[1,2-a]pyrimidin-4-one (exemple 1e), de 108 mg de 1-iodo-4-méthoxybenzène et de 79 mg de 4,7-diméthoxy-1,10-phénanthroline. Après purification par chromatographie sur silice (éluant : CH2Cl2/MeOH 98/02), on obtient 31 mg de (8S)-9-(4-méthoxyphényl)-2-(morpholin-4-yl)-8-(trifluorométhyl)-6,7,8,9-tétrahydro-4H-pyrimido[1,2-a]pyrimidin-4-one, sous forme d'une meringue crème, dont les caractéristiques sont les suivantes :
Spectre RMN 1 H : 2,38 à 2,46 (m, 2 H) ; 3,04 à 3,15 (m, 4 H) ; 3,20 à 3,35 (m partiellement masqué, 1 H) ; 3,45 (m, 4 H) ; 3,77 (s, 3 H) ; 4,34 (m, 1 H) ; 4,83 (m, 1 H) ; 4,98 (s, 1 H) ; 6,95 (d, J=8,8 Hz, 2 H) ; 7,25 (d, J=8,8 Hz, 2 H)
Spectrométrie de Masse : méthode A
Temps de rétention Tr (min) = 0,83
[M+H]+ : m/z 411

### Exemple 28 : (8S)-9-[(2-fluorophényl)carbonyl]-2-(morpholin-4-yl)-8-(trifluorométhyl)-6,7,8,9-tétrahydro-4H-pyrimido[1,2-a]pyrimidin-4-one

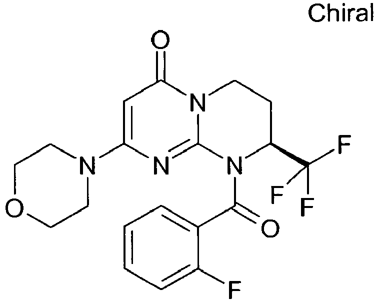

Le produit est préparé en suivant le mode opératoire décrit à l'exemple 18, à partir de 300 mg de la (8S)-2-morpholin-4-yl-8-trifluorométhyl-6,7,8,9-tétrahydro-pyrimido[1,2-a]pyrimidin-4-one (exemple 1e), de 47 mg d'hydrure de sodium et de 156 mg de chlorure de 2-fluorobenzoyle dans 5 mL de tétrahydrofuranne. Après purification par chromatographie sur silice (éluant : CH2Cl2/MeOH 98/02), on obtient 35mg de la (8S)-9-[(2-fluorophényl)carbonyl]-2-(morpholin-4-yl)-8-(trifluorométhyl)-6,7,8,9-tétrahydro-4H-pyrimido[1,2-a]pyrimidin-4-one, sous forme d'une meringue blanche, dont les caractéristiques sont les suivantes :
Spectre RM N 1H : 2,13 à 2,26 (m, 1 H) ; 2,65 à 2,85 (m, 3 H) ; 2,94 à 3,02 (m, 2 H) ; 3,20 à 3,40 (m partiellement masqué, 4 H) ; 3,43 à 3,55 (m, 1 H) ; 4,42 (m, 1 H) ; 5,19 (s, 1 H) ; 5,55 à 5,69 (m, 1 H) ; 7,18 à 7,31 (m, 2 H) ; 7,49 à 7,64 (m, 2 H)
Spectrométrie de Masse : méthode A
Temps de rétention Tr (min) = 0,79
[M+H]+ : m/z 427

### Exemple 29 : (8S)-9-(3,5-difluorobenzyl)-2-(morpholin-4-yl)-8-(trifluorométhyl)-6,7,8,9-tétrahydro-4H-pyrimido[1,2-a]pyrimidin-4-one

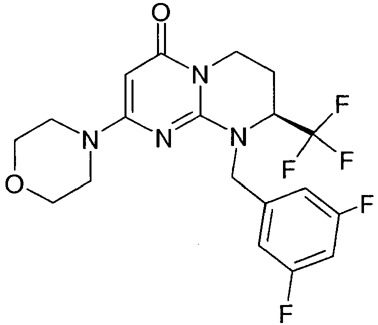

Le produit est préparé en suivant le mode opératoire décrit à l'exemple 22, à partir de 100 mg de la (8S)-2-morpholin-4-yl-8-trifluorométhyl-6,7,8,9-tétrahydro-pyrimido[1,2-a]pyrimidin-4-one (exemple 1 e), de 214 mg de carbonate de césium et de 75 mg de 1-(bromométhyl)-3,5-difluorobenzène dans 2 mL de diméthylformamide. Après purification par HPLC/MS préparative (Méthode C), on obtient 85 mg de la (8S)-9-(3,5-difluorobenzyl)-2-(morpholin-4-yl)-8-(trifluorométhyl)-6,7,8,9-tétrahydro-4H-pyrimido[1,2-a]pyrimidin-4-one, sous forme d'une huile, dont les caractéristiques sont les suivantes :
Spectre RMN 1H : 2,21 à 2,44 (m, 2 H) ; 3,14 à 3,33 (m, 5 H) ; 3,36 à 3,52 (m, 4 H) ; 4,23 (m, 1 H) ; 4,61 (d, J=16,4 Hz, 1 H) ; 4,68 à 4,81 (m, 1 H) ; 4,98 (s, 1 H) ; 5,07 (d, J=16,4 Hz, 1 H) ; 6,90 à 7,02 (m, 2 H) ; 7,07 (tt, J=2,3 et 9,3 Hz, 1 H)
Spectrométrie de Masse : méthode B
Temps de rétention Tr (min) = 3,93
[M+H]+ : m/z 431

### Exemple 30 : (8S)-9-(2,4-difluorobenzyl)-2-(morpholin-4-yl)-8-(trifluorométhyl)-6,7,8,9-tétrahydro-4H-pyrimido[1,2-a]pyrimidin-4-one

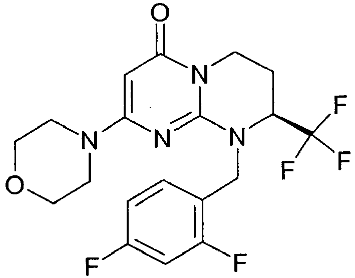

Le produit est préparé en suivant le mode opératoire décrit à l'exemple 22, à partir de 100 mg de la (8S)-2-morpholin-4-yl-8-trifluorométhyl-6,7,8,9-tétrahydro-pyrimido[1,2-a]pyrimidin-4-one (exemple 1e), de 214 mg de carbonate de césium et de 75 mg de 1-(bromométhyl)-2,4-difluorobenzène dans 2 mL de diméthylformamide. Après purification par HPLC/MS préparative (Méthode C), on obtient 86 mg de la (8S)-9-(2,4-difluorobenzyl)-2-(morpholin-4-yl)-8-(trifluorométhyl)-6,7,8,9-tétrahydro-4H-pyrimido[1,2-a]pyrimidin-4-one, sous forme d'une huile, dont les caractéristiques sont les suivantes :
Spectre RMN 1H : 2,16 à 2,30 (m, 1 H) ; 2,35 à 2,45 (m, 1 H) ; 3,17 à 3,32 (m, 5 H) ; 3,41 à 3,49 (m, 4 H) ; 4,22 (dd, J=5,9 et 14,2 Hz, 1 H) ; 4,60 (d, J=16,1 Hz, 1 H) ; 4,66 à 4,76 (m, 1 H) ; 4,97 (s, 1 H) ; 5,11 (d, J=16,1 Hz, 1 H) ; 7,03 (ddt, J=1,1 - 2,6 et 9,0 Hz,1 H) ; 7,22 (ddd, J=2,6 - 9,0 et 10,9 Hz,1 H) ; 7,30 (dt, J=6,7 et 9,0 Hz, 1 H)
Spectrométrie de Masse : méthode B
Temps de rétention Tr (min)= 3,96
[M+H]+ : m/z 431

### Exemple 31 : (8S)-2-(morpholin-4-yl)-9-(2,3,4-trifluorobenzyl)-8-(trifluorométhyl)-6,7,8,9-tétrahydro-4H-pyrimido[1,2-a]pyrimidin-4-one

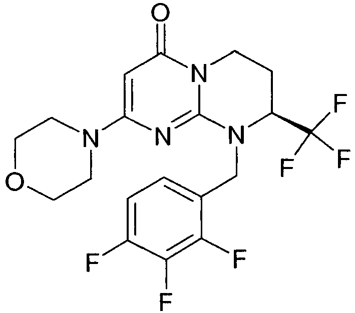

Le produit est préparé en suivant le mode opératoire décrit à l'exemple 22, à partir de 100 mg de la (8S)-2-morpholin-4-yl-8-trifluorométhyl-6,7,8,9-tétrahydro-pyrimido[1,2-a]pyrimidin-4-one (exemple 1e), de 214 mg de carbonate de césium et de 82 mg de 1-(bromométhyl)-2,3,4-trifluorobenzène dans 2 mL de diméthylformamide. Après purification par HPLC/MS préparative (Méthode C), on obtient 76 mg de la (8S)-2-(morpholin-4-yl)-9-(2,3,4-trifluorobenzyl)-8-(trifluorométhyl)-6,7,8,9-tétrahydro-4H-pyrimido[1,2-a]pyrimidin-4-one, sous forme d'une huile, dont les caractéristiques sont les suivantes :
Spectre RMN 1H : 2,15 à 2,31 (m, 1 H) ; 2,35 à 2,44 (m, 1 H) ; 3,18 à 3,32 (m, 5 H) ; 3,42 à 3,54 (m, 4 H) ; 4,22 (dd, J=5,5 et 14,3 Hz, 1 H) ; 4,65 (d, J=16,1 Hz, 1 H) ; 4,73 (m, 1 H) ; 4,98 (s, 1 H) ; 5,16 (d, J=16,1 Hz, 1 H) ; 7,05 à 7,17 (m, 1 H) ; 7,20 à 7,32 (m, 1 H)
Spectrométrie de Masse : méthode B
Temps de rétention Tr (min) = 4,08
[M+H]+ : m/z 449

### Exemple 32 : (8S)-9-[(5-chloro-1-benzothiophén-3-yl)méthyl]-2-(morpholin-4-yl)-8-(trifluorométhyl)-6,7,8,9-tétrahydro-4H-pyrimido[1,2-a]pyrimidin-4-one

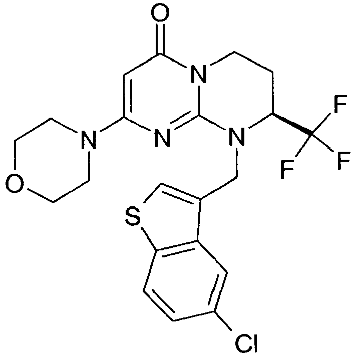

Le produit est préparé en suivant le mode opératoire décrit à l'exemple 22, à partir de 100 mg de la (8S)-2-morpholin-4-yl-8-trifluorométhyl-6,7,8,9-tétrahydro-pyrimido[1,2-a]pyrimidin-4-one (exemple 1e), de 214 mg de carbonate de césium et de 95 mg de 3-(bromométhyl)-5-chloro-1-benzothiophène dans 2 mL de diméthylformamide. Après purification par HPLC/MS préparative (Méthode C), on obtient 72 mg de la (8S)-9-[(5-chloro-1-benzothiophén-3-yl)méthyl]-2-(morpholin-4-yl)-8-(trifluorométhyl)-6,7,8,9-tétrahydro-4H-pyrimido[1,2-a]pyrimidin-4-one, sous forme d'une huile, dont les caractéristiques sont les suivantes :
Spectre RMN 1 H : 2,13 à 2,25 (m, 1 H) ; 2,34 à 2,43 (m, 1 H) ; 3,16 à 3,32 (m, 5 H) ; 3,35 à 3,48 (m, 4 H) ; 4,21 (dd, J=6,0 et 14,3 Hz, 1 H) ; 4,62 à 4,74 (m, 2 H) ; 5,00 (s, 1 H) ; 5,52 (d, J=16,1 Hz, 1 H) ; 7,42 (dd, J=2,0 et 8,6 Hz, 1 H); 7,67 (s, 1 H) ; 8,01 (d, J=2,0 Hz, 1 H); 8,03 (d, J=8,6 Hz, 1 H)
Spectrométrie de Masse : méthode B
Temps de rétention Tr (min) = 4,42
[M+H]+ : m/z 485

### Exemple 33 : (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one

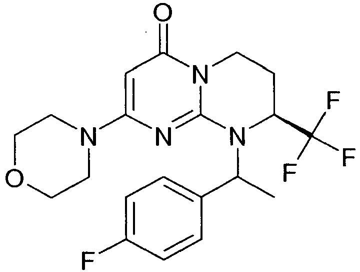

### Stade a :

La séparation des deux diastéréoisomères de la (8S)-9-[(1R et 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one est réalisée par chromatographie chirale : Colonne Chiralpak IC 20 µm; Elution 70 % Heptane 30% EtOH, à partir de 130 mg d'un mélange 70/30 des deux diastéréoisomères.

Le premier diastéréoisomère est concentré pour obtenir 42 mg de la (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one, sous forme d'un solide incolore, dont les caractéristiques sont les suivantes :
Spectre RMN 1 H (400MHz): 1,72 (d, J=6,8 Hz, 3 H) ; 2,22 (m, 1 H) ; 2,43 (m, 1 H) ; 3,14 à 3,27 (m, 5 H) ; 3,39 à 3,54 (m, 4 H) ; 4,13 (dd, J=5,6 et 14,4 Hz, 1 H) ; 4,80 à 4,88 (m, 1 H) ; 4,93 (s, 1 H) ; 5,43 (q, J=6,8 Hz, 1 H) ; 7,12 (t, J=8,8 Hz, 2 H) ; 7,36 (dd, J=5,6 et 8,8 Hz, 2 H)
Spectrométrie de Masse : méthode A
Temps de rétention Tr (min) = 0,95
[M+H]+ : m/z 427

### Stade b : (8S)-9-[(1R et 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one

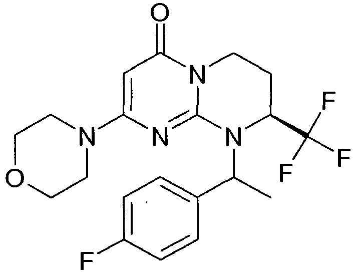

Le mélange de (8S)-9-[(1R et 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one est préparée en suivant le mode opératoire décrit à l'exemple 24 à partir de 500 mg de la (8S)-2-(morpholin-4-yl)-8-(trifluorométhyl)-6,7,8,9-tétrahydro-4H-pyrimido[1,2-a]pyrimidin-4-one, de 1 g de carbonate de césium et de 391 mg de 1-(1-chloroéthyl)-4-fluorobenzène dans 20 mL d'acétonitrile. Après purification par chromatographie sur silice (éluant : CH2Cl2/MeOH 97/3), on obtient 130 mg de la (8S)-9-[(1R et 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one, sous forme d'un mélange 70/30 de deux diastéréoisomères, dont les caractéristiques sont les suivantes :
Spectre RMN 1 H : Il s'agit d'un mélange 70-30 de deux isomères avec : 1,65 (d, J=7,0 Hz, 2,1 H) ; 1,72 (d, J=7,0 Hz, 0,9 H) ; 1,75 à 1,86 (m, 0,7 H) ; 2,25 à 2,48 (m, 1,3 H) ; 3,12 à 3,27 (m, 5 H) ; 3,40 à 3,56 (m, 4 H) ; 4,00 à 4,22 (m, 1 H) ; 4,42 (m, 0,7 H) ; 4,80 à 4,87 (m, 0,3 H) ; 4,93 (s, 0,3 H) ; 4,96 (s, 0,7 H) ; 5,44 (q, J=7,0 Hz, 0,3 H) ; 5,65 (q, J=7,0 Hz, 0,7 H) ; 7,06 à 7,21 (m, 2 H) ; 7,32 à 7,40 (m, 2 H)

### Exemple 34 : (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one

La purification précédente (exemple 33, stade a) conduit aussi à 85 mg de la (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one, sous forme d'un solide incolore, dont les caractéristiques sont les suivantes :
Spectre RMN 1 H : 1,65 (d, J=7,0 Hz, 3 H) ; 1,69 à 1,86 (m, 1 H) ; 2,29 à 2,37 (m, 1 H) ; 3,14 à 3,28 (m, 5 H) ; 3,44 à 3,58 (m, 4 H) ; 4,08 (dd, J=5,9 et 14,7 Hz, 1 H) ; 4,42 (m, 1 H) ; 4,96 (s, 1 H) ; 5,64 (q, J=7,0 Hz, 1 H) ; 7,17 (t, J=8,8 Hz, 2 H) ; 7,35 (dd, J=5,6 et 8,8 Hz, 2 H)
Spectrométrie de Masse : méthode A
Temps de rétention Tr (min) = 0,95
[M+H]+ : m/z 427

### Exemple 35 : (8S)-9-(3-méthylphényl)-2-(morpholin-4-yl)-8-(trifluorométhyl)-6,7,8,9-tétrahydro-4H-pyrimido[1,2-a]pyrimidin-4-one

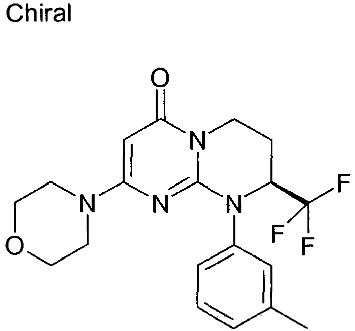

Le produit est préparé en suivant le mode opératoire décrit dans l'exemple 12, mais à partir de 250 mg de la (8S)-2-morpholin-4-yl-8-trifluorométhyl-6,7,8,9-tétrahydro-pyrimido[1,2-a]pyrimidin-4-one (exemple 1e) dans 4 mL de diméthylformamide, de 251 mg de 1-iodo-3-méthylbenzène, de 349 mg de phosphate de tripotassium, de 156 mg d'iodure de cuivre et de 93 mg de (1S,2S)-cyclohexane-1,2-diamine. Après 1 heure à 150°C sous irradiation micro-ondes et purification sur colonne de silice du mélange réactionnel résultant (gradient d'élution de CH2Cl2 à CH2Cl2/MeOH 98/02), on obtient 195 mg de la (8S)-9-(3-méthylphényl)-2-(morpholin-4-yl)-8-(trifluorométhyl)-6,7,8,9-tétrahydro-4H-pyrimido[1,2-a]pyrimidin-4-one, sous forme d'un solide vert, dont les caractéristiques sont les suivantes :
Spectre RMN 1H : 2,31 (s, 3 H) ; 2,37 à 2,47 (m, 2 H) ; 3,02 à 3,16 (m, 4 H) ; 3,19 à 3,39 (m partiellement masqué, 1 H) ; 3,40 à 3,53 (m, 4 H) ; 4,28 à 4,40 (m, 1 H) ; 4,93 (m, 1 H) ; 4,99 (s, 1 H) ; 7,13 (m, 2 H) ; 7,19 (s large, 1 H) ; 7,29 (t, J=7,5 Hz, 1 H)
Spectrométrie de Masse : méthode A
Temps de rétention Tr (min) = 0,90
[M+H]+ : m/z 395

### Exemple 36 : (8S)-9-(4-chlorophényl)-2-(morpholin-4-yl)-8-(trifluorométhyl)-6,7,8,9-tétrahydro-4H-pyrimido[1,2-a]pyrimidin-4-one

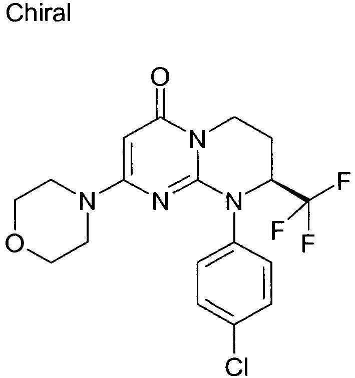

Le produit est préparé en suivant le mode opératoire décrit dans l'exemple 12, mais à partir de 250 mg de la (8S)-2-morpholin-4-yl-8-trifluorométhyl-6,7,8,9-tétrahydro-pyrimido[1,2-a]pyrimidin-4-one (exemple 1e), de 274 mg de 1-chloro-4-iodobenzène, de 349 mg de phosphate de tripotassium, de 156 mg d'iodure de cuivre et de 93 mg de (1S,2S)-cyclohexane-1,2-diamine. Après 1 heure à 150°C sous irradiation micro-ondes et purification du mélange réactionnel résultant sur colonne de silice (gradient d'élution de CH2Cl2 à CH2Cl2/MeOH 98/02), on obtient 145 mg de la (8S)-9-(4-chlorophényl)-2-(morpholin-4-yl)-8-(trifluorométhyl)-6,7,8,9-tétrahydro-4H-pyrimido[1,2-a]pyrimidin-4-one, sous forme d'une meringue verte, dont les caractéristiques sont les suivantes :
Spectre RMN 1H : 2,41 à 2,47 (m, 2 H) ; 3,07 à 3,12 (m, 4 H) ; 3,20 à 3,43 (m partiellement masqué, 1 H) ; 3,46 (m, 4 H) ; 4,35 (m, 1 H) ; 4,94 (m, 1 H) ; 5,01 (s, 1 H) ; 7,39 (d, J=8,8 Hz, 2 H) ; 7,49 (d, J=8,8 Hz, 2 H)
Spectrométrie de Masse : méthode A
Temps de rétention Tr (min) = 0,94 ;
[M+H]+ : m/z 415

### Exemple 37 : (8S)-2-(morpholin-4-yl)-8-(trifluorométhyl)-9-[4-(trifluorométhyl)phényl]-6,7,8,9-tétrahydro-4H-pyrimido[1,2-a]pyrimidin-4-one

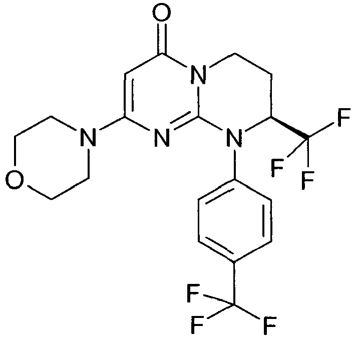

Le produit est préparé en suivant le mode opératoire décrit dans l'exemple 12 mais à partir de 250 mg de la (8S)-2-morpholin-4-yl-8-trifluorométhyl-6,7,8,9-tétrahydro-pyrimido[1,2-a]pyrimidin-4-one (exemple 1e), de 313 mg de 1-iodo-4-(trifluorométhyl)benzène, de 349 mg de phosphate de tripotassium, de 156 mg d'iodure de cuivre et de 93 mg de (1S,2S)-cyclohexane-1,2-diamine. Après 1 heure à 150°C sous irradiation micro-ondes et purification du mélange réactionnel résultant sur colonne de silice (gradient d'élution de CH2Cl2 à CH2Cl2/MeOH 98/02), on obtient 120 mg de la (8S)-2-(morpholin-4-yl)-8-(trifluorométhyl)-9-[4-(trifluorométhyl)phényl]-6,7,8,9-tétrahydro-4H-pyrimido[1,2-a]pyrimidin-4-one sous forme d'un solide verdâtre dont les caractéristiques sont les suivantes :
Spectre RMN 1 H (400MHz): 2,42 à 2,53 (m partiellement masqué, 2 H) ; 3,01 à 3,13 (m, 4 H) ; 3,22 à 3,39 (m partiellement masqué, 1 H) ; 3,41 à 3,46 (m, 4 H) ; 4,37 (m, 1 H) ; 5,03 (s, 1 H) ; 5,05 (m, 1 H) ; 7,62 (d, J=8,6 Hz, 2 H) ; 7,81 (d, J=8,6 Hz, 2 H)
Spectrométrie de masse : Méthode A
Temps de rétention Tr (min) = 0,98
[M+H]+ : m/z 449

### Exemple 38 : (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one

### Stade c :

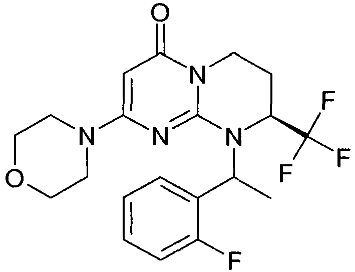

La séparation des deux diastéréoisomères de la (8S)-9-[(1R et 1 S)-1-(2-fluorophényl)éthyl]-2-(morpholin-4-yl)-8-(trifluorométhyl)-6,7,8,9-tétrahydro-4H-pyrimido[1,2-a]pyrimidin-4-one est réalisée par chromatographie chirale (colonne Chiralpak AD 20 µm; élution 80 % Heptane 10% EtOH 10 % MeOH) à partir de 70 mg d'un mélange 70/30 des deux diastéréoisomères.

On obtient ainsi 41.5 mg de la (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one dont les caractéristiques sont les suivantes
Spectre RMN 1 H (400MHz) :
   1,62 (d, J=7,2 Hz, 3 H) ; 1,68 à 1,85 (m, 1 H) ; 2,30 à 2,39 (m, 1 H) ; 3,20 à 3,42 (m, 5 H) ; 3,50 à 3,65 (m, 4 H) ; 4,03 (m, 1 H) ; 4,22 à 4,36 (m, 1 H) ; 4,99 (s, 1 H) ; 6,05 (q, J=7,2 Hz, 1 H) ; 7,14 à 7,29 (m, 2 H) ; 7,35 à 7,43 (m, 1 H) ; 7,47 (m, 1 H)
   Spectrométrie de masse : méthode B
   Temps de rétention Tr (min) = 4.02
   [M+H]+ : m/z 427
   Pouvoir rotatoire : PR = +33 ; C = 2.543mg/1ML DMSO.

### Stade b : (8S)-9-[(1R et 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one

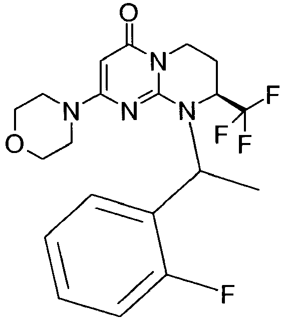

Le produit peut être préparé en suivant le mode opératoire décrit à l'exemple 17, mais à partir de 500 mg de la (8S)-2-(morpholin-4-yl)-8-(trifluorométhyl)-6,7,8,9-tétrahydro-4H-pyrimido[1,2-a]pyrimidin-4-one, de 1 g de carbonate de césium, de 391 mg de 1-(1-chloroéthyl)-2-fluorobenzène (voir stade a ci-dessous) dans 22 mL d'acétonitrile. Après purification sur colonne de silice (éluant : CH2Cl2 / MeOH 97/03), on obtient 70 mg d'un mélange 70/30 des deux diastéréoisomères de la (8S)-9-[(1R et 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one, sous forme d'une poudre jaune pâle, dont les caractéristiques sont les suivantes :
Temps de rétention Tr (min) = 0,93 et 0,90 : mélange 70% - 30% d'isomères ;
[M+H]+ : m/z 427

### Stade a : 1-(1-chloroéthyl)-2-fluorobenzène

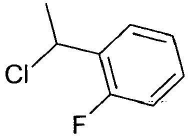

A une solution de 1 g de 1-(2-fluorophényl)éthanol commercial dans 20 mL de chloroforme sont ajoutés 767 mg de chlorure de thionyle. Après une nuit d'agitation à une température voisine de 20°C, le mélange réactionnel est lavé par une solution aqueuse saturée en bicarbonate de sodium puis séché sur sulfate de magnésium anhydre, filtré et concentré à sec sous pression réduite. On obtient ainsi 780 mg de 1-(1-chloroéthyl)-2-fluorobenzène qui est utilisé tel que dans l'étape suivante.

### Exemple 39 : (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one

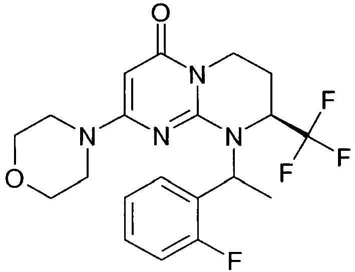

La purification précédente (exmple 38, stade c) conduit aussi à 17.9 mg de la (8S)-9-[(1R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one, sous forme d'un solide ambre, dont les caractéristiques sont les suivantes :
Spectre RMN 1 H (400MHz): 1,69 (d, J=7,0 Hz, 3 H) ; 1,97 à 2,12 (m, 1 H) ; 2,36 à 2,46 (m, 1 H) ; 3,15 à 3,35 (m partiellement masqué, 5 H) ; 3,43 à 3,59 (m, 4 H) ; 4,09 (m, 1 H) ; 4,72 (m, 1 H) ; 4,93 (s, 1 H) ; 5,73 (q, J=7,0 Hz, 1 H) ; 7,07 à 7,25 (m, 2 H) ; 7,28 à 7,40 (m, 1 H) ; 7,51 (m, 1 H)
Spectrométrie de masse : méthode B
Temps de rétention Tr (min) = 3,93
[M+H]+ : m/z 427
Pouvoir Rotatoire : PR = -96.3+/-1.4 ; C = 2.812mg/0.5ML DMSO.

### Exemple 40 : (8S)-9-[2-(3-fluorophényl)éthyl]-2-(morpholin-4-yl)-8-(trifluorométhyl)-6,7,8,9-tétrahydro-4H-pyrimido[1,2-a]pyrimidin-4-one

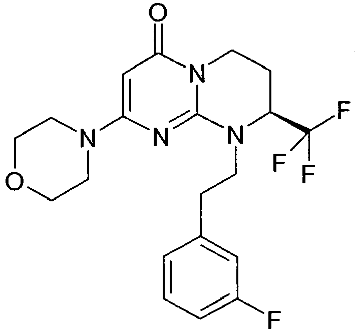

A une solution de 150 mg de la (8S)-2-(morpholin-4-yl)-8-(trifluorométhyl)-6,7,8,9-tétrahydro-4H-pyrimido[1,2-a]pyrimidin-4-one dans 2.5 mL de toluène sont ajoutés 296 mg d'hydroxyde de sodium dans 2.5 mL d'eau, 33 mg d'hydrogènosulfate de tétrabutylammonium et 200 mg de 1-(2-bromoéthyl)-3-fluorobenzène. Après une heure sous irradiation micro-ondes (puissance 100 Watts sur appareil CEM discover) à 60°C puis une heure à nouveau à 60°C et deux fois six heures à 70°C. le mélange réactionnel est dilué avec de l'acétate d'éthyle. Le mélange résultant est lavé par de l'eau. La phase organique est séparée puis concentrée à sec sous pression réduite. Après purification par HPLC/MS préparative du résidu obtenu (Méthode D), on obtient 43 mg de la (8S)-9-[2-(3-fluorophényl)éthyl]-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre RMN 1 H (400MHz): 1,87 à 2,05 (m, 1 H) ; 2,29 à 2,38 (m, 1 H) ; 2,82 à 3,24 (m, 3 H) ; 3,38 à 3,50 (m, 5 H) ; 3,60 à 3,66 (m, 4 H) ; 4,11 à 4,25 (m, 2 H) ; 4,50 à 4,69 (m, 1 H) ; 4,99 (s, 1 H) ; 6,96 à 7,15 (m, 3 H) ; 7,28 à 7,41 (m, 1 H)
Spectrométrie de masse : méthode B
Temps de rétention Tr (min) = 4,10
[M+H]+ : m/z 427

### Exemple 41 : (8S)-9-benzyl-3-fluoro-2-(morpholin-4-yl)-8-(trifluorométhyl)-6,7,8,9-tétrahydro-4H-pyrimido[1,2-a]pyrimidin-4-one

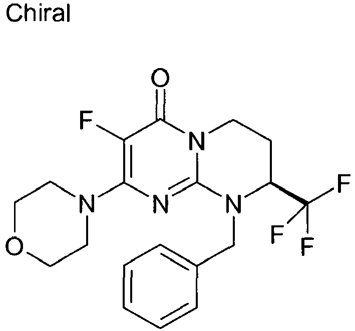

### Stade e :

A une suspension de 1 g de la (8S)-3-fluoro-2-(morpholin-4-yl)-8-(trifluorométhyl)-6,7,8,9-tétrahydro-4H-pyrimido[1,2-a]pyrimidin-4-one dans 11.8 mL d'acétonitrile sont ajoutés 4 g de carbonate de césium et 796 mg de bromure de benzyle. Après une nuit d'agitation à une température voisine de 20°C, la suspension obtenue est filtrée et le filtrat résultant concentré à sec sous pression réduite. Le résidu huileux jaune est purifié sur colonne de silice (éluant : CH2Cl2 / MeOH 98/02). Les fractions d'intérêt sont réunies et concentrées à sec sous pression réduite. Le résidu est repris par de l'oxyde de diéthyle, essoré puis séché sous vide. On obtient ainsi 600 mg de la (8S)-9-benzyl-3-fluoro-2-(morpholin-4-yl)-8-(trifluorométhyl)-6,7,8,9-tétrahydro-4H-pyrimido[1,2-a]pyrimidin-4-one, sous forme d'une poudre blanche, dont les caractéristiques sont les suivantes :
Spectre RMN 1 H (400MHz): 2,17 à 2,30 (m, 1 H) ; 2,39 à 2,46 (m, 1 H) ; 3,31 à 3,52 (m, 9 H) ; 4,23 (m, 1 H) ; 4,55 (d, J=16,1 Hz, 1 H) ; 4,61 à 4,73 (m, 1 H) ; 5,13 (d, J=16,1 Hz, 1 H) ; 7,20 à 7,26 (m, 3 H) ; 7,28 à 7,36 (m, 2 H)
Spectrométrie de masse : méthode B
Temps de rétention Tr (min) = 4,01
[M+H]+ : m/z 413

### Stade d : (8S)-3-fluoro-2-(morpholin-4-yl)-8-(trifluorométhyl)-6,7,8,9-tétrahydro-4H-pyrimido[1,2-a]pyrimidin-4-one

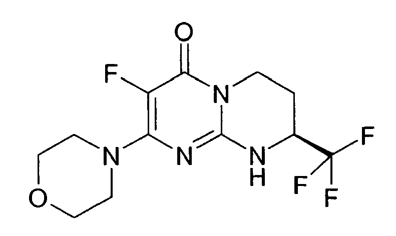

Le produit peut être préparé en suivant le mode opératoire décrit à l'exemple 16, stade b, mais à partir de 1 g de la (8S)-2-chloro-3-fluoro-8-(trifluorométhyl)-6,7,8,9-tétrahydro-4H-pyrimido[1,2-a]pyrimidin-4-one dans 5 mL d'acétonitrile et de 1.6 mL de morpholine. Après une nuit à 65°C, on obtient 1.1 g de la (8S)-3-fluoro-2-(morpholin-4-yl)-8-(trifluorométhyl)-6,7,8,9-tétrahydro-4H-pyrimido[1,2-a]pyrimidin-4-one, sous forme d'une poudre beige, dont les caractéristiques sont les suivantes :
Spectrométrie de masse : Méthode A
Temps de rétention Tr (min) = 0,56
[M+H]+ : m/z 323 ; [M-H]- : m/z 321

### Stade c : (8S)-3-fluoro-2-chloro-8-(trifluorométhyl)-6,7,8,9-tétrahydro-4H-pyrimido[1,2-a]pyrimidin-4-one

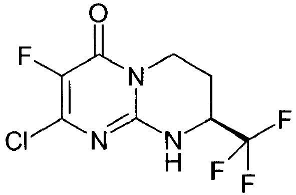

La séparation des énantiomères de la (8R,8S)-2-chloro-3-fluoro-8-(trifluorométhyl)-6,7,8,9-tétrahydro-4H-pyrimido[1,2-a]pyrimidin-4-one est réalisée par chromatographie chirale (Chiralpak AD 20µm 80X350 mm 250ml/min 254nm ;
5% EtOH 5% MeOH 90% Heptane +0.1% TEA), à partir de 6.8 g d'un mélange racémique.

L'énantiomère dextrogyre est concentré pour obtenir 3.13 g de la (8S)-2-chloro-3-fluoro-8-(trifluorométhyl)-6,7,8,9-tétrahydro-4H-pyrimido[1,2-a]pyrimidin-4-one, sous forme d'un solide blanc, dont les caractéristiques sont les suivantes :
Spectrométrie de masse : Méthode A
Temps de rétention Tr (min) = 0,62
[M+H]+ : m/z 272 ; [M-H]- : m/z 270
Pouvoir rotatoire : PR= +19.6+/-0.6; C = 2.488mg/0.5ML CH3OH.

### Stade b : (8R, 8S)-2-chloro-3-fluoro-8-(trifluorométhyl)-6,7,8,9-tétrahydro-4H-pyrimido[1,2-a]pyrimidin-4-one

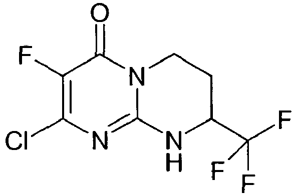

A une solution de 6.5 g de (8R, 8S)-3-fluoro-2-hydroxy-8-(trifluorométhyl)-6,7,8,9-tétrahydro-4H-pyrimido[1,2-a]pyrimidin-4-one dans 20 mL de 1,2-dichloroéthane sont ajoutés 8 mL de trichlorure de phosphore. Après 4 heures d'agitation à une température de 65 °C et retour à une température voisine de 20°C, le mélange réactionnel, est concentré à sec sous pression réduite. Le résidu est dilué dans 150 mL d'acétate d'éthyle et 10mL d'eau glacée. A une température comprise entre 0°C et 10°C, est ajoutée une solution d'hydroxyde de sodium concentrée jusqu'à obtention d'un pH compris entre 6 et 7. Le solide formé est filtré pour donner 3.5 g d'un solide beige S1. Le filtrat est décanté, et la phase organique est séchée sur sulfate de magnésium anhydre, filtrée et concentrée à sec sous pression réduite. Après purification du résidu sur colonne de silice (éluant : CH2Cl2 / MeOH 97/03), on obtient 3.3 g d'un solide jaune pâle S2. Les deux solides S1 et S2 sont réunis pour donner 6.8 g de la (8R, 8S)-2-chloro-3-fluoro-8-(trifluorométhyl)-6,7,8,9-tétrahydro-4H-pyrimido[1,2-a]pyrimidin-4-one, sous forme d'une poudre jaune pâle, dont les caractéristiques sont les suivantes :
Spectrométrie de masse : Méthode B
Temps de rétention Tr (min) = 2,90
[M+H]+ : m/z 272 ; [M-H]- : m/z 270

### Stade a : (8R, 8S)-3-fluoro-2-hydroxy-8-(trifluorométhyl)-6,7,8,9-tétrahydro-4H-pyrimido[1,2-a]pyrimidin-4-one

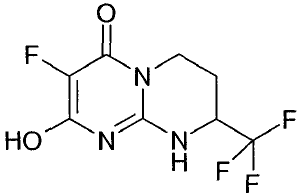

A une suspension de 7 g de 6-(trifluorométhyl)-1,4,5,6-tétrahydropyrimidin-2-amine chlorhydrate (exemple 1, stade a) dans 35 mL de fluoro propanedioate de diméthyle sont ajoutés 5.6 g de méthylate de sodium. Après 3 heures d'agitation de la suspension à une température de 100°C, le milieu obtenu est concentré à sec sous pression réduite. Le résidu est repris dans de l'oxyde de diéthyle puis essoré sous vide. Le solide obtenu est repris dans 14 mL d'eau et le mélange résultant est, refroidi dans de la glace avant acidification jusqu'à pH 5-6 par ajout d'acide chlorhydrique concentré (25%). Après 2 heures d'agitation à une température de 0°C puis une nuit à une température voisine de 20°C, la suspension est filtrée puis le solide est essoré et séché sous vide sur P₂O₅. On obtient 6.5 g de (8R, 8S)-3-fluoro-2-hydroxy-8-(trifluorométhyl)-6,7,8,9-tétrahydro-4H-pyrimido[1,2-a]pyrimidin-4-one, sous forme d'une poudre jaune, dont les caractéristiques sont les suivantes :
Spectrométrie de masse : Méthode A
Temps de rétention Tr (min) = 0,28
[M+H]+ : m/z 254 ; [M-H]- : m/z 252

### Exemple 42 : (8S)-9-(3,5-difluorophényl)-2-(morpholin-4-yl)-8-(trifluorométhyl)-6,7,8,9-tétrahydro-4H-pyrimido[1,2-a]pyrimidin-4-one

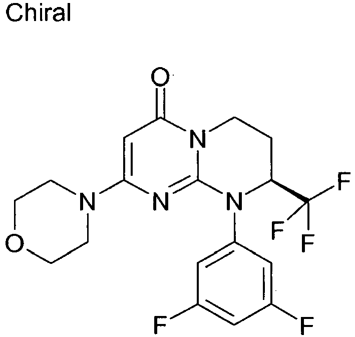

Le produit est préparé en suivant le mode opératoire décrit dans l'exemple 12, mais à partir de 250 mg de la (8S)-2-morpholin-4-yl-8-trifluorométhyl-6,7,8,9-tétrahydro-pyrimido[1,2-a]pyrimidin-4-one (exemple 1e), de 276 mg de 1,3-difluoro-5-iodobenzène, de 349 mg de phosphate de tripotassium, de 156 mg d'iodure de cuivre et de 93 mg de (1S,2S)-cyclohexane-1,2-diamine. Après 1 heure à 150°C sous irradiation micro-ondes et purification sur colonne de silice du mélange réactionnel (gradient d'élution de CH2Cl2 à CH2Cl2/MeOH 98/02), on obtient 91 mg de (8S)-9-(3,5-difluorophényl)-2-(morpholin-4-yl)-8-(trifluorométhyl)-6,7,8,9-tétrahydro-4H-pyrimido[1,2-a]pyrimidin-4-one, sous forme d'une meringue ocre, dont les caractéristiques sont les suivantes :
Spectre RMN 1 H (400MHz): 2,45 (m, 2 H) ; 3,07 à 3,16 (m, 4 H) ; 3,32 à 3,37 (m, 1 H) ; 3,45 à 3,55 (m, 4 H) ; 4,36 (m, 1 H) ; 5,01 (m, 1 H) ; 5,04 (s, 1 H) ; 7,18 à 7,34 (m, 3 H)
Spectrométrie de masse : méthode A
Temps de rétention Tr (min) = 0,95
[M+H]+ : m/z 417 ; [M-H+HCO₂H]- : m/z 461

### Exemple 43: (8S)-9-[(2,6-difluorophényl)carbonyl]-2-(morpholin-4-yl)-8-(trifluorométhyl)-6,7,8,9-tétrahydro-4H-pyrimido[1,2-a]pyrimidin-4-one

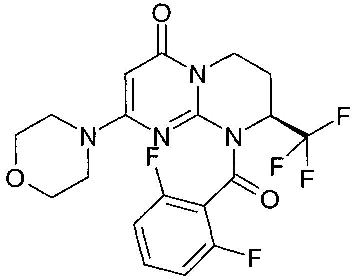

Le produit est préparé en suivant le mode opératoire décrit à l'exemple 18, à partir de. 300 mg de (8S)-2-morpholin-4-yl-8-trifluorométhyl-6,7,8,9-tétrahydro-pyrimido[1,2-a]pyrimidin-4-one (exemple 1e), de 47 mg d'hydrure de sodium et de 174 mg de chlorure de 2,6-difluorobenzoyle dans 4 mL de tétrahydrofuranne. Après trois purifications successives par chromatographie sur silice (éluant: CH₂Cl₂/MeOH; gradient de 100/0 à 98/02 puis 98/1 et 98/2), on obtient 22 mg de la (8S)-9-[(2,6-difluorophényl)carbonyl]-2-(morpholin-4-yl)-8-(trifluorométhyl)-6,7,8,9-tétrahydro-4H-pyrimido[1,2-a]pyrimidin-4-one, sous forme d'un solide blanc, dont les caractéristiques sont les suivantes :
Spectre RMN 1 H (400MHz) : 2,06 à 2,23 (m, 1 H) ; 2,69 à 2,79 (m, 1 H) ; 2,91 (m, 2 H) ; 3,06 (m, 2 H) ; 3,18 à 3,34 (m partiellement masqué, 1 H) ; 3,37 à 3,50 (m, 4 H) ; 4,52 à 4,61 (m, 1 H) ; 5,23 (s, 1 H) ; 5,62 à 5,86 (m, 1 H) ; 7,04 à 7,35 (m, 2 H) ; 7,50 à 7,67 (m, 1 H)
Spectrométrie de masse : méthode A
Temps de rétention Tr (min) = 0,93
[M+H]+ : m/z 445

### Exemple 44: (8S)-9-[(2,4-difluorophényl)carbonyl]-2-(morpholin-4-yl)-8-(trifluorométhyl)-6,7,8,9-tétrahydro-4H-pyrimido[1,2-a]pyrimidin-4-one

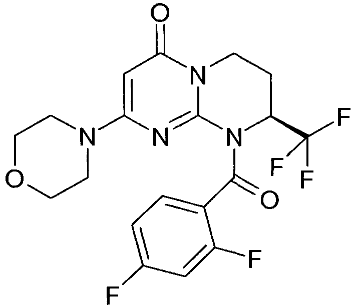

Le produit est préparé en suivant le mode opératoire décrit à l'exemple 18, à partir de 300 mg de la (8S)-2-morpholin-4-yl-8-trifluorométhyl-6,7,8,9-tétrahydro-pyrimido[1,2-a]pyrimidin-4-one (exemple 1e), de 47 mg d'hydrure de sodium et de 174 mg de chlorure de 2,4-difluorobenzoyle dans 4 mL de tétrahydrofuranne. Après deux purifications successives par chromatographie sur silice (éluant: CH₂Cl₂/MeOH gradient de 100/0 à 98/02 puis 99/01), on obtient 24 mg de la (8S)-9-[(2,4-difluorophényl)carbonyl]-2-(morpholin-4-yl)-8-(trifluorométhyl)-6,7,8,9-tétrahydro-4H-pyrimido[1,2-a]pyrimidin-4-one, sous forme d'une laque incolore, dont les caractéristiques sont les suivantes :
Spectre RMN 1 H (400MHz): 2,20 (m, 1 H) ; 2,67 à 2,77 (m, 1 H) ; 2,81 à 2,91 (m, 2 H) ; 3,03 (m, 2 H) ; 3,24 à 3,45 (m partiellement masqué, 2 H) ; 3,37 à 3,46 (m, 2 H) ; 3,52 (m, 1 H) ; 4,30 à 4,48 (m, 1 H) ; 5,21 (s, 1 H) ; 5,53 à 5,67 (m, 1 H) ; 7,18 (dt, J=2,5 et 8,6 Hz, 1 H) ; 7,35 (ddd, J=2,5 et 9,3 et 11,2 Hz, 1 H) ; 7,61 à 7,71 (m, 1 H)
Spectrométrie de masse : méthode A
Temps de rétention Tr (min) = 0,94
[M+H]+ : m/z 445

### Exemple 45 : (8S)-2-(morpholin-4-yl)-9-(phénylacétyl)-8-(trifluorométhyl)-6,7,8,9-tétrahydro-4H-pyrimido[1,2-a]pyrimidin-4-one

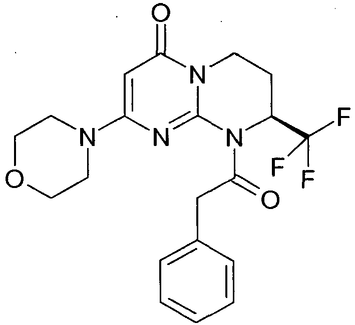

Le produit est préparé en suivant le mode opératoire décrit à l'exemple 18, à partir de 300 mg de la (8S)-2-morpholin-4-yl-8-trifluorométhyl-6,7,8,9-tétrahydro-pyrimido[1,2-a]pyrimidin-4-one (exemple 1e), de 47 mg d'hydrure de sodium et de 152 mg de chlorure de phénylacétyle dans 4 mL de tétrahydrofuranne. Après deux purifications successives par chromatographie sur silice (éluant: CH₂Cl₂/MeOH gradient de 100/0 à 98/02 puis CH₂Cl₂/AcOEt 95/05), on obtient 12 mg de la (8S)-2-(morpholin-4-yl)-9-(phénylacétyl)-8-(trifluorométhyl)-6,7,8,9-tétrahydro-4H-pyrimido[1,2-a]pyrimidin-4-one, sous forme d'une laque incolore, dont les caractéristiques sont les suivantes :
Spectre RMN 1 H (400MHz): 1,91 (m, 1 H) ; 2,59 à 2,69 (m, 1 H) ; 2,88 (m, 1 H) ; 3,33 à 3,45 (m, 4 H) ; 3,60 (m, 4 H) ; 4,07 (d, J=16,0 Hz, 1 H) ; 4,16 (d, J=16,0 Hz, 1 H) ; 4,43 à 4,52 (m, 1 H) ; 5,30 (s, 1 H) ; 5,48 à 5,61 (m, 1 H) ; 7,11 (d, J=7,7 Hz, 2 H) ; 7,17 à 7,23 (t, J=7,7 Hz, 1 H); 7,28 (t, J=7,7 Hz, 2 H)
Spectrométrie de masse : méthode A
Temps de rétention Tr (min) = 0,99 ;
[M+H]+ : m/z 423 ;
[M-H]- : m/z 421 ; pic de base : m/z 303

### Exemple 46 : (8S)-9-[2-(3-chlorophényl)éthyl]-2-(morpholin-4-yl)-8-(trifluorométhyl)-6,7,8,9-tétrahydro-4H-pyrimido[1,2-a]pyrimidin-4-one

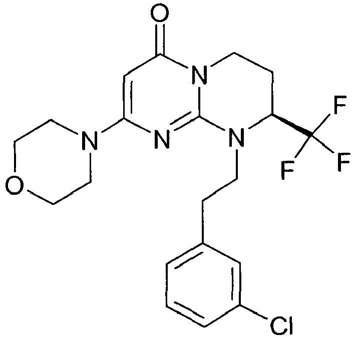

Le produit est préparé en suivant le mode opératoire décrit à l'exemple 40, à partir de 150 mg de la (8S)-2-(morpholin-4-yl)-8-(trifluorométhyl)-6,7,8,9-tétrahydro-4H-pyrimido[1,2-a]pyrimidin-4-one dans 2.5 mL de toluène sont ajoutés 296 mg d'hydroxyde de sodium dans 2.5 mL d'eau, 33 mg d'hydrogènosulfate de tétrabutylammonium et 216 mg de 1-(2-bromoéthyl)-3-chlorobenzène. Après 44 heures à 60°C. Après refroidissement, le mélange réactionnel est dilué avec de l'acétate d'éthyle. La phase organique est séparée et la phase aqueuse est extraite par de l'acétate d'éthyle. Les phases organiques rassemblées sont concentrées à sec sous pression réduite et le résidu est purifié par HPLC/MS préparative (Méthode D). On obtient ainsi 42 mg de la (8S)-9-[2-(3-chlorophényl)éthyl]-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre RMN 1 H (400MHz): 1,91 à 2,05 (m, 1 H) ; 2,34 (m, 1 H) ; 2,81 à 2,92 (m, 1 H) ; 2,94 à 3,04 (m, 1 H) ; 3,17 (m, 1 H) ; 3,38 à 3,50 (m, 5 H) ; 3,65 (m, 4 H) ; 4,09 à 4,22 (m, 2 H) ; 4,57 à 4,71 (m, 1 H) ; 4,99 (s, 1 H) ; 7,17 (d, J=7,8 Hz, 1 H) ; 7,25 à 7,38 (m, 3 H)
Spectrométrie de masse : méthode A
Temps de rétention Tr (min) = 1,03
[M+H]+ : m/z 443 ; [M-H+HCO₂H]- : m/z 487

### Exemple 47 composition pharmaceutique

On a préparé des comprimés répondant à la formule suivante :

| | |
|---|---|
| Produit de l'exemple 1 | 0,2 g |
| Excipient pour un comprimé terminé à | 1 g |
| (détail de l'excipient : lactose, talc, amidon, stéarate de magnésium). | |

L'exemple 1 est pris à titre d'exemple de préparation pharmaceutique, cette préparation pouvant être réalisée si désiré avec d'autres produits de formule (I) selon la présente invention et notamment en exemples dans la présente demande, parmi les exemples 2 à 46 et 48 à 56.

Les produits du tableau ci-dessous sont des produits de formule (I) tels que définis ci-dessus et constituent les exemples 48 à 56 de la présente invention. Ces produits de 48 à 56 sont préparés comme indiqué ci-dessus dans la partie expérimentale.

| Exemple | Nom | Spectrométrie de masse : Méthode E | |
|---|---|---|---|
| | | Tr (min) | [M+H]+:m/z |
| Exemple 48 | 9-((R)-2-Benzo[b]thiophen-2-yl-2-hydroxy-ethyl)-2-morpholin-4-yl-8-(S)-trifluoromethyl-6,7,8,9-tetrahydro-pyrimido[1,2-a]pyrimidin-4-one | 0.83 | m/z 481 |
| Exemple 49 | 9-[(S)-2-Hydroxy-2-(3-hydroxy-phenyl)-ethyl]-2-morpholin-4-yl-8-(S)-trifluoromethyl-6,7,8,9-tetrahydro-pyrimido[1,2-a]pyrimidin-4-one | 0.63 | m/z 441 |
| Exemple 50 | 2-Dimethylamino-N-{3-[(S)-1-hydroxy-2-((S)-8-morpholin-4-yl-6-oxo-2-trifluoromethyl-3,4-dihydro-2H,6H-pyrimido[1,2-a]pyrimidin-1-yl)-ethyl]-phenyl}-acetamide | 0.51 | m/z 525 |
| Exemple 51 | 9-[(S)-2-Hydroxy-2-(2-methoxy-phenyl)-ethyl]-2-morpholin-4-yl-8-(S)-trifluoromethyl-6,7,8,9-tetrahydro-pyrimido[1,2-a]pyrimidin-4-one | 0.74 | m/z 455 |
| Exemple 52 | 9-[(S)-2-(4-Fluoro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one | 0.76 | m/z 473 |
| Exemple 53 | 9-[(S)-2-(4-Chloro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one | 0.81 | m/z 489 |
| Exemple 54 | 9-[(S)-2-(2-Chloro-4-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one | 0.78 | m/z 489 |
| Exemple 55 | 9-(2-Hydroxy-3-phenyl-propyl)-2-morpholin-4-yl-8-(S)-trifluoromethyl-6,7,8,9-tetrahydro-pyrimido[1,2-a]pyrimidin-4-on | 0.74 | m/z 439 |
| Exemple 56 | 9-[2-(4-Hydroxy-phenyl)-ethyl]-2-morpholin-4-yl-8-(S)-trifluoromethyl-6,7,8,9-tetrahydro-pyrimido[1,2-a]pyrimidin-4-one | 0.68 | m/z 425 |

### Partie pharmacologique :

### Protocoles expérimentaux

### Procédures expérimentales in vitro

L'activité inhibitrice des molécules sur la phosphorylation d'AKT est mesurée soit par western blotting par la technique décrite ci-dessous, soit par la technique MSD Multi-spot Biomarker détection de Meso Scale Discovery également décrite ci-dessous. Il a été démontré sur un set de molécules que les 2 techniques donnent des résultats compatibles.

### Etude de l'expression de pAKT dans les cellules humaines PC3 de carcinome de prostate mesurée par western blotting (Test A):

Cet essai est basé sur la mesure de l'expression de la protéine AKT phosphorylée sur la serine 473. La phosphorylation d'AKT (pAKT) est mesurée par western blotting dans la lignée de carcinome de prostate humaine PC3 (ATCC CRL-1435), en utilisant un anticorps reconnaissant spécifiquement pAKT-S473.

Le jour 1, les cellules PC3 sont ensemencées en plaques 6 puits (TPP, # 92006) à la concentration de 0.8x106 cellules/puits dans 1800 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2 heures à 37°C en présence de 5% CO2. Les molécules diluées dans du diméthylsulfoxyde (DMSO Sigma #D2650), sont ajoutées à partir d'une solution mère concentrée 10 fois, le pourcentage final de DMSO étant de 0.1%. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Après aspiration du milieu de culture, les cellules sont rincées par 1ml de PBS (DPBS Gibco, #14190-094), récupérées par grattage dans 200µl de tampon HNTG complet et transfert en plaque 96 puits (Greiner #651201), et lysées pendant 1 H sur glace. Le tampon HNTG est composé du mélange suivant :Hepes 50 mM, NaCl 150 mM, Triton 1%, Glycerol 10%, avec ajoût extemporané d'une pastille de Protease Inhibitor Cocktail Mini (Roche 1836153) et d'une pastille de Phosphatase Inhibitor Cocktail (Roche104906837001) pour 10ml de tampon.

Le lysat est centrifugé 10min à 6000RPM. 155µl de surnageant sont récupérés. 150 µl sont incubés pour dénaturation pendant 5min à 95°C en présence de tampon NuPAGE LDS Sample Buffer 4X dilué 4 fois (Ref InVitrogen NP0007) et de NuPAGE Sample Reducing Agent 10X dilué 10 fois (Ref InVitrogen NP0009). Ces échantillons sont ensuite congelés à -20°C. 5 µl sont dosées par la technique microBCA selon la fiche technique du MicroBCA Proteine Assay Kit (Pierce #23235).

Pour la séparation des protéines, 20µg de protéines sont déposées sur gel NU-PAGE 4-12% Bis Tris Gel 12 puits (Ref InVitrogen NP0322BOX) et la migration est effectuée pendant 1 h30 en tampon de migration NU-PAGE MOPS SDS Running Buffer 20X dilué 20 fois (Ref InVitrogen NP0001), à 150 Volts.

Le gel est ensuite transféré sur une membrane Invitrolon PVDF (Invitrogen #LC2007) préalablement perméabilisée quelques secondes dans de l'éthanol (Ethanol Fischer Scientific #E/0600DF/15).

Le transfert s'effectue dans une cuve Biorad à 30 Volts pendant la nuit ou à 60 volts pendant 3 heures, en présence de tampon de transfert NUPAGE Transfer Buffer 20X dilué 20 fois (Ref InVitrogen NP0006).

La membrane est ensuite saturée en solution de saturation composé de TBS (Tris Buffer Saline 10x, Sigma #T5912 Sigma, dilué 10 fois), Tween 20 0.1% (#P5927 Sigma) et BSA 3% (Bovine Albumin Serum Fraction V, Sigma #A4503) pendant 6h après un transfert d'une durée de une nuit ou bien pendant 1 h après un transfert d'une durée de 3h.

Les anticorps primaires sont dilués au 1/1000e pour l'anticorps anti-phospho AKT-Ser473 (193H2, monoclonal de lapin, cat#4058 de chez Cell Signaling Technology) Abcam), en solution de saturation composée de PBS, Tween 20 0.1 %, BSA 3%, puis mis sous agitation pendant la nuit à 4°C.

Deux rinçages de 5 min en solution de lavage composée de TBS, Tween 20 0.1 % sont effectués avant l'hybridation des anticorps secondaires.

Les anticorps secondaires sont dilués au 1/10000e pour l'anticorps Rabbit anti-Mouse IgG HRP (W402 Promega) et au 1/10000e pour l'anticorps Goat anti-Rabbit IgG HRP (W401 Promega) en solution de saturation puis mis sous agitation pendant 1 h à température ambiante.

Deux rinçages de 30 min en solution de lavage sont effectués puis un rinçage de 5 min à l'H2O est effectué pour éliminer le Tween 20 restant.

La solution de révélation est préparée volume à volume selon la fiche technique du Western Lightning Chemiluminescence Reagent Plus (Western Lightning Chemiluminescence Reagent Plus Perkin Elmer #NEL104).

La membrane est placée pendant 1 min dans la solution de révélation, égouttée, insérée entre deux transparents puis placée dans l'appareil de mesure pour la lecture de la luminescence et la quantification du signal. La lecture de la luminescence est effectuée avec l'appareil FujiFilm (Ray Test).

L'appareil FUJI mesure le signal total de luminescence obtenu (AU) pour chaque bande sélectionnée. Puis il soustrait le bruit de fond (BG) proportionnel à la taille de la bande sélectionnée (Area), bruit de fond calculé à partir d'une bande de bruit de fond spécifique, en vue d'obtenir le signal spécifique (AU-BG) pour chaque bande. La bande obtenue en absence de produit et en présence de 0.1% DMSO est considérée comme le 100 % de signal. Le logiciel calcule le % d'activité spécifique (Ratio) obtenu pour chaque bande sélectionnée en fonction de ce 100 % de signal. Le calcul du pourcentage d'inhibition est fait pour chaque concentration selon la formule (100% - Ratio).

2 expériences indépendantes permettent de calculer la moyenne des pourcentages d'inhibition obtenus à une concentration donnée pour les produits testés uniquement à une concentration.

Le cas échéant, l'activité des produits est traduite en CI50 approchée, obtenue à partir d'une courbe dose-réponse de différentes concentrations testées et représentant la dose donnant 50 % d'inhibition spécifique (CI50 absolue). 2 expériences indépendantes permettent de calculer la moyenne des CI50s.

### Etude de l'expression de pAKT dans les cellules humaines PC3 de carcinome de prostate mesurée par la technique MSD Multi-spot Biomarker Detection de Meso Scale Discovery (Test B):

Cet essai est basé sur la mesure de l'expression de la protéine AKT phosphorylée sur la sérine 473 (P-AKT-S473), dans la lignée de carcinome de prostate humaine PC3, par la technique basée sur un immuno-essai sandwich utilisant le kit MSD Multi-spot Biomarker Detection de Meso Scale Discovery: kits phospho-Akt (Ser473) whole cell lysate (#K151CAD) ou phospho-Akt (Ser473)/Total Akt whole cell lysate (#K151OOD). L'anticorps primaire spécifique de P-AKT-S473 (Kit #K151CAD) est coaté sur une électrode dans chaque puits des plaques 96 puits du kit MSD : après ajoût d'un lysat de protéines dans chaque puits, la révélation du signal se fait par l'addition d'un anticorps secondaire de détection marqué avec un composé électrochimioluminescent. La procédure suivie est celle décrite dans le kit.

Le jour 1, les cellules PC3 sont ensemencées en plaques 96 puits (TPP, #92096) à la concentration de 35000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2h à 37°C en présence de 5% CO2. Les molécules diluées dans du diméthylsulfoxyde (DMSO Sigma #D2650), sont ajoutées à partir d'une solution mère concentrée 20 fois, le pourcentage final de DMSO étant de 0.1 %. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Pour cela, après aspiration du milieu de culture, 50µl de tampon de lyse Tris Lysis Buffer complet du kit MSD contenant les solutions d'inhibiteurs de protéases et phosphatases sont ajoutés dans les puits et les cellules sont lysées pendant 1 H à 4°C sous agitation. A ce stade les plaques contenant les lysats peuvent être congelées à -20°C ou à -80°C.

Les puits des plaques 96 puits du kit MSD sont saturés pendant 1h à température ambiante avec la solution bloquante du kit MSD. Quatre lavages sont effectués avec 150µl de solution de lavage Tris Wash Buffer du kit MSD. Les lysats préparés précédemment sont transférés dans les plaques Multi-spot 96 puits du kit MSD et incubés pendant 1 h à température ambiante, sous agitation. Quatre lavages sont effectués avec 150µl de solution de lavage Tris Wash Buffer du kit MSD. 25µl de la solution MSD sulfo-tag détection antibody sont ajoutés dans les puits et incubés pendant 1h à température ambiante, sous agitation. Quatre lavages sont effectués avec 150µl de solution de lavage Tris Wash Buffer du kit MSD. 150µl de tampon de révélation Read Buffer du kit MSD sont ajoutés dans les puits et les plaques sont lues immédiatement sur l'instrument S12400 de Meso Scale Discovery.

L'appareil mesure un signal pour chaque puits. Des puits sans cellules et contenant le tampon de lyse servent à déterminer le bruit de fond qui sera soustrait à toutes les mesures (min). Les puits contenant des cellules en absence de produit et en présence de 0.1% DMSO sont considérés comme le 100 % de signal (max). Le calcul du pourcentage d'inhibition est fait pour chaque concentration de produit testé selon la formule suivante : (1- ((essai-min)/(max-min)))x100.

L'activité du produit est traduite en CI₅₀, obtenue à partir d'une courbe dose-réponse de différentes concentrations testées et représentant la dose donnant 50 % d'inhibition spécifique (CI₅₀ absolue). 2 expériences indépendantes permettent de calculer la moyenne des CI₅₀ₛ.

L'activité inhibitrice des molécules sur l'autophagie est mesurée par la translocation de la protéine LC3 du cytoplasme vers les autophagososmes. Pour cela les cellules Hela ont été transfectées avec un vecteur codant pour la protéine chimérique GFP-LC3. Un clone Hela exprimant la protéine GFP-LC3 de manière stable a été sélectionné. La translocation de la protéine LC3 est déterminée en mesurant le nombre de cellules présentant des granulations de LC3 après un stress métabolique, à l'aide d'un cytomètre d'analyse automatique d'images iCyte (Compucyte)

### Etude de la translocation de la protéine LC3 dans les cellules humaines Hela mesurée parcytomètre d'analyse d'images (Test C):

Le jour 1, les cellules Hela GFP-LC3 sont ensemencées en plaques 96 puits coatées poly D lysine (Greiner, #655946) à la concentration de 15000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont lavées deux fois avec de l'EBSS (Sigma#E3024).. Les cellules sont ensuite incubées dans de l'EBSS, 10µM d'hydroxychloroquine et des produits à tester pendant 2h à 37°C en présence de 5% CO2. Les molécules sont diluées dans du diméthylsulfoxyde (DMSO Sigma #D2650). Le pourcentage final de DMSO étant de 0.1%. Les molécules sont testées à des concentrations croissantes dans une gamme pouvant s'étendre de 10 nM à 1µM.

Après cette incubation, les cellules sont fixées avec 4% paraformaldéhyde (Sigma#HT501128 4L) pendant 10min. Les cellules sont ensuite lavées 2 fois avec du PBS puis les noyaux colorées avec 2µg/ml de Hoechst 33342 (Invitrogen#H3570) Les plaques 96 puits sont ensuite lues avec le cytomètre analyse d'image iCyte (Compucyte). L'analyseur quantifie le nombre de cellules présentant des granulations de LC3. Une cellule est considérée comme positive lorsqu'elle présente au moins 4 granulations de LC3. Le pourcentage de cellules présentant plus de 4 granulations est calculé par rapport au nombre total de cellules.

L'activité du produit est traduite en CI50, obtenue à partir d'une courbe dose-réponse de différentes concentrations testées et représentant la dose donnant 50 % d'inhibition spécifique (CI50 absolue). 2 expériences indépendantes permettent de calculer la moyenne des CI50s.

Les résultats obtenus pour les produits en exemples dans la partie expérimentale sont donnés dans le tableau de résultats pharmacologiques ci-après:

**Tableau 1 de résultats pharmacologiques :**

| exemple | Test A* | Test B* | Test C* |
|---|---|---|---|
| Exemple 1 | 15 | | 11 |
| Exemple 2 | 315 | | 16 |
| Exemple 3 | 15 | | 20 |
| Exemple 4 | 265 | | 569 |
| Exemple 5 | 10 | 1 | 7 |
| Exemple 6 | | 23 | 310 |
| Exemple 7 | | 1 | 5 |
| Exemple 8 | 75 | | 462 |
| Exemple 9 | 676 | | |
| Exemple 10 | 357 | | 249 |
| Exemple 11 | | 46 | 319 |
| Exemple 12 | 6596 | | 441 |
| Exemple 13 | | 25 | 632 |
| Exemple 14 | | 15 | 337 |
| Exemple 15 | 2715 | 7 | >1000 |
| Exemple 16 | 41 | | 187 |
| Exemple 17 | 350 | | 119 |
| Exemple 18 | 335 | | >1000 |
| Exemple 19 | | 39 | > 1000 |
| Exemple 20 | 60 | | >1000 |
| Exemple 21 | | 24 | 157 |
| Exemple 22 | | 23 | 785 |
| Exemple 23 | | 24 | 663 |
| Exemple 24 | | 9 | 103 |
| Exemple 25 | | 7 | 38 |
| Exemple 26 | | 18 | 270 |
| Exemple 27 | | 10 | 363 |
| Exemple 28 | | 33 | >10000 |
| Exemple 29 | | 22 | >1000 |
| Exemple 30 | | 43 | 212 |
| Exemple 31 | | 150 | 557 |
| Exemple 32 | | 33 | 780 |
| Exemple 33 | | 24 | 221 |
| Exemple 34 | | 22 | 35 |
| Exemple 35 | | 20 | 650 |
| Exemple 36 | | 18 | 157 |
| Exemple 37 | | 18 | 270 |
| Exemple 38 | | 2 | 167 |
| Exemple 39 | | 5 | 237 |
| Exemple 40 | | 3 | 82 |
| Exemple 41 | | 4 | 192 |
| Exemple 42 | | 20 | 310 |
| Exemple 43 | | 16 | 986 |
| Exemple 44 | | 108 | >1000 |
| Exemple 45 | | 14 | >1000 |
| Exemple 46 | | 4 | 16 |

| | | | |
|---|---|---|---|
| * Tests A, B et C : CI50 (nM) | | | |

### Test d'activité anti-paludique

Les tests d'activité antipaludique sont effectués selon la micro méthode radioactive de Desjardins (R.E. Desjardins, C.J. Canfield, J.D. Haynes, J.D. Chulay, Antimicrob. Agents Chemother., 1979, 16, 710-718). Les essais sont réalisés dans des microplaques de 96 puits (Test Plates Réf. 92696, Techno Plastic Products Ag, Zollstrasse 155, CH-8219 Trasadingen). Les souches de P. falciparum sont mises en culture dans des solutions de RPMI 1640 complémenté avec 5 % de sérum humain avec un hématocrite à 2 % et une parasitémie à 1,5 %. Pour chaque essai, les parasites sont incubés avec des concentrations choisies de drogues pendant 48 h à 37 °C en atmosphère humide et à 5 % de CO2. L'artémisinine, l'artésunate ainsi que la chloroquine di-phosphate sont utilisées comme molécules référence. La première dilution de la drogue est réalisée à 1 mg/mL dans du diméthylsulfoxyde. La gamme de dilutions des solutions filles successives est également réalisée dans du diméthylsulfoxyde. Chaque dilution fille est ensuite diluée au 1/50 ème dans du RPMI 1640 complémenté avec 5 % de sérum humain, l'ensemble des dilutions étant réalisé à 37 °C. Ces dilutions sont ensuite ajoutées aux parasites en culture dans les microplaques. Après ajout de la drogue, les parasites sont en culture dans du RPMI 1640 à 5 % de sérum humain et à 1 % de diméthylsulfoxyde. La croissance des parasites est mesurée par l'incorporation d'hypoxanthine tritiée (ajoutée 24 h après le début de l'exposition à la drogue) comparée à l'incorporation en l'absence de drogue.

L'activité du produit est traduite en % inhibition de la croissance de P. falciparum (hautement résistant à la chloroquine souche Fcm29-Cameroun) à 1 uM et 0,1 uM dans un test in vitro utilisant des érythrocytes humains infectés.

Les résultats obtenus pour les produits en exemples dans la partie expérimentale sont donnés dans le tableau 2 de résultats pharmacologiques ci-dessous :

**Tableau 2 de résultats pharmacologiques :**

| Exemple | P. falciparum % Inhibition 1 µM | P. falciparum % Inhibition 0.1 µM |
|---|---|---|
| Exemple 1 | 99 | 79 |
| Exemple 4 | 97 | 19 |
| Exemple 5 | 92 | 97 |
| Exemple 12 | 59 | / |
| Exemple 13 | 52 | / |
| Exemple 24 | 100 | 40 |
| Exemple 27 | 36 | / |
| Exemple 48 | 96 | 89 |
| Exemple 49 | 99 | 81 |
| Exemple 50 | 99 | 79 |
| Exemple 51 | 99 | 75 |
| Exemple 52 | 99 | 75 |
| Exemple 53 | 99 | 75 |
| Exemple 54 | 99 | 83 |
| Exemple 55 | 93 | 81 |
| Exemple 56 | 99 | 94 |

## Revendications

1. Produits de formule (I): 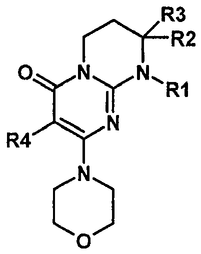 dans laquelle :
R1 représente un radical -L-aryle ou -L-hétéroaryle, tel que L représente :
soit une simple liaison,
soit un radical alkyle linéaire ou ramifié renfermant de 1 à 6 atomes de carbone et éventuellement substitué par un radical hydroxyle,
soit un groupe CO ou -CO-Alk- ,
soit un groupe L'-X où L' représente un radical alkyle linéaire ou ramifié renfermant de 1 à 6 atomes de carbone et X un atome d'oxygène ou de soufre ;
les radicaux aryle et hétéroaryle étant éventuellement substitués par un ou plusieurs radicaux identiques ou différents choisis parmi les atomes d'halogène et les radicaux hydroxyle, CN, nitro, -COOH, -COOalk, -NRxRy,-CONRxRy, -NRxCORy, -NRxCO2Rz, -CORy, alcoxy, phénoxy, alkylthio, alkyle, cycloalkyle et hétérocycloalkyle ;
ces derniers radicaux alcoxy, phénoxy, alkylthio, alkyle et hétérocycloalkyle, étant eux-mêmes éventuellement substitués par un ou plusieurs radicaux identiques ou différents choisis parmi les atomes d'halogène et NRvRw ;
les radicaux hétérocycloalkyle et hétéroaryle pouvant de plus renfermer un radical oxo ;
R2 représente un atome d'hydrogène ou un radical alkyle ;
R3 représente un radical alkyle éventuellement substitué par un ou plusieurs atomes d'halogène ;
R4 représente un atome d'hydrogène ou un atome d'halogène ;
NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogéne ;
Rx, Ry et Rz dans les radicaux -NRxCORy, -CORy et NRxCO2Rz étant choisis parmi les significations indiquées ci-dessus pour Rx, Ry, et Rz ;
tous les radicaux alkyle (alk), alcoxy et alkylthio ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Produits de formule (I) tels que définis à la revendication 1 dans laquelle :
R1 représente un radical -L-phényle ou -L-hétéroaryle, tel que L représente :
soit une simple liaison,
soit un radical alkyle linéaire ou ramifié renfermant de 1 à 6 atomes de carbone et éventuellement substitué par un radical hydroxyle,
soit un groupe CO ou -CO-Alk-,,
soit un groupe L'-X où L' représente un radical alkyle linéaire ou ramifié renfermant de 1 à 6 atomes de carbone et X un atome d'oxygène ou de soufre ;
les radicaux phényle et hétéroaryle étant éventuellement substitués par un ou
plusieurs radicaux identiques ou différents choisis parmi les atomes d'halogène et les radicaux -NRxRy, alcoxy et alkyle ;
ces derniers radicaux alcoxy et alkyle étant eux-mêmes éventuellement substitués par un ou plusieurs radicaux choisis parmi les atomes d'halogène ;
R2 représente un radical alkyle ;
R3 représente un radical alkyle éventuellement substitué par un ou plusieurs atomes d'halogène;
R4 représente un atome d'hydrogène ou un atome de fluor ;
NRxRy étant tel que Rx représente un atome d'hydrogène ou un radical alkyle et Ry représente un atome d'hydrogène ou un radical alkyle; soit Rx et
Ry forment avec l'atome d'azote auquel ils sont liés un radical morpholino ;
tous les radicaux alkyle (alk) ou alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

3. Produits de formule (I) tels que définis à l'une quelconque des revendications 1 ou 2, répondant aux formules suivantes :
- (8S)-9-[2-(4-méthoxyphényl)éthyl]-2-(morpholin-4-yl)-8-(trifluorométhyl)-6,7,8,9-tétrahydro-4H-pyrimido[1,2-a]pyrimidin-4-one
- 9-[2-(4-méthoxyphényl)éthyl]-2-(morpholin-4-yl)-8-(trifluorométhyl)-6,7,8,9-tétrahydro-4H-pyrimido[1,2-a]pyrimidin-4.-one
- (8S)-2-(morpholin-4-yl)-9-(2-phényléthyl)-8-(trifluorométhyl)-6,7,8,9-tétrahydro-4H-pyrimido[1,2-a]pyrimidin-4-one
- (8S)-9-benzyl-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2R)-2-hydroxy-2-phényléthyl]-2-(morpholin-4-yl)-8-(trifluorométhyl)-6,7,8,9-tétrahydro-4H-pyrimido[1,2-a]pyrimidin-4-one
- (8S)-9-[(2S)-2-hydroxy-2-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-[(1R)-1-phényléthyl]-8-(trifluorométhyl)-6,7,8,9-tétrahydro-4H-pyrimido[1,2-a]pyrimidin-4-one
- (8S)-9-[1-(4-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-[(1S)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R)-1-(4-bromophényl)éthyl]-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-phényl-8-(trifluorométhyl)-6,7,8,9-tétrahydro-4H-pyrimido[1,2-a]pyrimidin-4-one
- (8S)-9-(4-fluorophényl)-2-(morpholin-4-yl)-8-(trifluorométhyl)-6,7,8,9-tétrahydro-4H-pyrimido[1,2-a]pyrimidin-4-one
- (8S)-9-(3-fluorophényl)-2-(morpholin-4-yl)-8-(trifluorométhyl)-6,7,8,9-tétrahydro-4H-pyrimido[1,2-a]pyrimidin-4-one
- (8S)-9-(2-fluorophényl)-2-(morpholin-4-yl)-8-(trifluorométhyl)-6,7,8,9-tétrahydro-4H-pyrimido[1,2-a]pyrimidin-4-one
- (8S)-9-[(1R)-1-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(4-fluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(phénylcarbonyl)-8-(trifluorométhyl)-6,7,8,9-tétrahydro-4H-pyrimido[1,2-a]pyrimidin-4-one
- (8S)-2-(morpholin-4-yl)-9-(pyridin-3-yl)-8-(trifluorométhyl)-6,7,8,9-tétrahydro-4H-pyrimido[1,2-a]pyrimidin-4-one
- (8S)-2-(morpholin-4-yl)-9-(pyridin-4-yl)-8-(trifluorométhyl)-6,7,8,9-tétrahydro-4H-pyrimido[1,2-a]pyrimidin-4-one
- (8S)-9-(4-méthylphényl)-2-(morpholin-4-yl)-8-(trifluorométhyl)-6,7,8,9-tétrahydro-4H-pyrimido[1,2-a]pyrimidin-4-one
- (8S)-9-(2-chlorobenzyl)-2-(morpholin-4-yl)-8-(trifluorométhyl)-6,7,8,9-tétrahydro-4H-pyrimido[1,2-a]pyrimidin-4-one
- (8S)-9-(3-fluorobenzyl)-2-(morpholin-4-yl)-8-(trifluorométhyl)-6,7,8,9-tétrahydro-4H-pyrimido[1,2-a]pyrimidin-4-one
- (8S)-9-[2-(2-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-méthoxyphényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthoxybenzyl)-2-(morpholin-4-yl)-8-(trifluorométhyl)-6,7,8,9-tétrahydro-4H-pyrimido[1,2-a]pyrimidin-4-one
- (8S)-9-(4-méthoxyphényl)-2-(morpholin-4-yl)-8-(trifluorométhyl)-6,7,8,9-tétrahydro-4H-pyrimido[1,2-a]pyrimidin-4-one
- (8S)-9-[(2-fluorophényl)carbonyl]-2-(morpholin-4-yl)-8-(trifluorométhyl)-6,7,8,9-tétrahydro-4H-pyrimido[1,2-a]pyrimidin-4-one
- (8S)-9-(3,5-difluorobenzyl)-2-(morpholin-4-yl)-8-(trifluorométhyl)-6,7,8,9-tétrahydro-4H-pyrimido[1,2-a]pyrimidin-4-one
- (8S)-9-(2,4-difluorobenzyl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-9-(2,3,4-trifluorobenzyl)-8-(trifluorométhyl)-6,7,8,9-tétrahydro-4H-pyrimido[1,2-a]pyrimidin-4-one
- (8S)-9-[(5-chloro-1-benzothiophén-3-yl)méthyl]-2-(morpholin-4-yl)-8-(trifluorométhyl)-6,7,8,9-tétrahydro-4H-pyrimido[1,2-a]pyrimidin-4-one
- (8S)-9-[(1 R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1R ou 1S)-1-(4-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-(3-méthylphényl)-2-(morpholin-4-yl)-8-(trifluorométhyl)-6,7,8,9-tétrahydro-4H-pyrimido[1,2-a]pyrimidin-4-one
- (8S)-9-(4-chlorophényl)-2-(morpholin-4-yl)-8-(trifluorométhyl)-6,7,8,9-tétrahydro-4H-pyrimido[1,2-a]pyrimidin-4-one
- (8S)-2-(morpholin-4-yl)-8-(trifluorométhyl)-9-[4-(trifluorométhyl)phényl]-6,7,8,9-tétrahydro-4H-pyrimido[1,2-a]pyrimidin-4-one
- (8S)-9-[(1 R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[(1 R ou 1S)-1-(2-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-[2-(3-fluorophényl)éthyl]-2-(morpholin-4-yl)-8-(trifluorométhyl)-6,7,8,9-tétrahydro-4H-pyrimido[1,2-a]pyrimidin-4-one
- (8S)-9-benzyl-3-fluoro-2-(morpholin-4-yl)-8-(trifluorométhyl)-6,7,8,9-tétrahydro-4H-pyrimido[1,2-a]pyrimidin-4-one
- (8S)-9-(3,5-difluorophényl)-2-(morpholin-4-yl)-8-(trifluorométhyl)-6,7,8,9-tétrahydro-4H-pyrimido[1,2-a]pyrimidin-4-one
- (8S)-9-[(2,6-difluorophényl)carbonyl]-2-(morpholin-4-yl)-8-(trifluorométhyl)-6,7,8,9-tétrahydro-4H-pyrimido[1,2-a]pyrimidin-4-one
- (8S)-9-[(2,4-difluorophényl)carbonyl]-2-(morpholin-4.-yl)-8-(trifluorométhyl)-6,7,8,9-tétrahydro-4H-pyrimido[1,2-a]pyrimidin-4-one
- (8S)-2-(morpholin-4-yl)-9-(phénylacétyl)-8-(trifluorométhyl)-6,7,8,9-tétrahydro-4H-pyrimido[1,2-a]pyrimidin-4-one
- (8S)-9-(2-(3-chlorophényl)éthyl]-2-(morpholin-4-yl)-8-(trifluorométhyl)-6,7,8,9-tétrahydro-4H-pyrimido[1,2-a]pyrimidin-4-one
- 9-((R)-2-Benzo[b]thiophen-2-yl-2-hydroxy-ethyl)-2-morpholin-4-yl-8-(S)-trifluoromethyl-6,7,8,9-tetrahydro-pyrimido[1,2-a]pyrimidin-4-one
- 9-[(S)-2-Hydroxy-2-(3-hydroxy-phenyl)-ethyl]-2-morpholin-4-yl-8-(S)-trifluoromethyl-6,7,8,9-tetrahydro-pyrimido[1,2-a]pyrimidin-4-one
- 2-Dimethylamino-N-{3-[(S)-1-hydroxy-2-((S)-8-morpholin-4-yl-6-oxo-2-trifluoromethyl-3,4-dihydro-2H,6H-pyrimido[1,2-a]pyrimidin-1-yl)-ethyl]-phenyl}-acetamide
- 9-[(S)-2-Hydroxy-2-(2-methoxy-phenyl)-ethyl]-2-morpholin-4-yl-8-(S)-trifluoromethyl-6,7,8,9-tetrahydro-pyrimido[1,2-a]pyrimidin-4-one
- 9-[(S)-2-(4-Fluoro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(4-Chloro-2-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-[(S)-2-(2-Chloro-4-methoxy-phenyl)-2-hydroxy-ethyl]-2-morpholin-4-yl-8-(S)-trifluoromethyl-6,7,8,9-tetrahydro-pyrimido[1,2-a]pyrimidin-4-one
- 9-(2-Hydroxy-3-phenyl-propyl)-2-morpholin-4-yl-8-(S)-trifluoromethyl-6,7,8,9-tetrahydro-pyrimido[1,2-a]pyrimidin-4-on 9-[2-(4-Hydroxy-phenyl)-ethyl]-2-morpholin-4-yl-8-(S)-trifluoromethyl-6,7,8,9-tetrahydro-pyrimido[1,2-a]pyrimidin-4-one
ainsi que les sels d'addition avec les acides minéraux et organiques ou avec les bases minérales et organiques desdits produits de formule (I).

4. Procédé de préparation des produits de formule (I) tels que définis à l'une quelconque des revendications 1 à 3 selon le schéma 1 tel que défini ci-après. 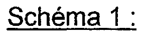 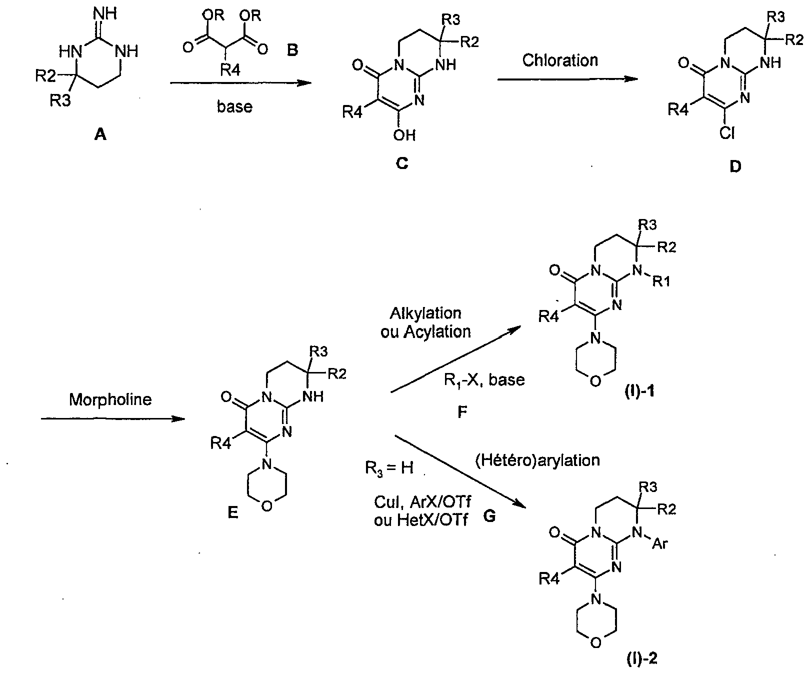 dans lequel les substituants R1, R2, R3 et R4 ont les significations indiquées à l'une quelconque des revendications 1 ou 2 et dans lequel R représente alkyle. X représente un atome de Chlore, brome ou iode ou un groupe sulfonyloxy tel que trifluorométhylsulfonyloxy.

5. Procédé de préparation des produits de formule (I) tels que définis à l'une quelconque des revendications 1 à 3 selon le schéma 2 tel que défini ci-après. 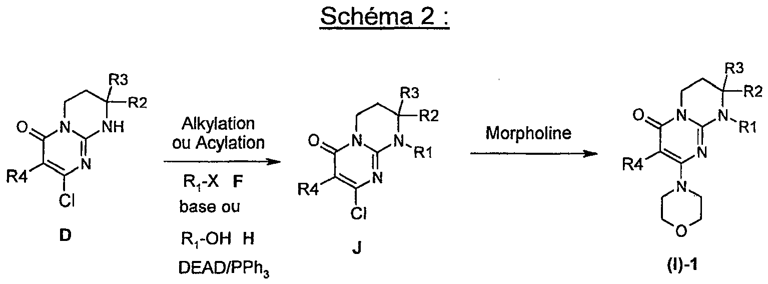 dans lequel les substituants R1, R2, R3 et R4 ont les significations indiquées à l'une quelconque des revendications 1 ou 2. X représente un atome de Chlore, brome ou iode ou un groupe sulfonyloxy tel que trifluorométhylsulfonyloxy.

6. A titre de médicaments, les produits de formule (I) telle que définie à l'une quelconque des revendications 1 à 3, ainsi que les sels d'addition avec les acides minéraux et organiques ou avec les bases minérales et organiques pharmaceutiquement acceptables desdits produits de formule (I).

7. A titre de médicaments, les produits de formule (I) telle que définie à la revendication 3, ainsi que les sels d'addition avec les acides minéraux et organiques ou avec les bases minérales et organiques pharmaceutiquement acceptables desdits produits de formule (I).

8. Compositions pharmaceutiques contenant à titre de principe actif l'un au moins des produits de formule (I) tel que défini à l'une quelconque des revendications 1 à 3, ou un sel pharmaceutiquement acceptable de ce produit et un support pharmaceutiquement acceptable.

9. Produits de formule (I) tels que définis à l'une quelconque des revendications 1 à 3 comme inhibiteurs de phosphorylation de AKT.

10. A titre de produits industriels, les intermédiaires de synthèse de formules C, D, E et J tels que définis aux revendications 4 et 5 ci-dessus et rappelés ci-après : 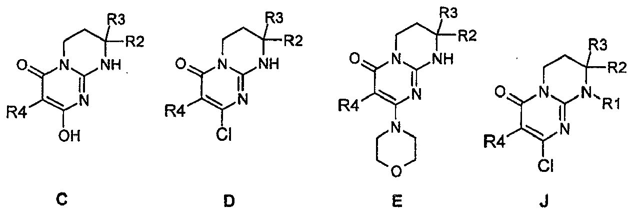 dans lesquels R1, R2, R3 et R4 ont les définitions indiquées à l'une quelconque des revendications 1 à 2.

11. Produits de formule (I) tels que définis à l'une quelconque des revendications 1 à 3 pour leur utilisation pour le traitement de cancers.

12. Produits de formule (I) tels que définis à l'une quelconque des revendications 1 à 3 pour leur utilisation pour le traitement de tumeurs solides ou liquides.

13. Produits de formule (I) tels que définis à l'une quelconque des revendications 1 à 3 pour leur utilisation pour le traitement de cancers résistant à des agents cytotoxiques.

14. Produits de formule (I) tels que définis à l'une quelconque des revendications 1 à 3 pour leur utilisation pour le traitement de tumeurs primaires et/ou de métastases.

15. Produits de formule (I) selon la revendication précédente pour leur utilisation pour le traitement de tumeurs primaires et/ou de métastases dans les cancers gastriques, hépatiques, rénaux, ovariens, du colon, de la prostate, de l'endomètre, du poumon, les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

16. Produits de formule (I) tels que définis à l'une quelconque des revendications 1 à 3 pour leur utilisation pour la chimiothérapie de cancers.

17. Produits de formule (I) tels que définis à l'une quelconque des revendications 1 à 3 pour leur utilisation pour la chimiothérapie de cancers, seuls ou en en association.

18. Produits de formule (I) tels que définis à l'une quelconque des revendications 1 à 3 pour la prévention ou le traitement de maladies lysosomales.

19. Produits de formule (I) selon la revendication précédente pour la prévention ou le traitement de la glycogénose de type II ou maladie de Pompe.

20. Produits tels que définis à la revendication 18 **caractérisés en ce qu'**ils sont utilisés seuls ou en association.

21. Produits de formule (I) tels que définis à l'une quelconque des revendications 1 à 3 pour le traitement de maladies parasitaires.

22. Produits selon la revendication précédente pour la prévention ou le traitement de la malaria, la maladie du sommeil, la maladie de Chagas, les leishmanioses.

## Patentansprüche

1. Produkte der Formel (I): 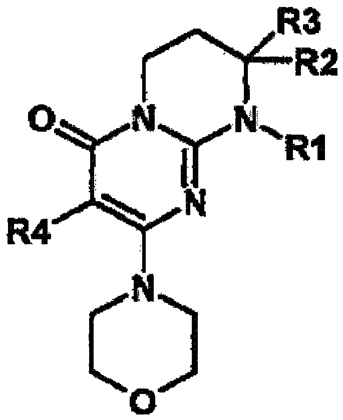 worin:
R1 für einen L-Aryl- oder L-Heteroarylrest steht, wobei L für:
eine Einfachbindung
oder einen linearen oder verzweigten Alkylrest, der 1 bis 6 Kohlenstoffatome enthält und gegebenenfalls durch einen Hydroxylrest substituiert ist,
oder eine CO- oder -CO-Alk-Gruppe
oder eine L'-X-Gruppe, wobei L' für einen linearen oder verzweigten Alkylrest mit 1 bis 6 Kohlenstoffatomen steht und X für ein Sauerstoff- oder
Schwefelatom steht;
steht;
wobei die Aryl- und Heteroarylreste gegebenenfalls durch einen oder mehrere gleiche oder verschiedene Reste, die aus Halogenatomen und Hydroxyl-, CN-, Nitro-, -COOH-, -COOalk-, -NRxRy-, -CONRxRy-, -NRxCORy-, -NRxCO₂Rz-, -CORy-, Alkoxy-, Phenoxy-, Alkylthio-, Alkyl-, Cycloalkyl- und Heterocycloalkylresten ausgewählt sind, substituiert sind;
wobei diese letztgenannten Alkoxy-, Phenoxy-, Alkylthio-, Alkyl- und Heterocycloalkylreste selbst gegebenenfalls durch einen oder mehrere gleiche oder verschiedene Reste, die aus Halogenatomen und NRvRw ausgewählt sind, substituiert sind;
wobei die Heterocycloalkyl- und Heteroarylreste außerdem einen Oxorest enthalten können;
R2 für ein Wasserstoffatom oder einen Alkylrest steht;
R3 für einen Alkylrest, der gegebenenfalls durch ein oder mehrere Halogenatome substituiert ist, steht;
R4 für ein Wasserstoffatom oder ein Halogenatom steht;
wobei NRxRy so beschaffen ist, dass Rx für ein Wasserstoffatom oder einen Alkylrest steht und Ry für ein Wasserstoffatom, einen Cycloalkylrest oder einen Alkylrest, der gegebenenfalls durch einen oder mehrere gleiche oder verschiedene Reste, die aus Hydroxyl-, Alkoxy-, NRvRw- und Heterocycloalkylresten ausgewählt sind, substituiert ist, steht; oder Rx und Ry mit dem Stickstoffatom, an das sie gebunden sind, einen 3-bis 10-gliedrigen cyclischen Rest bilden, der gegebenenfalls ein oder mehrere aus O, S, NH und N-Alkyl ausgewählte Heteroatome enthält und gegebenenfalls substituiert ist;
wobei NRvRw so beschaffen ist, dass Rv für ein Wasserstoffatom oder einen Alkylrest steht und Rw für ein Wasserstoffatom, einen Cycloalkylrest oder einen Alkylrest, der gegebenenfalls durch einen oder mehrere gleiche oder verschiedene Reste, die aus Hydroxyl-, Alkoxy- und Heterocycloalkylresten ausgewählt sind, substituiert ist, steht; oder Rv und Rw mit dem Stickstoffatom, an das sie gebunden sind, einen 3- bis 10-gliedrigen cyclischen Rest bilden, der gegebenenfalls ein oder mehrere aus O, S, NH und N-Alkyl ausgewählte Heteroatome enthält und gegebenenfalls substituiert ist;
wobei die cyclischen Reste, die Rx und Ry bzw. Rv und Rw bilden können, mit dem Stickstoffatom, an das sie gebunden sind, gegebenenfalls durch einen oder mehrere gleiche oder verschiedene Reste, die aus Halogenatomen und Alkyl-, Hydroxyl-, Oxo-, Alkoxy-, NH₂-, NHalk- und N(alk)₂-Resten ausgewählt sind, substituiert sind;
Rz für die Werte von Ry mit Ausnahme von Wasserstoff steht;
wobei Rx, Ry und Rz in den -NRxCORy-, -CORy- und NRxCO₂Rz-Resten aus den oben für Rx, Ry und Rz angegebenen Bedeutungen ausgewählt sind;
wobei alle obigen Alkyl- (alk-), Alkoxy- und Alkylthioreste linear oder verzweigt sind und 1 bis 6 Kohlenstoffatome enthalten,
wobei die Produkte der Formel (I) in allen möglichen racemischen, enantiomeren und diastereoisomeren isomeren Formen vorliegen, sowie die Additionssalze der Produkte der Formel (I) mit anorganischen und organischen Säuren oder mit anorganischen und organischen Basen.

2. Produkte der Formel (I) gemäß Anspruch 1, worin:
R1 für einen L-Phenyl- oder L-Heteroarylrest steht, wobei L für:
eine Einfachbindung
oder einen linearen oder verzweigten Alkylrest,
der 1 bis 6 Kohlenstoffatome enthält und gegebenenfalls durch einen Hydroxylrest substituiert ist, oder eine CO- oder -CO-Alk-Gruppe oder eine L'-X-Gruppe, wobei L' für einen linearen oder verzweigten Alkylrest mit 1 bis 6 Kohlenstoffatomen steht und X für ein Sauerstoff- oder
Schwefelatom steht;
steht;
wobei die Phenyl- und Heteroarylreste gegebenenfalls durch einen oder mehrere gleiche oder
verschiedene Reste, die aus Halogenatomen und -NRxRy-, Alkoxy- und Alkylresten ausgewählt sind, substituiert sind;
wobei diese letztgenannten Alkoxy- und Alkylreste selbst gegebenenfalls durch einen oder mehrere Reste, die aus Halogenatomen ausgewählt sind,
substituiert sind;
R2 für einen Alkylrest steht;
R3 für einen Alkylrest, der gegebenenfalls durch ein oder mehrere Halogenatome substituiert ist, steht;
R4 für ein Wasserstoffatom oder ein Fluoratom steht;
wobei NRxRy so beschaffen ist, dass Rx für ein Wasserstoffatom oder einen Alkylrest steht und Ry für ein Wasserstoffatom oder einen Alkylrest steht; oder Rx und Ry mit dem Stickstoffatom, an das sie gebunden sind, einen Morpholinorest bilden;
wobei alle obigen Alkyl- (alk-) oder Alkoxyreste linear oder verzweigt sind und 1 bis 6 Kohlenstoffatome enthalten,
wobei die Produkte der Formel (I) in allen möglichen racemischen, enantiomeren und diastereoisomeren isomeren Formen vorliegen, sowie die Additionssalze der Produkte der Formel (I) mit anorganischen und organischen Säuren oder mit anorganischen und organischen Basen.

3. Produkte der Formel (I) gemäß einem der Ansprüche 1 oder 2, die den folgenden Formeln entsprechen:
- (8S)-9-[2-(4-Methoxyphenyl)ethyl]-2-(morpholin-4-yl)-8-(trifluormethyl)-6,7,8,9-tetrahydro-4H-pyrimido[1,2-a]pyrimidin-4-on
- 9-[2-(4-Methoxyphenyl)ethyl]-2-(morpholin-4-yl)-8-(trifluormethyl)-6,7,8,9-tetrahydro-4H-pyrimido-[1,2-a]pyrimidin-4-on
- (8S)-2-(Morpholin-4-yl)-9-(2-phenylethyl)-8-(trifluormethyl)-6,7,8,9-tetrahydro-4H-pyrimido-[1,2-a]pyrimidin-4-on
- (8S)-9-Benzyl-2-(morpholin-4-yl)-8-(trifluormethyl)-8,7,8,9-tetrahydro-4H-pyrimido[1,2-a]-pyrimidin-4-on
- (8S)-9-[(2S)-2-Hydroxy-2-phenylethyl]-2-(morpholin-4-yl)-8-(trifluormethyl)-6,7,8,9-tetrahydro-4H-pyrimido[1,2-a]pyrimidin-4-on
- (8S)-9-[(2R)-2-Hydroxy-2-phenylethyl]-2-(morpholin-4-yl)-8-(trifluormethyl)-6,7,8,9-tetrahydro-4H-pyrimido[1,2-a]pyrimidin-4-on
- (8S)-9-[(2S)-2-Hydroxy-2-(4-methoxyphenyl)ethyl]-2-(morpholin-4-yl)-8-(trifluormethyl)-6,7,8,9-tetrahydro-4H-pyrimido[1,2-a]pyrimidin-4-on
- (8S)-2-(Morpholin-4-yl)-9-[(1R)-1-phenylethyl]-8-(trifluormethyl)-6,7,8,9-tetrahydro-4H-pyrimido-[1,2-a]pyrimidin-4-on
- (8S)-9-[1-(4-Methoxyphenyl)ethyl]-2-(morpholin-4-yl)-8-(trifluormethyl)-6,7,8,9-tetrahydro-4H-pyrimido[1,2-a]pyrimidin-4-on
- (8S)-9-[(1S)-1-(4-Bromphenyl)ethyl]-2-(morpholin-4-yl)-8-(trifluormethyl)-6,7,8,9-tetrahydro-4H-pyrimido[1,2-a]pyrimidin-4-on
- (8S)-9-[(1R)-1-(4-Bromphenyl)ethyl]-2-(morpholin-4-yl)-8-(trifluormethyl)-6,7,8,9-tetrahydro-4H-pyrimido[1,2-a]pyrimidin-4-on
- (8S)-2-(Morpholin-4-yl)-9-phenyl-8-(trifluormethyl)-6,7,8,9-tetrahydro-4H-pyrimido[1,2-a]-pyrimidin-4-on
- (8S)-9-(4-Fluorphenyl)-2-(morpholin-4-yl)-8-(trifluormethyl)-6,7,8,9-tetrahydro-4H-pyrimido[1,2-a]pyrimidin-4-on
- (8S)-9-(3-Fluorphenyl)-2-(morpholin-4-yl)-8-(trifluormethyl)-6,7,8,9-tetrahydro-4H-pyrimido[1,2-a]pyrimidin-4-on
- (8S)-9-(2-Fluorphenyl)-2-(morpholin-4-yl)-8-(trifluormethyl)-6,7,8,9-tetrahydro-4H-pyrimido[1,2-a]pyrimidin-4-on
- (8S)-9-[(1R)-1-(3-Fluorphenyl)ethyl]-2-(morpholin-4-yl)-8-(trifluormethyl)-6,7,8,9-tetrahydro-4H-pyrimido[1,2-a]pyrimidin-4-on
- (8S)-9-(4-Fluorbenzyl)-2-(morpholin-4-yl)-8-(trifluormethyl)-6,7,8,9-tetrahydro-4H-pyrimido[1,2-a]pyrimidin-4-on
- (8S)-2-(Morpholin-4-yl)-9-(phenylcarbonyl)-8-(trifluormethyl)-6,7,8,9-tetrahydro-4H-pyrimido-[1,2-a]pyrimidin-4-on
- (8S)-2-Morpholin-4-yl)-9-(pyridin-3-yl)-8-(trifluormethyl)-6,7,8,9-tetrahydro-4H-pyrimido-[1,2-a]pyrimidin-4-on
- (8S)-2-(Morpholin-4-yl)-9-(pyridin-4-yl)-8-(trifluormethyl)-6,7,8,9-tetrahydro-4H-pyrimido-[1,2-a]pyrimidin-4-on
- (8S)-9-(4-Methylphenyl)-2-(morpholin-4-yl)-8-(trifluormethyl)-6,7,8,9-tetrahydro-4H-pyrimido-[1,2-a]pyrimidin-4-on
- (8S)-9-(2-Chlorbenzyl)-2-(morpholin-4-yl)-8-(trifluormethyl)-6,7,8,9-tetrahydro-4H-pyrimido-[1,2-a]pyrimidin-4-on
- (8S)-9-(3-Fluorbenzyl)-2-(morpholin-4-yl)-8-(trifluormethyl)-6,7,8,9-tetrahydro-4H-pyrimido-[1,2-a]pyrimidin-4-on
- (8S)-9-[2-(2-Methoxyphenyl)ethyl]-2-(morpholin-4-yl)-8-(trifluormethyl)-6,7,8,9,tetrahydro-4H-pyrimido[1,2-a]pyrimidin-4-on
- (8S)-9-[2-(3-Methoxyphenyl)ethyl]-2-(morpholin-4-yl)-8-(trifluormethyl)-6,7,8,9-tetrahydro-4H-pyrimido[1,2-a]pyrimidin-4-on
- (8S)-9-(3-Methoxybenzyl)-2-(morpholin-4-yl)-8-(trifluormethyl)-6,7,8,9-tetrahydro-4H-pyrimido-[1,2-a]pyrimidin-4-on
- (8S)-9-(4-Methoxyphenyl)-2-(morpholin-4-yl)-8-(trifluormethyl)-6,7,8,9-tetrahydro-4H-pyrimido-[1,2-a]pyrimidin-4-on
- (8S)-9-[(2-Fluorphenyl)carbonyl]-2-(morpholin-4-yl)-8-(trifluormethyl)-6,7,8,9-tetrahydro-4H-pyrimido[1,2-a]pyrimidin-4-on
- (8S)-9-(3,5-Difluorbenzyl)-2-(morpholin-4-yl)-8-(trifluormethyl)-6,7,8,9-tetrahydro-4H-pyrimido-[1,2-a]pyrimidin-4-on
- (8S)-9-(2,4-Difluorbenzyl)-2-(morpholin-4-yl)-8-(trifluormethyl)-6,7,8,9-tetrahydro-4H-pyrimido-[1,2-a]pyrimidin-4-on
- (8S)-2-(Morpholin-4-yl)-9-(2,3,4-trifluorbenzyl)-8-(trifluormethyl)-6,7,8,9-tetrahydro-4H-pyrimido-[1,2-a]pyrimidin-4-on
- (8S)-9-[(5-Chlor-1-benzothiophen-3-yl)methyl]-2-(morpholin-4-yl)-8-(trifluormethyl)-6,7,8,9-tetrahydro-4H-pyrimido[1,2-a]pyrimidin-4-on
- (8S)-9-[(1R oder 1S)-1-(4-Fluorphenyl)ethyl]-2-(morpholin-4-yl)-8-(trifluormethyl)-6,7,8,9-tetrahydro-4H-pyrimido[1,2-a]pyrimidin-4-on
- (8S)-9-[(1R oder 1S)-1-(4-Fluorphenyl)ethyl]-2-(morpholin-4-yl)-8-(trifluormethyl)-6,7,8,9-tetrahydro-4H-pyrimido[1,2-a]pyrimidin-4-on
- (8S)-9-(3-Methylphenyl)-2-(morpholin-4-yl)-8-(trifluormethyl)-6,7,8,9-tetrahydro-4H-pyrimido-[1,2-a]pyrimidin-4-on
- (8S)-9-(4-Chlorphenyl)-2-(morpholin-4-yl-8-(trifluormethyl)-6,7,8,9-tetrahydro-4H-pyrimido-[1,2-a]pyrimidin-4-on
- (8S)-2-(Morpholin-4-yl)-8-(trifluormethyl)-9-[4-(trifluormethyl)phenyl]-6,7,8,9-tetrahydro-4H-pyrimido[1,2-a]pyrimidin-4-on
- (8S)-9-[(1R oder 1S)-1-(2-Fluorphenyl)ethyl]-2-(morpholin-4-yl)-8-(trifluormethyl)-6,7,8,9-tetrahydro-4H-pyrimido[1,2-a]pyrimidin-4-on
- (8S)-9-[(1R oder 1S)-1-(2-Fluorphenyl)ethyl]-2-(morpholin-4-yl)-8-(trifluormethyl)-6,7,8,9-tetrahydro-4H-pyrimido[1,2-a]pyrimidin-4-on
- (8S)-9-[2-(3-Fluorphenyl)ethyl]-2-(morpholin-4-yl)-8-(trifluormethyl)-5,7,8,9-tetrahydro-4H-pyrimido[1,2-a]pyrimidin-4-on
- (8S)-9-Benzyl-3-fluor-2-(morpholin-4-yl)-8-(trifluormethyl)-6,7,8,9-tetrahydro-4H-pyrimido[1,2-a]pyrimidin-4-on
- (8S)-9-(3,5-Difluorphenyl)-2-(morpholin-4-yl)-8-(trifluormethyl)-6,7,8,9-tetrahydro-4H-pyrimido-[1,2-a]pyrimidin-4-on
- (8S)-9-[(2,6-Difluorphenyl)carbonyl]-2-(morpholin-4-yl)-8-(trifluormethyl)-6,7,8,9-tetrahydro-4H-pyrimido[1,2-a]pyrimidin-4-on
- (8S)-9-[(2,4-Difluorphenyl)carbonyl]-2-(morpholin-4-yl)-8-(trifluormethyl)-6,7,8,9-tetrahydro-4H-pyrimido[1,2-a]pyrimidin-4-on
- (8S)-2-(Morpholin-4-yl)-9-(phenylacetyl)-8-(trifluormethyl)-6,7,8,9-tetrahydro-4H-pyrimido-[1,2-a]pyrimidin-4-on
- (8S)-9-[2-(3-Chlorphenyl)ethyl]-2-(morpholin-4-yl)-8-(trifluormethyl)-6,7,8,9-tetrahydro-4H-pyrimido[1,2-a]pyrimidin-4-on
- 9-((R)-2-Benzo[b]thiophen-2-yl-2-hydroxyethyl)-2-morpholin-4-yl-8-(S)-trifluormethyl-6,7,8,9-tetrahydropyrimido[1,2-a]pyrimidin-4-on
- 9-[(S)-2-Hydroxy-2-(3-hydroxyphenyl)ethyl]-2-morpholin-4-yl-8-(S)-trifluormethyl-6,7,8,9-tetrahydropyrimido[1,2-a]pyrimidin-4-on
- 2-Dimethylamino-N-{3-[(S)-1-hydroxy-2-((S)-8-morpholin-4-yl-6-oxo-2-trifluormethyl-3,4-dihydro-2H,6H-pyrimido[1,2-a]pyrimidin-1-yl)ethyl]phenyl}-acetamid
- 9-[(S)-2-Hydroxy-2-(2-methoxyphenyl)ethyl]-2-morpholin-4-yl-8-(S)-trifluormethyl-6,7,8,9-tetrahydropyrimido[1,2-a]pyrimidin-4-on
- 9-[(S)-2-(4-Fluor-2-methoxyphenyl)-2-hydroxyethyl]-2-morpholin-4-yl-8-(S)-trifluormethyl-6,7,8,9-tetrahydropyrimido[1,2-a]pyrimidin-4-on
- 9-[(S)-2-(4-Chlor-2-methoxyphenyl)-2-hydroxyethyl]-2-morpholin-4-yl-8-(S)-trifluormethyl-6,7,8,9-tetrahydropyrimido[1,2-a]pyrimidin-4-on
- 9-[(S)-2-(2-Chlor-4-methoxyphenyl)-2-hydroxyethyl]-2-morpholin-4-yl-8-(S)-trifluormethyl-6,7,8,9-tetrahydropyrimido[1,2-a]pyrimidin-4-on
- 9-(2-Hydroxy-3-phenylpropyl)-2-morpholin-4-yl-8-(S)-trifluormethyl-6,7,8,9-tetrahydropyrimido[1,2-a]pyrimidin-4-on
- 9-[2-(4-Hydroxyphenyl)ethyl]-2-morpholin-4-yl-8-(S)-trifluormethyl-6,7,8,9-tetrahydropyrimido[1,2-a]pyrimidin-4-on
sowie die Additionssalze der Produkte der Formel (I) mit anorganischen und organischen Säuren oder mit anorganischen und organischen Basen.

4. Verfahren zur Herstellung der Produkte der Formel (I) gemäß einem der Ansprüche 1 bis 3 gemäß Schema 1 gemäß nachstehender Definition: 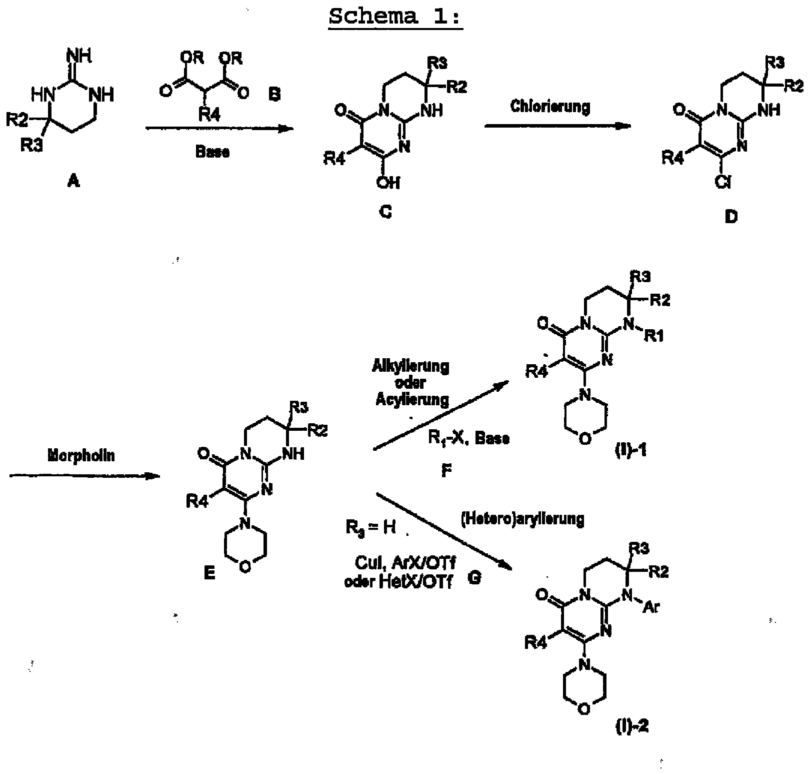 wobei die Substituenten R1, R2, R3 und R4 die in einem der Ansprüche 1 oder 2 angegebenen Bedeutungen besitzen und R für Alkyl steht und X für ein Chlor-, Brom- oder Iodatom oder eine Sulfonyloxygruppe wie Trifluormethylsulfonyloxy steht.

5. Verfahren zur Herstellung der Produkte der Formel (I) gemäß einem der Ansprüche 1 bis 3 gemäß Schema 2 gemäß nachstehender Definition: 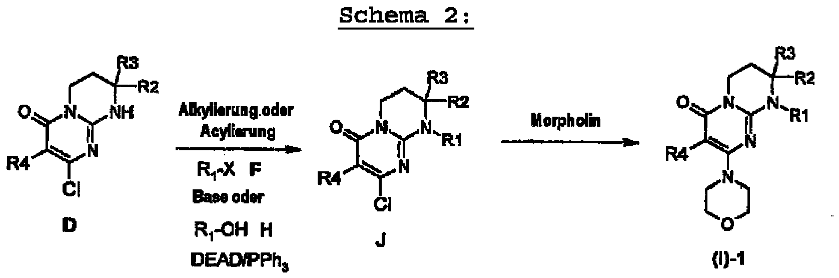 wobei die Substituenten R1, R2, R3 und R4 die in einem der Ansprüche 1 oder 2 angegebenen Bedeutungen besitzen und X für ein Chlor-, Brom- oder Iodatom oder eine Sulfonyloxygruppe wie Trifluormethylsulfonyloxy steht.

6. Produkte der Formel (I) gemäß einem der Ansprüche 1 bis 3 sowie die Additionssalze der Produkte der Formel (I) mit anorganischen und organischen Säuren oder mit anorganischen und organischen Basen, die pharmazeutisch unbedenklich sind, als Medikamente.

7. Produkte der Formel (I) gemäß Anspruch 3 sowie die Additionssalze der Produkte der Formel (I) mit anorganischen und organischen Säuren oder mit anorganischen und organischen Basen, die pharmazeutisch unbedenklich sind, als Medikamente.

8. Pharmazeutische Zusammensetzungen, die als Wirkstoff mindestens eines der Produkte der Formel (I) gemäß einem der Ansprüche 1 bis 3 oder ein pharmazeutisch unbedenkliches Salz dieses Produkts und einen pharmazeutisch unbedenklichen Träger enthalten.

9. Produkte der Formel (I) gemäß einem der Ansprüche 1 bis 3 als Inhibitoren der AKT-Phosphorylierung.

10. Synthesezwischenprodukte der Formeln C, D, E und J gemäß den Ansprüchen 4 und 5, die nachstehend noch einmal aufgeführt sind: 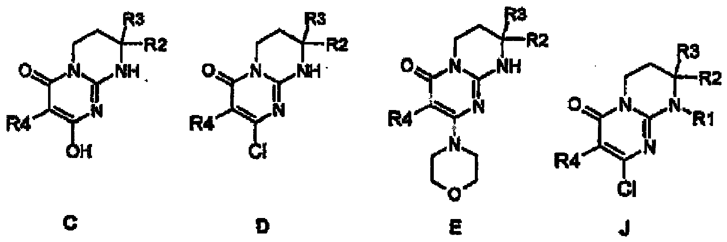 wobei die Substituenten R1, R2, R3 und R4 die in einem der Ansprüche 1 bis 2 angegebenen Bedeutungen besitzen, als technische Produkte.

11. Produkte der Formel (I) gemäß einem der Ansprüche 1 bis 3 zur Verwendung für die Behandlung von Krebserkrankungen.

12. Produkte der Formel (I) gemäß einem der Ansprüche 1 bis 3 zur Verwendung für die Behandlung von soliden oder flüssigen Tumoren.

13. Produkte der Formel (I) gemäß einem der Ansprüche 1 bis 3 zur Verwendung für die Behandlung von Krebserkrankungen, die gegenüber Zytotoxika resistent sind.

14. Produkte der Formel (I) gemäß einem der Ansprüche 1 bis 3 zur Verwendung für die Behandlung von primären Tumoren und/oder Metastasen.

15. Produkte der Formel (I) gemäß einem der Ansprüche 1 bis 3 zur Verwendung für die Behandlung von primären Tumoren und/oder Metastasen bei Krebserkrankungen des Magens, der Leber, der Niere, der Eierstöcke, des Kolons, der Prostata, des Endometriums, der Lunge, Glioblastomen, Krebserkrankungen der Schilddrüse, der Blase, der Brust, bei Melanom, bei lymphoiden oder myeloiden hämatopoetischen Tumoren, bei Sarkomen, bei Krebserkrankungen des Gehirns, des Kehlkopfs, des Lymphsystems, Krebserkrankungen der Knochen und der Bauchspeicheldrüse und bei Hamartomen.

16. Produkte der Formel (I) gemäß einem der Ansprüche 1 bis 3 zur Verwendung für die Chemotherapie von Krebserkrankungen.

17. Produkte der Formel (I) gemäß einem der Ansprüche 1 bis 3 zur Verwendung für die Chemotherapie von Krebserkrankungen, allein oder in Kombination.

18. Produkte der Formel (I) gemäß einem der Ansprüche 1 bis 3 zur Prävention oder Behandlung von lysosomalen Krankheiten.

19. Produkte der Formel (I) nach dem vorhergehenden Anspruch zur Prävention oder Behandlung von Glykogenose Typ II oder Pompe-Krankheit.

20. Produkte gemäß Anspruch 18, **dadurch gekennzeichnet, dass** sie allein oder in Kombination verwendet werden.

21. Produkte der Formel (I) gemäß einem der Ansprüche 1 bis 3 zur Behandlung von Parasitenkrankheiten.

22. Produkte nach dem vorhergehenden Anspruch zur Prävention oder Behandlung von Malaria, Schlafkrankheit, Chagas-Krankheit oder Leishmaniosen.

## Claims

1. Products of formula (I): 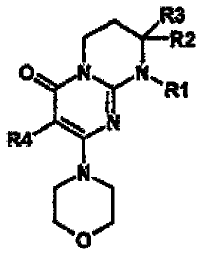 in which:
R1 represents an -L-aryl or -L-heteroaryl radical, such that L represents:
either a single bond,
or a linear or branched alkyl radical which includes from 1 to 6 carbon atoms and which is optionally substituted by a hydroxyl radical,
or a CO or -CO-Alk- group,
or an L'-X group, where L' represents a linear or branched alkyl radical including from 1 to 6 carbon atoms and X represents an oxygen or sulphur atom;
the aryl and heteroaryl radicals optionally being substituted by one or more identical or different radicals chosen from halogen atoms and hydroxyl, CN, nitro, -COOH, -COOalk, -NRxRy, -CONRxRy, -NRxCORy, -NRxCO₂Rz, -CORy, alkoxy, phenoxy, alkylthio, alkyl, cycloalkyl and heterocycloalkyl radicals;
the latter alkoxy, phenoxy, alkylthio, alkyl and heterocycloalkyl radicals being themselves optionally substituted by one or more identical or different radicals chosen from halogen atoms and NRvRw;
it being possible in addition for the heterocycloalkyl and heteroaryl radicals to include an oxo radical;
R2 represents a hydrogen atom or an alkyl radical;
R3 represents an alkyl radical optionally substituted by one or more halogen atoms;
R4 represents a hydrogen atom or a halogen atom;
NRxRy being such that Rx represents a hydrogen atom or an alkyl radical and Ry represents a hydrogen atom, a cycloalkyl radical or an alkyl radical optionally substituted by one or more identical or different radicals chosen from hydroxyl, alkoxy, NRvRw and heterocycloalkyl radicals; or Rx and Ry form, with the nitrogen atom to which they are bonded, a cyclic radical including from 3 to 10 ring members and optionally one or more other heteroatoms chosen from 0, S, NH and N-alkyl, this cyclic radical optionally being substituted;
NRvRw being such that Rv represents a hydrogen atom or an alkyl radical and Rw represents a hydrogen atom, a cycloalkyl radical or an alkyl radical optionally substituted by one or more identical or different radicals chosen from hydroxyl, alkoxy or heterocycloalkyl radicals; or Rv and Rw form, with the nitrogen atom to which they are bonded, a cyclic radical including from 3 to 10 ring members and optionally one or more other heteroatoms chosen from O, S, NH and
N-alkyl, this cyclic radical optionally being substituted;
the cyclic radicals which Rx and Ry or Rv and Rw respectively can form with the nitrogen atom to which they are bonded being optionally substituted by one or more identical or different radicals chosen from halogen atoms or alkyl, hydroxyl, oxo, alkoxy, NH₂, NHalk and N(alk)₂ radicals;
Rz represents the values of Ry with the exception of hydrogen;
Rx, Ry and Rz in the -NRxCORy, -CORy and NRxCO₂Rz radicals being chosen from the meanings indicated above for Rx, Ry and Rz;
all the alkyl (alk), alkoxy and alkylthio radicals above being linear or branched and including from 1 to 6 carbon atoms,
the said products of formula (I) being in all the possible isomeric forms, racemic, enantiomeric and diastereoisomeric, and also the addition salts with inorganic and organic acids or with inorganic and organic bases of the said products of formula (I).

2. Products of formula (I) as defined in Claim 1, in which:
R1 represents an -L-phenyl or -L-heteroaryl radical, such that L represents:
either a single bond,
or a linear or branched alkyl radical which includes from 1 to 6 carbon atoms and which is optionally substituted by a hydroxyl radical,
or a CO or -CO-Alk- group,
or an L'-X group, where L' represents a linear or branched alkyl radical including from 1 to 6 carbon atoms and X represents an oxygen or sulphur atom;
the phenyl and heteroaryl radicals being optionally substituted by one or more identical or different radicals chosen from halogen atoms and -NRxRy, alkoxy and alkyl radicals;
these latter alkoxy and alkyl radicals being themselves optionally substituted by one or more radicals chosen from halogen atoms;
R2 represents an alkyl radical;
R3 represents an alkyl radical optionally substituted by one or more halogen atoms;
R4 represents a hydrogen atom or a fluorine atom;
NRxRy being such that Rx represents a hydrogen atom or an alkyl radical and Ry represents a hydrogen atom or an alkyl radical; or Rx and Ry form, with the nitrogen atom to which they are bonded, a morpholino radical;
all the alkyl (alk) or alkoxy radicals above being linear or branched and including from 1 to 6 carbon atoms,
the said products of formula (I) being in all the possible isomeric forms, racemic, enantiomeric and diastereoisomeric, and also the addition salts with inorganic and organic acids or with inorganic and organic bases of the said products of formula (I).

3. Products of formula (I) as defined in either one of Claims 1 and 2, corresponding to the following formulae:
- (8S)-9-[2-(4-methoxyphenyl)ethyl]-2-(morpholin-4-yl)-8-(trifluoromethyl)-6,7,8,9-tetrahydro-4H-pyrimido[1,2-a]pyrimidin-4-one
- 9-[2-(4-methoxyphenyl)ethyl]-2-(morpholin-4-yl)-8-(trifluoromethyl)-6,7,8,9-tetrahydro-4H-pyrimido[1,2-a]pyrimidin-4-one
- (8S)-2-(morpholin-4-y1)-9-(2-phenylethyl)-8-(trifluoromethyl)-6,7,8,9-tetrahydro-4H-pyrimido-[1,2-a]pyrimidin-4-one
- (8S)-9-benzyl-2-(morpholin-4-yl)-8-(trifluoro-methyl)-8,7,8,9-tetrahydro-4H-pyrimido[1,2-a]-pyrimidin-4-one
- (8S)-9-[(2S)-2-hydroxy-2-phenylethyl]-2-(morpholin-4-yl)-8-(trifluoromethyl)-6,7,8,9-tetrahydro-4H-pyrimido[1,2]pyrimidin-4-one
- (8S)-9-[(2R)-2-hydroxy-2-phenylethyl]-2-(morpholin-4-yl)-8-(trifluoromethyl)-6,7,8,9-tetrahydro-4H-pyrimido[1,2-a]pyrimidin-4-one
- (8S)-9-[(2S)-2-hydroxy-2-(4-methoxyphenyl)-ethyl]-2-(morpholin-4-yl)-8-(trifluoromethyl)-6,7,8,9-tetrahydro-4H-pyrimido[1,2-a]pyrimidin-4-one
- (8S)-2-(morpholin-4-yl)-9-[(1R)-1-phenylethyl]-8-(trifluoromethyl)-6,7,8,9-tetrahydro-4H-pyrimido[1,2-a]pyrimidin-4-one
- (8S)-9-[1-(4-methoxyphenyl)ethyl]-2-(morpholin-4-yl)-8-(trifluoromethyl)-6,7,8,9-tetrahydro-4H-pyrimido[1,2-a]pyrimidin-4-one
- (8S)-9-[(1S)-1-(4-bromophenyl)ethyl]-2-(morpholin-4-yl)-8-(trifluoromethyl)-6,7,8,9-tetrahydro-4H-pyrimido[1,2-a]pyrimidin-4-one
- (8S)-9-[(1R)-1-(4-bromophenyl)ethyl]-2-(morpholin-4-yl)-8-(trifluoromethyl)-6,7,8,9-tetrahydro-4H-pyrimido[1,2-a]pyrimidin-4-one
- (8S)-2-(morpholin-4-yl)-9-phenyl-8-(trifluoro-methyl)-6,7,8,9-tetrahydro-4H-pyrimido[1,2-a]-pyrimidin-4-one
- (8S)-9-(4-fluorophenyl)-2-(morpholin-4-yl)-8-(trifluoromethyl)-6,7,8,9-tetrahydro-4H-pyrimido-[1,2-a]pyrimidin-4-one
- (8S)-9-(3-fluorophenyl)-2-(morpholin-4-yl)-8-(trifluoromethyl)-6,7,8,9-tetrahydro-4H-pyrimido-[1,2-a]pyrimidin-4-one
- (8S)-9-(2-fluorophenyl)-2-(morpholin-4-yl)-8-(trifluoromethyl)-6,7,8,9-tetrahydro-4H-pyrimido-[1,2-a]pyrimidin-4-one
- (8S)-9-[(1R)-1-(3-fluorophenyl)ethyl]-2-(morpholin-4-yl)-8-(trifluoromethyl)-6,7,8,9-tetrahydro-4H-pyrimido[1,2-a]pyrimidin-4-one
- (8S)-9-(4-fluorobenzyl)-2-(morpholin-4-yl)-8-(trifluoromethyl)-6,7,8,9-tetrahydro-4H-pyrimido-[1,2-a]pyrimidin-4-one
- (8S)-2-(morpholin-4-yl)-9-(phenylcarbonyl)-8-(trifluoromethyl)-6,7,8,9-tetrahydro-4H-pyrimido-[1,2-a]pyrimidin-4-one
- (8S)-2-(morpholin-4-yl)-9-(pyridin-3-yl)-8-(trifluoromethyl)-6,7,8,9-tetrahydro-4H-pyrimido-[1,2-a]pyrimidin-4-one
- (8S)-2-(morpholin-4-yl)-9-(pyridin-4-yl)-8-(trifluoromethyl)-6,7,8,9-tetrahydro-4H-pyrimido-[1,2-a]pyrimidin-4-one
- (8S)-9-(4-methylphenyl)-2-(morpholin-4-yl)-8-(trifluoromethyl)-6,7,8,9-tetrahydro-4H-pyrimido-[1,2-a]pyrimidin-4-one
- (8S)-9-(2-chlorobenzyl)-2-(morpholin-4-yl)-8-(trifluoromethyl)-6,7,8,9-tetrahydro-4H-pyrimido-[1,2-a]pyrimidin-4-one
- (8S)-9-(3-fluorobenzyl)-2-(morpholin-4-yl)-8-(trifluoromethyl)-6,7,8,9-tetrahydro-4H-pyrimido-[1,2-a]pyrimidin-4-one
- (8S)-9-[2-(2-methoxyphenyl)ethyl]-2-(morpholin-4-yl)-8-(trifluoromethyl)-6,7,8,9-tetrahydro-4H-pyrimido[1,2-a]pyrimidin-4-one
- (8S)-9-[2-(3-methoxyphenyl)ethyl]-2-(morpholin-4-yl)-8-(trifluoromethyl)-6,7,8,9-tetrahydro-4H-pyrimido[1,2-a]pyrimidin-4-one
- (8S)-9-(3-methoxybenzyl)-2-(morpholin-4-yl)-8-(trifluoromethyl)-6,7,8,9-tetrahydro-4H-pyrimido-[1,2-a]pyrimidin-4-one
- (8S)-9-(4-methoxyphenyl)-2-(morpholin-4-yl)-8-(trifluoromethyl)-6,7,8,9-tetrahydro-4H-pyrimido-[1,2-a]pyrimidin-4-one
- (8S)-9-[(2-fluorophenyl)carbonyl]-2-(morpholin-4-yl)-8-(trifluoromethyl)-6,7,8,9-tetrahydro-4H-pyrimido[1,2-a]pyrimidin-4-one
- (8S)-9-(3,5-difluorobenzyl)-2-(morpholin-4-yl)-8-(trifluoromethyl)-6,7,8,9-tetrahydro-4H-pyrimido[1,2-a]pyrimidin-4-one
- (8S)-9-(2,4-difluorobenzyl)-2-(morpholin-4-yl)-8-(trifluoromethyl)-6,7,8,9-tetrahydro-4H-pyrimido[1,2-a]pyrimidin-4-one
- (8S)-9-(morpholin-4-yl)-9-(2,3,4-trifluorobenzyl)-8-(trifluoromethyl)-6,7,8,9-tetrahydro-4H-pyrimido[1,2-a]pyrimidin-4-one
- (8S)-9-[(5-chloro-1-benzothiophen-3-yl)methyl]-2-(morpholin-4-yl)-8-(trifluoromethyl)-6,7,8,9-tetrahydro-4H-pyrimido[1,2-a]pyrimidin-4-one
- (8S)-9-[(1R or 1S)-1-(4-fluorophenyl)ethyl]-2-(morpholin-4-yl)-8-(trifluoromethyl)-6,7,8,9-tetrahydro-4H-pyrimido[1,2-a]pyrimidin-4-one
- (8S)-9-[(1R or 1S)-1-(4-fluorophenyl)ethyl]-2-(morpholin-4-yl)-8-(trifluoromethyl)-6,7,8,9-tetrahydro-4H-pyrimido[1,2-a]pyrimidin-4-one
- (8S)-9-(3-methylphenyl)-2-(morpholin-4-yl)-8(trifluoromethyl)-6,7,8,9-tetrahydro-4H-pyrimido-[1,2-a]pyrimidin-4-one
- (8S)-9-(4-chlorophenyl)-2-(morpholin-4-yl)-8-(trifluoromethyl)-6,7,8,9-tetrahydro-4H-pyrimido-[1,2-a]pyrimidin-4-one
- (8S)-2-(morpholin-4-yl)-8-(trifluoromethyl)-9-[4-(trifluoromethyl)phenyl]-6,7,8,9-tetrahydro-4H-pyrimido[1,2-a]pyrimidin-4-one
- (8S)-9-[(1R or 1S)-1-(2-fluorophenyl)ethyl]-2-(morpholin-4-yl)-8-(trifluoromethyl)-6,7,8,9-tetrahydro-4H-pyrimido[1,2-a]pyrimidin-4-one
- (8S-9-[(1R or 1S)-1-(2-fluorophenyl)ethyl]-2-(morpholin-4-yl)-8-(trifluoromethyl)-6,7,8,9-tetrahydro-4H-pyrimido[1,2-a]pyrimidin-4-one
- (8S)-9-[2-(3-fluorophenyl)ethyl]-2-(morpholin-4-yl)-8-(trifluoromethyl)-6,7,8,9-tetrahydro-4H-pyrimido[1,2-a]pyrimidin-4-one
- (8S)-9-benzyl-3-fluoro-2-(morpholin-4-yl)-8-(trifluoromethyl)-6,7,8,9-tetrahydro-4H-pyrimido-[1,2-a]pyrimidin-4-one
- (8S)-9-(3,5-difluorophenyl)-2-(morpholin-4-yl)-8-(trifluoromethyl)-6,7,8,9-tetrahydro-4H-pyrimido[1,2-a]pyrimidin-4-one
- (8S)-9-[(2,6-difluorophenyl)carbonyl]-2-(morpholin-4-yl)-8-(trifluoromethyl)-6,7,8,9-tetrahydro-4H-pyrimido[1,2-a]pyrimidin-4-one
- (8S)-9-[(2,4-difluorophenyl)carbonyl]-2-(morpholin-4-yl)-8-(trifluoromethyl)-6,7,8,9-tetrahydro-4H-pyrimido[1,2-a]pyrimidin-4-one
- (8S)-2-(morpholin-4-yl)-9-(phenylacetyl)-8-(trifluoromethyl)-6,7,8,9-tetrahydro-4H-pyrimido-[1,2-a]pyrimidin-4-one
- (8S)-9-[2-(3-chlorophenyl)ethyl]-2-(morpholin-4-yl)-8-(trifluoromethyl)-6,7,8,9-tetrahydro-4H-pyrimido[1,2-a]pyrimidin-4-one
- 9-[(R)-2-(Benzo[b]thiophen-2-yl)-2-hydroxyethyl]-2-(morpholin-4-yl)-8-[(S)-trifluoromethyl]-6,7,8,9-tetrahydro-pyrimido[1,2-a]pyrimidin-4-one
- 9-[(S)-2-hydroxy-2-(3-hydroxyphenyl)ethyl]-2-(morpholin-4-yl)-8-[(S)-trifluoromethyl]-6,7,8,9-tetrahydro-pyrimido[1,2-a]pyrimidin-4-one
- 2-dimethylamino-N-(3-[(S)-1-hydroxy-2-((S)-8-(morpholin-4-yl)-6-oxo-2-trifluoromethyl-3,4-dihydro-2H,6H-pyrimido[1,2-a]pyrimidin-1-yl)-ethyl]phenyl}acetamide
- 9-[(S)-2-hydroxy-2-(2-methoxyphenyl)ethyl]-2-(morpholin-4-yl)-8-[(S)-trifluoromethyl]-6,7,8,9-tetrahydro-pyrimido[1,2-a]pyrimidin-4-one
- 9-[(S)-2-(4-fluoro-2-methoxyphenyl)-2-hydroxyethyl]-2-(morpholin-4-yl)-8-[(S)-trifluoromethyl]-6,7,8,9-tetrahydro-pyrimido[1,2-a]pyrimidin-4-one
- 9-[(S)-2-(4-chloro-2-methoxyphenyl)-2-hydroxyethyl]-2-(morpholin-4-yl)-8-[(S)-trifluoromethyl]-6,7,8,9-tetrahydro-pyrimido[1,2-a]pyrimidin-4-one
- 9-[(S)-2-(2-chloro-4-methoxyphenyl)-2-hydroxyethyl]-2-(morpholin-4-yl)-8-[(S)-trifluoromethyl]-6,7,8,9-tetrahydro-pyrimido[1,2-a]pyrimidin-4-one
- 9-(2-hydroxy-3-phenylpropyl)-2-(morpholin-4-yl)-8-[(S)-trifluoromethyl]-6,7,8,9-tetrahydro-pyrimido[1,2-a]pyrimidin-4-one
- 9-[2-(4-hydroxyphenyl)ethyl]-2-(morpholin-4-yl)-8-[(S)-trifluoromethyl]-6,7,8,9-tetrahydro-pyrimido[1,2-a]pyrimidin-4-one
and the addition salts with inorganic and organic acids or with inorganic and organic bases of the said products of formula (I).

4. Process for the preparation of the products of formula (I) as defined in any one of Claims 1 to 3 according to Scheme 1 as defined below: 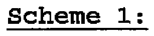 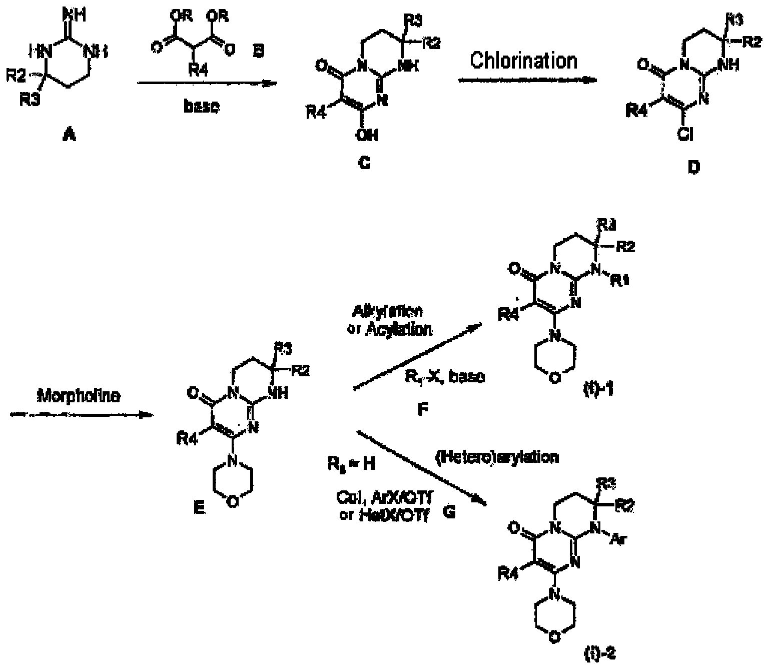 in which the substituents R1, R2, R3 and R4 have the meanings indicated in either one of Claims 1 and 2 and in which R represents alkyl, and X represents a chlorine, bromine or iodine atom or a sulphonyloxy group, such as the trifluoromethyl-sulphonyloxy group.

5. Process for the preparation of the products of formula (I) as defined in any one of Claims 1 to 3 according to Scheme 2 as defined below: 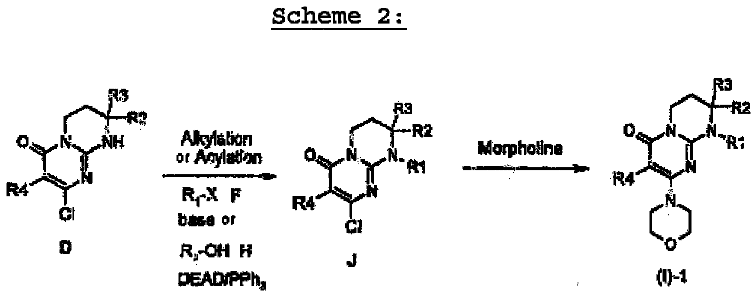 in which the substituents R1, R2, R3 and R4 have the meanings indicated in either one of Claims 1 and 2 and X represents a chlorine, bromine or iodine atom or a sulphonyloxy group, such as the trifluoromethylsulphonyloxy group.

6. As medicaments, the products of formula (I) as defined in any one of Claims 1 to 3 and also the pharmaceutically acceptable addition salts with inorganic and organic acids or with inorganic and organic bases of the said products of formula (I).

7. As medicaments, the products of formula (I) as defined in Claim 3 and also the pharmaceutically acceptable addition salts with inorganic and organic acids or with inorganic and organic bases of the said products of formula (I).

8. Pharmaceutical compositions comprising, as active principle, at least one of the products of formula (I) as defined in any one of Claims 1 to 3 or a pharmaceutically acceptable salt of this product and a pharmaceutically acceptable carrier.

9. Products of formula (I) as defined in any one of Claims 1 to 3 as AKT phosphorylation inhibitors.

10. As industrial products, the synthetic intermediates of formulae C, D, E and J as defined in Claims 4 and 5 above and restated below: 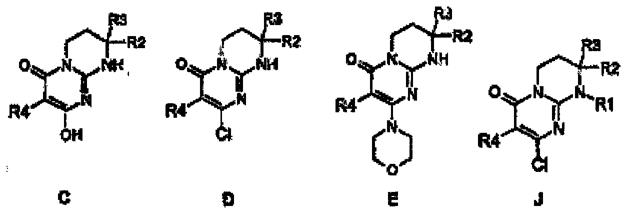 in which R1, R2, R3 and R4 have the definitions indicated in either one of Claims 1 and 2.

11. Products of formula (I) as defined in any one of Claims 1 to 3 for their use in the treatment of cancers.

12. Products of formula (I) as defined in any one of Claims 1 to 3 for their use in the treatment of solid or liquid tumours.

13. Products of formula (I) as defined in any one of Claims 1 to 3 for their use in the treatment of cancers which are resistant to cytotoxic agents.

14. Products of formula (I) as defined in any one of Claims 1 to 3 for their use in the treatment of primary tumours and/or metastases.

15. Products of formula (I) according to the preceding claim for their use in the treatment of primary tumours and/or metastases in gastric, liver, kidney, ovarian, colon, prostate, endometrial or lung cancers, glioblastomas, thyroid, bladder or breast cancers, in melanomas, in lymphoid or myeloid haematopoietic tumours, in sarcomas, in brain, laryngeal, lymphatic, bone and pancreatic cancers, and in hamartomas.

16. Products of formula (I) as defined in any one of Claims 1 to 3 for their use in cancer chemotherapy.

17. Products of formula (I) as defined in any one of Claims 1 to 3 for their use in cancer chemotherapy, alone or in combination.

18. Products of formula (I) as defined in any one of Claims 1 to 3 for the prevention or treatment of lysosomal diseases.

19. Products of formula (I) according to the preceding claim for the prevention or treatment of glycogen storage disease type II or Pompe disease.

20. Products as defined in Claim 18, **characterized in that** they are used alone or in combination.

21. Products of formula (I) as defined in any one of Claims 1 to 3 for the treatment of parasitic diseases.

22. Products according to the preceding claim for the prevention or treatment of malaria, sleeping sickness, Chagas disease and leishmaniases.
